# FASCICULE DE BREVET EUROPEEN

(11) **EP 2 007 902 B1**
(45) Date de publication et mention de la délivrance du brevet: **16.01.2013**
(21) Numéro de dépôt: 07727052.8
(22) Date de dépôt: 19.03.2007
(51) Int. Cl.: C12Q 1/68

(54) **COMBINAISON DE MARQUEURS DE CELLULES AVIAIRES**
KOMBINATION VON VOGELZELLMARKERN
COMBINATION OF AVIAN CELL MARKERS

(30) Priorité: 24.03.2006 FR 0602592
(43) Date de publication de la demande: 31.12.2008
(73) Titulaire: INSTITUT NATIONAL DE LA RECHERCHE AGRONOMIQUE, 75007 Paris (FR); CENTRE NATIONAL DE LA RECHERCHE SCIENTIFIQUE (CNRS), 75016 Paris (FR); ECOLE NORMALE SUPERIEURE DE LYON, 69007 Lyon (FR)
(72) Inventeur: PAIN, Bertrand, F-63100 Clermont-Ferrand (FR); LAVIAL, Fabrice, F-42100 Saint Etienne (FR); BACHELARD, Elodie, F-69280 Marcy L'Etoile (FR); MONTILLET, Guillaume, F-69150 Decines (FR); SAMARUT, Jacques, F-69003 Lyon (FR)
(74) Mandataire: Regimbeau
(86) Numéro de dépôt international: PCT/EP2007/052574
(87) Numéro de publication internationale: WO 2007/110343

(56) Documents cités:
- WO-A-01/85938
- WO-A-98/38283
- JUNG JIN GYOUNG ET AL: "Development of novel markers for the characterization of chicken primordial germ. cells" STEM CELLS (MIAMISBURG), vol. 23, no. 5, mai 2005 (2005-05), pages 689-698, XP002406222 ISSN: 1066-5099
- KAMACHI YUSUKE ET AL: "Involvement of Sox1, 2 and 3 in the early and subsequent molecular events of lens induction" DEVELOPMENT (CAMBRIDGE), vol. 125, no. 13, juillet 1998 (1998-07), pages 2521-2532, XP002406223 ISSN: 0950-1991
- SWEENEY SHEILA A ET AL: "Messenger RNA and protein expression analysis of betaglycan in the pituitary and ovary of the domestic hen" BIOLOGY OF REPRODUCTION, vol. 72, no. 1, janvier 2005 (2005-01), pages 172-178, XP002406224 ISSN: 0006-3363
- HARDUF HAGGAR ET AL: "Sef is synexpressed with FGFs during chick embryogenesis and its expression is differentially regulated by FGFs in the developing limb" DEVELOPMENTAL DYNAMICS, vol. 233, no. 2, juin 2005 (2005-06), pages 301-312, XP002406225 ISSN: 1058-8388
- YUSUF FAISAL ET AL: "Expression of chemokine receptor CXCR4 during chick embryo development" ANATOMY AND EMBRYOLOGY, vol. 210, no. 1, août 2005 (2005-08), pages 35-41, XP002406226 ISSN: 0340-2061

## Description

La présente invention concerne une nouvelle combinaison de marqueurs de cellules aviaires permettant de caractériser lesdites cellules selon leur phénotype, qu'il s'agisse de cellules StX, ou de cellules souches. La présente invention concerne également un procédé de caractérisation des cellules aviaires avec lesdits marqueurs, ainsi qu'un procédé de culture de cellules aviaires, dans lequel on caractérise les cellules par le procédé selon l'invention.

L'objet de l'invention est d'identifier et d'utiliser une combinatoire unique de gènes susceptibles d'être utilisés comme marqueur pour définir, modifier, contrôler de façon positive ou négative la compétence germinale d'une cellule souche aviaire de quelque nature embryonnaire, germinale ou adulte que ce soit.

Une cellule souche est une cellule pluripotente ou multipotente d'origine embryonnaire ou adulte qui présente une capacité d'auto-renouvellement et qui est capable de donner des cellules différenciées spécialisées. En d'autres termes, une cellule souche est une cellule non cancéreuse capable de se diviser indéfiniment en culture et de donner une cellule fille présentant la même capacité de prolifération et de différenciation que la cellule mère dont elle est issue.

Différentes classes de cellules souches ont été isolées selon leur origine embryonnaire ou adulte et leur origine tissulaire somatique ou germinal. Il a été montré que les cellules souches embryonnaires de souris (MESC) étaient capables de contribuer à la lignée germinale quand elles sont réintroduites dans un embryon. Les mécanismes moléculaires qui contrôlent cette compétence germinale ne sont pas complètement identifiés.

Les cellules souches sont des cellules qui ont la prodigieuse propriété de s'autorenouveller à la fois *in vivo* où elles assurent le maintien et le renouvellement des tissus et *in vitro* où elles peuvent être maintenues avec des conditions définies de culture.

En fonction de leur origine tissulaire et de leur potentialité de différenciation, on distingue les cellules souches embryonnaires (ESC), cellules pluripotentes d'origine embryonnaire, les cellules souches germinales (EGC) et les cellules souches somatiques ou adultes d'origine tissulaire (ASC). Ces dernières présentent une certaine plasticité dans leur potentialité de différenciation à la fois *in vitro* et *in vivo.*

### Les cellules souches embryonnaires (ESC)

### Les cellules MESC

Les cellules souches embryonnaires (cellules ESC) ont été isolées et identifiées chez la souris dans les années 80 (Martin, 1981 ; Evans & Kaufman., 1981) et leur isolement s'est inscrit dans la continuité des nombreuses études menées auparavant avec les cellules de carcinome embryonnaire (ou cellules EC) (pour revue Chambers & Smith, 2004). Ces cellules ESC de souris (MESC) ont été obtenues à partir de la mise en culture in vitro de blastocystes de souris 129/SV selon différents protocoles décrits (Robertson, 1987, Hogan et al., 1994). Les blastocystes de souris avant gastrulation présentent une masse cellulaire interne ou ICM de 50 à 100 cellules, dont la caractérisation moléculaire est encore en cours. Le devenir de ces cellules n'est pas identique *in vivo* et *in vitro.*

*In vivo,* les cellules de l'épiblaste vont donner les tissus embryonnaires et les cellules germinales se forment dans l'épiblaste proximal sous l'induction de l'ectoderme extra-embryonnaire. Les cellules germinales apparaissent ainsi au sein d'une niche sous l'influence de différents facteurs et cytokines. Les mécanismes moléculaires complexes responsables de cette induction commencent seulement à être identifiés chez la souris (Saitou et al., 2003).

*In vitro,* la mise en culture des blastocystes conduit à l'éclosion de celui ci, à l'adhésion et à la prolifération des cellules de l'ICM qui dans des conditions de cultures appropriées donnent naissance aux cellules MESC. Ces conditions de culture *in vitro* sont désormais relativement bien identifiées et standardisées, notamment par l'addition dans le milieu de culture d'au moins une cytokine de la famille du LIF (Yoshida et al., 1994). De nouvelles voies de signalisation semblent également impliquées dans le maintien du phénotype de pluripotence des cellules MESC (Dani et al., 1998). De très nombreux travaux ont été publiés sur les mécanismes moléculaires qui contrôlent la pluripotence des cellules MESC. Dans ce modèle, différentes approches moléculaires et fonctionnelles ont permis de mettre en évidence le rôle clef des gènes *oct-3*/*4* (Niwa et al., 2000 ; Niwa et al., 2002), *nanog* (Chambers et al., 2003 ; Mitsui et al., 2003) *et stat3* (Niwa et al., 1998 ; Matsuda et al., 1999). Un schéma consensus place le LIF et le BMP au centre du signal mitotique et les gènes *Oct*/*nanog et stat3* comme régulateurs et effecteurs de la balance prolifération-différenciation des cellules ES (Ying et al., 2003 ; Chambers et al., 2004). Un des effecteurs aval serait le protooncogène c-myc directement régulé par STAT3 (Cartwright et al., 2005). D'autres acteurs sont impliquées comme la protéine P53 qui semble contrôler le niveau d'expression de nanog (Lin et al., 2005). La protéine OCT-3/4 contrôlerait sa transcription au niveau de son propre promoteur en association avec des protéines de la famille des HMG comme SOX-2 et/ou SOX-3 (Okomura et al., 2005). En particulier, la restriction de l'expression du gène Oct-3/4 à la lignée germinale de la souris serait sous l'action répressive du GCNF (Furhmann et al., 2001). Le récepteur Irh-1 serait quant à lui impliqué dans le maintien à un stade plus précoce du développement, comme dans les cellules de l'épiblaste (Gu et al., 2005) Au moins chez la souris, une compétence germinale semble donc requérir, entre autre, un fort niveau de GCNF et un niveau relativement faible de OCT-3/4, même si l'expression de ce gène oct-3/4 est indispensable à la lignée germinale (Kehler et al.; 2004). L'existence de nombreuses interactions entre des acteurs de différents réseaux de signalisation est une réalité qui n'est pas encore complètement explicitée pour comprendre le maintien de la pluripotence des cellules MESC.

On notera que le succès de l'établissement des cellules MESC dépend également de la souche de souris utilisée, avec une facilité d'établissement à partir du fond génétique 129SV. L'identification moléculaire de cette limitation n'a pas été documentée.

### Les cellules PESC et HESC

Au niveau des primates, les premières cellules ESC de singe (PESC) ont été isolées par J. Thomson de l'Université de Madison (WS) (Thomson et al., 1995) à partir de blastocystes. Depuis, d'autres laboratoires ont isolé de nouvelles lignées de cellules de singe à partir de différentes espèces. La caractérisation de ces cellules a également fait l'objet de nombreuses publications à la fois au niveau des caractères biochimiques (AP, télomérase, antigènes de surface,...) et au niveau des gènes impliqués dans le maintien de la pluripotence.

Au niveau humain, des cellules ESC (HESC) ont été obtenues *in vitro* à partir de la mise en culture de blastocystes (Thomson et al., 1998). Ces cellules présentent de nombreuses similarités avec les cellules MESC, mais aussi de grandes différences comme des sensibilités à des facteurs de croissance différents.

Au niveau des mécanismes impliqués dans le maintien de la pluripotence des cellules PESC et HESC, les gènes identifiés chez la souris semblent également impliqués, mais la situation apparaît plus complexe. A titre d'exemple, la sensibilité des cellules PESC et HESC au LIF et l'activation de la voie Jak/stat ne semble pas aussi critique que celle des cellules murines (Raz et al., 1999; Sumi et al., 2004 ; Humphrey et al., 2004). Ces cellules ne sont ainsi pas exclusivement dépendantes de cette seule cytokine, ce qui rend l'analyse du signal de prolifération plus complexe. Même si on retrouve l'expression des acteurs majeurs *oct-3*/*4, nanog et stat3* ainsi que d'autres comme *rex1, foxD3,* etc...dans les cellules ESC de primates, le rôle de ces gènes ne serait peut être pas aussi déterminant et l'existence de voies alternatives est envisagée.

### Les cellules CESC

Les cellules souches embryonnaires de poulet (CESC) ont été isolées par la mise en culture de cellules de blastodermes de poulet au stade X (Pain et al., 1996 ; demande de brevet N° FR94/12 598). Ces cellules CESC présentent toutes les caractéristiques de cellules souches embryonnaires (ESC) y compris la colonisation somatique et germinale. Néanmoins, cette colonisation germinale semble difficile à maintenir après quelques passages en culture *in vitro,* contrairement aux cellules ES murines. Au stade de prélèvement et de mise en culture des cellules CES, l'embryon de poulet est considéré comme une blastula déjà constituée de 50 000 à 60 000 cellules, organisée en deux feuillets embryonnaires, l'épiblaste et l'hypoblaste. Les seuls critères de morphologie, de répartition tridimensionnelle et d'agencement des cellules ne permettent pas une identification spécifique de différents sous-types cellulaires de façon certaine, notamment les cellules qui présentent une compétence germinale (Petitte et al., 1990 ; Carsience et al., 1993 ; Thoraval et al., 1994). La détection de cellules positives pour la protéine VASA dans l'embryon de poulet très précoce (stade III-IV Eg&K) a relancé l'hypothèse de la préformation de la lignée germinale chez le poulet. Les cellules seraient individualisées dans la lignée germinale dès les premières divisions de l'embryon (Tsukenawa et al., 2000) sur un modèle plus proche de celui de la drosophile que du modèle d'induction observé chez la souris (Extavour et al., 2003 ; Saitou et al., 2002). Ces cellules persisteraient alors au cours des divisions successives jusqu'au stade X.

Pour les cellules CESC de poulet, aucune donnée n'est disponible quant aux gènes impliqués dans le maintien de la pluripotence et de l'émergence de la lignée germinale dans l'embryon de poulet. Le profil d'expression de la plupart des gènes identifiés dans les autres espèces ainsi que leur régulation sont complètement inconnus.

### Les cellules souches adultes (Cellules ASC)

Des cellules présentant des propriétés d'autorenouvellement et de différenciation *in vitro* et *in vivo* ont été isolées à partir de tissus et sont appelées ASC 'Adult Stem Cells' (Wagers and Weissman, 2004). Ces cellules ont été isolées dans différentes espèces, principalement mammifères à partir de différents tissus, notamment, le tissu hématopoiétique (HSC hemopoietic Stem Cells) et le tissu nerveux (NSC Neural Stem Cells). Une littérature très fournie illustre et vise à caractériser ces cellules (pour revue Shizuru et al., 2005, Mayhall et al., 2004). De nombreux autres types cellulaires ont été identifiés qui présentent partiellement les propriétés de cellules souches. On peut citer :
- les cellules mesenchymales (MSC) (Hamada et al., 2005)
- les cellules satellites musculaires (Charge and Rudnicki, 2004 ; Seale et al ;, 2004)
- les précurseurs multipotents 'MAPC' (Jiang et al., 2002)
- les cellules mésangioblastes (Cossu and Bianco, 2003 ; Minasi et al, 2002).

Leur grande plasticité de différenciation observée permet de considérer les cellules souches adultes comme des cellules susceptibles de changer leur déterminisme de différenciation selon le contexte, le lieu et les signaux qu'elles reçoivent (Lakshmipathy, 2005, Galli et al., 2000) . Le lien qui existe, au moins *in vitro* entre une cellule ESC et une cellule ASC, peut apparaître comme un continuum d'étapes de différenciation limitée. On peut en effet reproduire *in vitro* des séquences de différenciation complète à partir de cellules ESC dans telle ou telle voie spécifique de différenciation. Le contrôle de ces mécanismes complexes d'induction des voies de différenciation revêt une grande importance dans la compréhension du déterminisme de ces cellules.

### Cellules souches germinales (Cellules EGC) et compétence germinale

A la différence des cellules ESC, dérivées directement à partir d'un embryon obtenu avant le stade de la gastrulation, les cellules souches embryonnaires germinales (cellules EGC) sont obtenues à partir des crêtes génitales déjà constituées dans l'embryon, crêtes qui ont été colonisées par les précurseurs germinaux. Au cours de l'émergence de la lignée germinale et selon les espèces, les cellules précurseurs s'individualisent dans différents territoires de l'embryon. L'exemple le plus étudié et celui qui commence le mieux à être décrypté au niveau moléculaire est celui de la lignée germinale de la souris.

Les cellules EG de souris (MEGC) présentent de nombreuses similitudes avec les cellules MESC, mais une des différences essentielle concerne le statut épigénétique de certains gènes (Shiota et al., 2002, Reik et al. 2001, Tada et al., 1998). Par statut épigénétique, on entend le degré de modification directe du DNA et des protéines associées comme les histones. Ce statut détermine pour une bonne partie l'activité transcriptionnelle du DNA qui correspond à de l'euchromatine ou à des domaines d'hétérochromatine qui sont transcriptionnellement inactifs. De nombreuses molécules interviennent pour modifier et contrôler ce statut notamment certaines methyltransférases comme les gènes dnmt, dont l'effet sur les cellules ESC et durant le développement commence à être étudié (Gaudet et al., 2004, Biniszkiewicz et al., 2002, Hattori et al., 2002).

Par compétence germinale, on entend la capacité d'une cellule à produire une cellule différenciée précurseur d'une cellule sexuelle fonctionnelle. Une cellule primordiale germinale donne ainsi des cellules de la lignée male (spermatogonies) ou femelle (ovocyte), capable de donner respectivement des spermatozoïdes matures et des ovocytes matures.

Cette compétence germinale peut être observée *in vivo* ou *in vitro.*

*In vivo,* selon les espèces, les cellules germinales s'individualisent selon un processus d'induction par les tissus et les cellules environnantes dans une niche. Les exemples les plus étudiés sont ceux de la drosophile D. melanogaster et du nématode C.elegans, espèce pour lesquelles de nombreux mutants ont été obtenus. Ce processus commence à être décrit au niveau moléculaire chez la souris (Saitou et al., 2002, Ohinata et al., 2005) pour lequel un premier modèle d'induction été proposé. Il fait intervenir la notion de niche spécifique dans laquelle s'individualisent les cellules germinales. L'induction est contrôlée par les cellules environnantes via les facteurs BMP-4 et le BMP-8 qui modifient le niveau transcriptionnel de gènes clefs comme oct-3/4, blimp-1, les marqueurs *stella, fragilis,* etc.

Dans d'autres espèces, l'individualisation de la lignée germinale aurait une forte composante maternelle. Les RNA binding protéines joueraient alors un rôle essentiel dans l'établissement d'un gradient de certains composants (protéines et RNA) dans l'embryon non segmenté qui induiraient ainsi une 'séquestration' de ces protéines et/ou ARN messagers capables d'induire l'identité des cellules germinales dans un endroit défini de l'embryon. Les gènes impliqués dans cette compartimentalisation commencent à être particulièrement étudiés dans les organismes modèles comme D. melanogaster et C. elegans (Extavour et al., 2003, Blackwell, 2004). La description, le nombre et l'implication des différents gènes dans ces déterminismes sont documentés notamment au travers de revues scientifiques et de différents sites accessibles comme http://germonline.igh.cnrs.fr/index.php.

*In vitro,* des exemples récents montrent qu'il est possible d'obtenir de reproduire partiellement des conditions de différenciation de cellules souches embryonnaires de souris (MESC) en cellules germinales compétentes. Ce processus est sous la dépendance expérimentale de facteurs de croissance et de conditions tridimensionnelles particulières. On trouve les facteurs BMP-4 et BMP-8, ainsi que des hormones comme les oestrogènes et la FSH pour l'obtention de cellules germinales à partir de MESC (Hubner et al., 2003 ; Geijsen et al., 2004).

On connaît différents procédés de culture de cellules aviaires, cellules de blastodermne de stade X (StX), cellules souches ou cellules germinales, notamment pour la réplication de virus ou encore pour la production de protéines hétérologues et le cas échéant la production de vaccins. De tels procédés sont en particulier décrits dans les demandes de brevet et brevets ci-après : WO 03/043415, WO2005/007840, WO 03/076601, WO 01/85938, WO 96/12793, EP 1149899, US 2002-192815 ou US 6500668.

On connaît différents moyens de différencier les différentes formes de cellules ci-dessus. Il est toutefois important de pourvoir disposer de techniques de caractérisation des phénotypes de différentes cellules d'usage plus aisé et de plus grande pertinence.

La demande de brevet WO 98/38283 décrit les épitopes EMA-1 et SSEA-1, spécifiques des cellules germinales de poulet au stade X. L'article de Jung et al., publié en 2005 dans 'Stem cells', décrit des marqueurs pour la caractérisation des cellules germinales primordiales (PGC) du poulet : les marqueurs SSEA-1, SSEA-3, SSEA-4, EMA-1 sont spécifiquement exprimés sur ces cellules PGC.

Des gènes ont été identifiés comme étant exprimés soit dans les cellules souches embryonnaires de poulet (CESC) soient dans les cellules souches germinales de poulet (CEGC). La comparaison des niveaux relatifs d'expression de ces différents acteurs permet de proposer une combinatoire spécifique et originale qui définit le profil moléculaire de chacune de ces cellules ainsi que les modifications nécessaires à l'obtention d'une compétence germinale certaine des cellules CESC.

La combinaison de marqueurs et le procédé selon l'invention permettent notamment d'assurer une meilleure traçabilité des lignées cellulaires aviaires, mais également d'assurer une bonne stabilité dans le temps des lignées cellulaires cultivées.
- Par cellule souche, on entend toute cellule capable de s'auto-renouveler *in vitro* et capable de donner des cellules différenciées spécialisées.
- Par cellule germinale, on entend toute cellule capable de donner des précurseurs ou des cellules différenciées sexuées de la lignée male ou femelle.
- Par cellule de blastoderme de stade X, encore désignée « cellule StX », on entend les cellules de blastoderme obtenues par dissociation de l'embryon d'un oeuf fécondé juste pondu et dont le stade de développement correspond à un stade X selon la table EG &K (Eyal Giladi & Kovak, 1976).
- Par puce à ADN, on entend un ensemble de gènes, de fragments de gènes, d'oligonucléotides déposés sur un support (lame de verre, membrane en nylon, ...) avec une haute densité.
- Par méiose, on entend le processus biologique qui conduit une cellule diploïde à réduire son patrimoine génétique à un niveau haploïde. Ce processus est accompagné d'au moins une division cellulaire.
- Par cellule sexuelle fonctionnelle, on entend une cellule haploïde capable de fusionner ou d'apporter un jeu de chromosomes à une autre cellule haploïde pour former en cas de fécondation un oeuf, cellule diploïde capable de se développer en embryon.

L'invention concerne donc une combinaison de marqueurs permettant de caractériser des cellules aviaires de type StX **(blastoderme de stade X) ou, des** cellules souches **aviaires**, comprenant **au moins deux marqueurs caractérisée en ce que l'un des marqueurs est spécifique du gène 1p06, et l'autre marqueur est choisi parmi:**
a. un marqueur d'un gène cible préférentiellement exprimé dans les cellules StX choisi parmi les gènes suivants : 2contig58, 60S-L14, ATM, Bloom syndrome, BTEB4, CD9, CHD helicase, Clock, cwf16 (FLJ10374), CXCR4, Dnmt2, enx1 (ho-zeste 2), eomes, EWS, FGF-4, GATA-5, HOJ-1, N-AGN6P deacetylase, N-Cor1, NF2, p53, pml, rbm6, SA-2, SA-3, SARA, SCYE1, SEF, sf-1, SnoN, SOCS13, SSB-1, TC87479, T-cell APP 2C, TGF-beta2, WD40/FYVE-d protein 2, WD-RP3, Zan75, ZPC,
b. un marqueur d'un gène cible préférentiellement exprimé dans les cellules souches choisi parmi les gènes suivants : 1P08-A09, activin RIIB, astacin, Claudin-3, dapper-1, Dorfin, FPP synthase (fps), GalNAc-T3, gcnf, HSPb7, IRX4, LMX, pax-6, Slc38a2, sox-3, tral gp96, wnt-10a, Wnt-11.

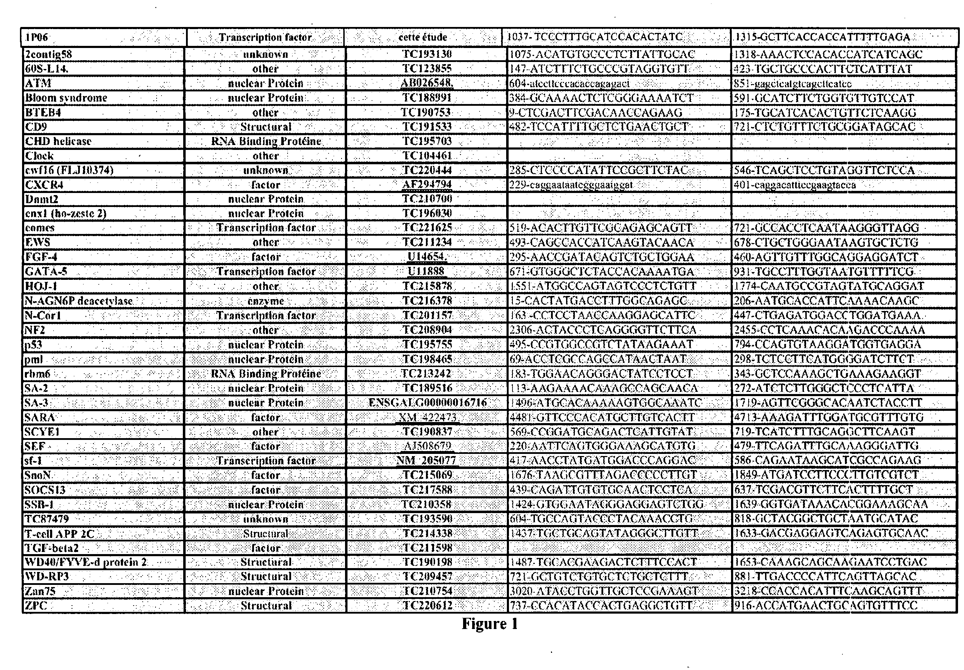

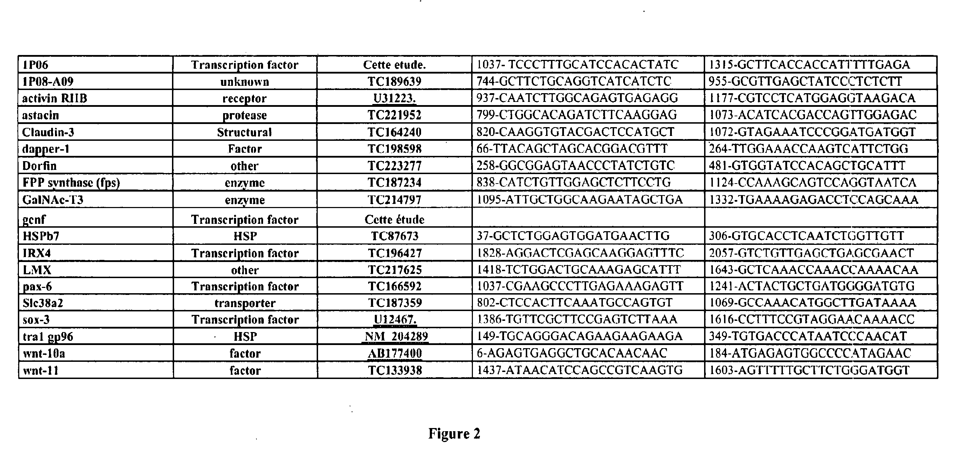

Une liste des gènes cibles préférentiellement exprimés dans les cellules StX, et les cellules souches est donnée sur les Figures 1 à 2 respectivement, avec les amorces employées pour les isoler par PCR.

De manière préférentielle, le marqueur d'un gène préférentiellement exprimé dans les cellules StX est choisi parmi les marqueurs des gènes suivants : ATM, CXCR4, eomes, FGF-4, GATA-5, NF2, SOCS13, SSB-1, TC87479, T-cell APP 2C, TGF-beta2, WD-RP3, ZPC. Plus préférentiellement, la combinaison selon l'invention comprend au moins un marqueur du gène ZPC.

Selon un autre mode préférentiel de réalisation de l'invention, le marqueur d'un gène préférentiellement exprimé dans les cellules souches est choisi parmi les marqueurs des gènes suivants : activin RIIB, astacin, Claudin-3, dapper-1, FPP synthase (fps), GalNAc-T3, gcnf, LMX, pax-6, tra1 gp96, wnt-10a. Plus préférentiellement, la combinaison selon l'invention comprend au moins un marqueur des gènes 1 P06 et tra-1.

Selon le mode préféré de réalisation de l'invention, la combinaison comprend la combinaison de marqueurs des gènes suivants : 1 P06-**et l'un des marqueurs suivants :** tra-1, FPP synthase, l'astacine, la GalnacT3, lmx, genf, eomes, id2, FGF-4, ZPC, gata-5, dmrt-1, Irh-1, pten.

Par « marqueur », on entend selon l'invention tout moyen, biologique, chimique ou physique permettant d'identifier et le cas échéant de quantifier l'expression d'un gène cible dans une cellule, cellule aviaire selon l'invention. De tels marqueurs sont bien connus de l'homme du métier. Leur composition dépendra en particulier du gène cible et de la méthode de détection de l'expression dudit gène cible. On citera notamment les méthodes employant les couples anticorps/antigène, l'anticorps venant se lier de manière spécifique à l'antigène, ce dernier étant constitué par le produit d'expression d'un gène identifié ci-dessus, ARNm ou ADNc ou polypeptide, ou un fragment de ces derniers. On citera également les fragments d'acide nucléique capables de s'hybrider de manière spécifique aux ARNm exprimés par lesdits gènes ou aux ADNc correspondants, ou des fragments de ces derniers.

Les différents types de marqueurs peuvent bien entendu être combinés dans la combinaison selon l'invention.

De manière avantageuse, les marqueurs selon l'invention sont des séquences d'acides nucléiques capables de s'hybrider aux ARN ou aux ADNc, produits d'expression des gènes cibles identifiés ci-dessus.

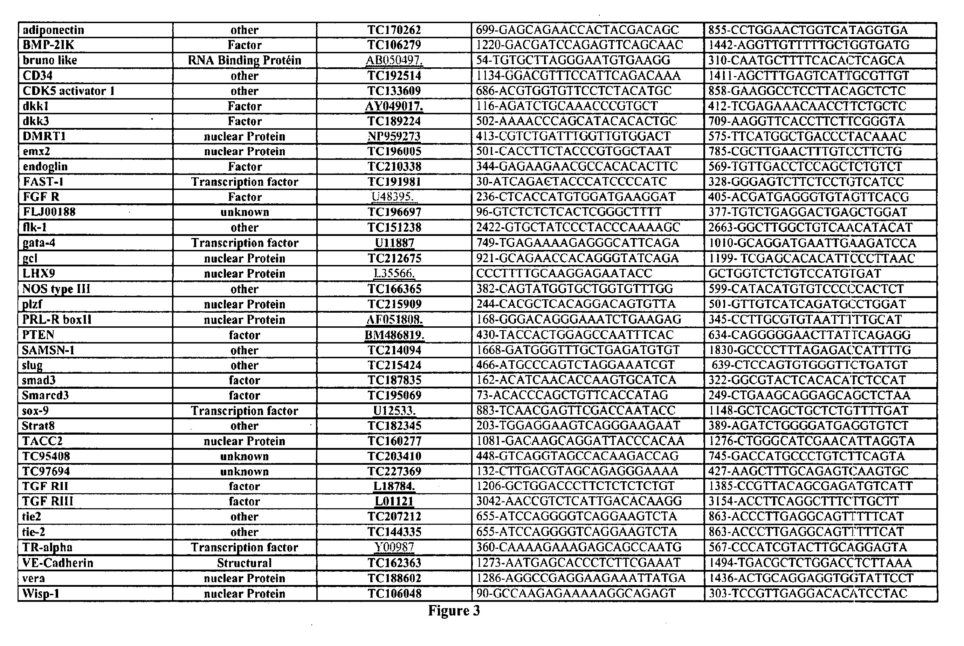

Parmi ces séquences d'acides nucléiques, on pourra citer les séquences particulières de amorces identifiées sur les Figures 1 à 3.

Selon un mode plus particulier de réalisation de l'invention, la combinaison des marqueurs est assemblée sur un même support, de préférence un support normé. L'homme du métier connaît ces différents supports, dont les dimensions varient selon le type de marqueurs et d'appareillages susceptibles d'être employés pour détecter l'expression du ou des gènes cibles.

De manière avantageuse, la combinaison de marqueurs selon l'invention se présente sous la forme d'une matrice à ADN, comprenant un support sur lequel sont disposés, préférentiellement de manière normée, les fragments d'acide nucléique susceptibles de s'hybrider avec les gènes cibles. Les dimensions de tels supports peuvent varier selon les technologies de préparation et de détection employées. Te tels supports de dimensions réduites sont également appelés puce à ADN.

De manière avantageuse, la combinaison de marqueurs selon l'invention est disposée sur une puce à ADN. La puce à ADN comprenant la combinaison de marqueurs selon l'invention est également partie de l'invention.

Selon un mode particulier de réalisation de l'invention, la combinaison de marqueurs peut comprendre en outre au moins un marqueur d'un gène qui s'exprime dans deux groupes de cellules, les cellules StX et les cellules souches.

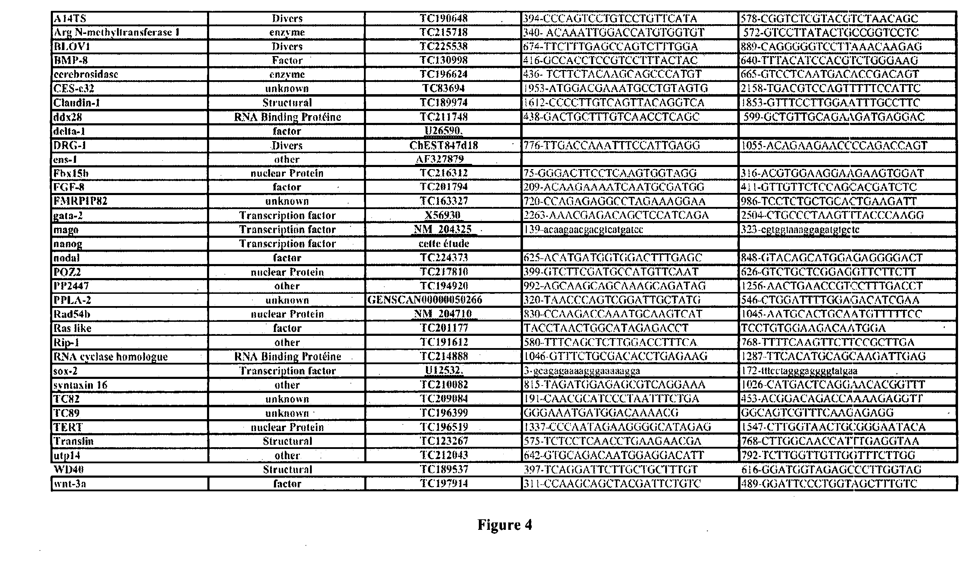

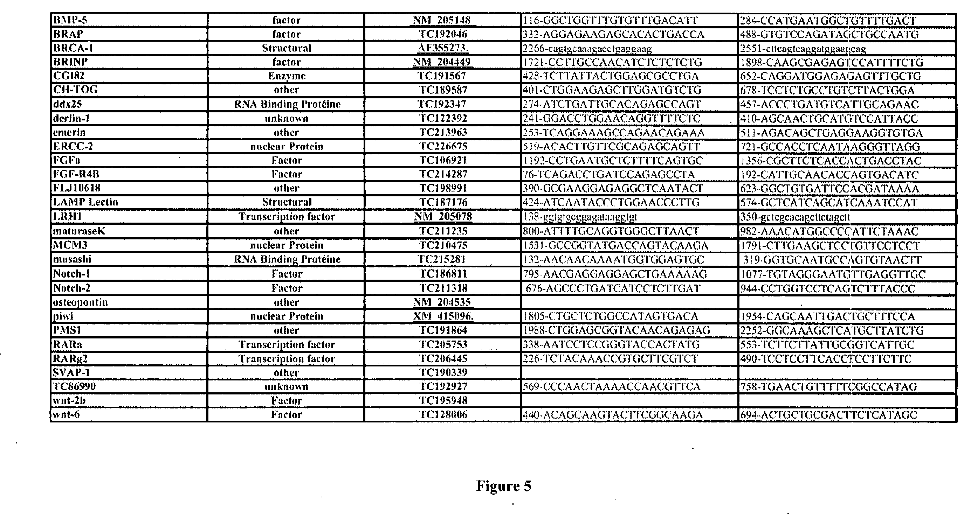

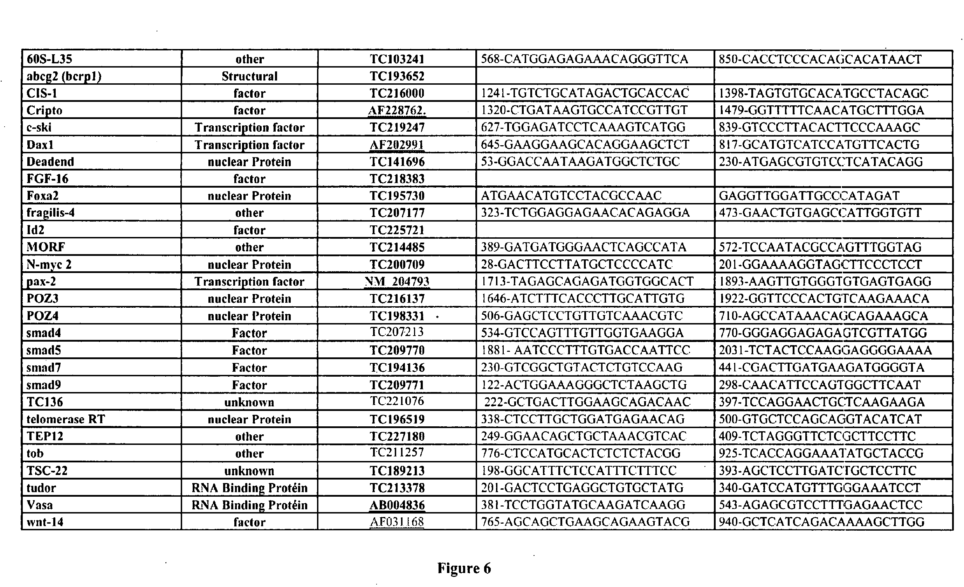

De tels gènes sont identifiés sur les Figures 4 à 6 respectivement avec les « amorces » correspondants.

Les moyens de détection des interactions « produit d'expression » / marqueur sont bien connus de l'homme du métier. De nombreuses références de publications scientifiques sur la préparation et l'utilisation de puces à ADN, comme concernant des organismes académiques de recherche ou d'entreprises commercialisant les moyens de préparation et d'utilisation des puces à ADN (appareils de lecture, logiciels de traitements de signal, bases de données, etc) sont données **notamment sur les sites Web** www.gene-chips.com, www.deathstramic.com/science/biology/chips.html ou http://cmgm.stanford.edu/pbrown/mguide/index.html dont le contenu, et celui des références citées est incorporé ici par référence.

La présente invention concerne également un procédé de caractérisation d'une cellule aviaire comprenant l'analyse de l'expression des gènes exprimés dans ladite cellule au moyen d'une combinaison de marqueurs telle que définie précédemment et dans les exemples, et la caractérisation du phénotype des cellules analysées. Les méthodes particulières d'analyse de l'expression de gènes dépendront des marqueurs employés dans la combinaison selon l'invention définis précédemment et dans les exemples.

L'invention concerne aussi un procédé de culture de cellules aviaires comprenant la culture de cellules **préalablement caractérisées** dans un milieu de culture approprié, leur caractérisation **étant faite** au moyen d'un procédé selon l'invention, avec une combinaison de marqueurs selon l'invention tels que définis précédemment et dans les exemples. Le procédé de culture selon l'invention comprend une étape d'isolation des cellules sélectionnées parmi les cellules StX,-et les cellules souches-aviaires.

L'invention concerne aussi un procédé de culture de cellules aviaires par lequel on obtient des cellules germinales aviaires à partir de cellules StX. Le procédé selon l'invention se distingue des procédés de l'art antérieur qui ne permettent pas une telle transformation, par le fait que les cellules StX sont cultivées dans un milieu de culture approprié sans adjonction de tapis nourricier « feeder » inactivé.

Les inventeurs ont pu constater que, alors que la pratique usuelle d'adjonction de « feeder » est nécessaire pour l'obtention de cellules souches, le fait de ne pas ajouter un tel « feeder » permet d'obtenir, de manière totalement inattendue, des cellules de type germinales.

Les caractéristiques du « feeder » sont bien connues de l'homme du métier, décrites notamment dans Robertson et al., 1987, karagenc et al., 2000 dont le contenu est incorporé ici par référence.

D'autres caractéristiques de l'invention apparaîtront à la lecture des exemples présentés ci-après.

### Brève Description des Figures

**Figure 1** **:** Tableau des gènes exprimés dans les cellules StX.
**Figure 2** **:** Tableau des gènes exprimés dans les cellules souches.
**Figure 3** **:** Tableau des gènes exprimés dans les cellules germinales.
**Figure 4** **:** Tableau des gènes exprimés dans les cellules StX et les cellules souches.
**Figure 5** **:** Tableau des gènes exprimés dans les cellules souches et les cellules germinales.
**Figure 6** **:** Tableau des gènes exprimés dans les cellules germinales et les cellules de stade X.
**Figure 7** **:** Courbe de croissance des cellules GF58.
**Figure 8** **:** Niveau d'expression de quelques gènes dans les cellules GF58 (p7).
**Figure 9** **:** Analyse de l'expression de gènes dans les fractions p3g et p4g par rapport au niveau observé dans les gonades (=1). Un ratio > 1 suggère que le gène est plus exprimé dans la fraction testée que dans la gonade embryonnaire entière et inversement.
**Figure 10** **:** Répartition par classe des gènes testés.
**Figure 11** **:** Variation du niveau d'expression des gènes de classe 1 au cours de l'induction de la différenciation.
**Figure 12** Variation du niveau d'expression des gènes de classe 2 au cours de l'induction de la différenciation.
**Figure 13** **:** Variation du niveau d'expression des gènes de classe 3 au cours de l'induction de la différenciation.
**Figure 14** **:** Comparaison des profils d'expression entre les cellules de blastoderme de stade X, les cellules CESC et les cellules CEGC.
**Figure 15** **:** Comparaison des profils d'expression entre les cellules CESC, les cellules de blastoderme de stade X et els cellules CEGC.
**Figure 16** **:** Comparaison des profils d'expression entre les cellules CEGC, les cellules de blastoderme de stade X et les cellules CESC.
**Figure 17** **:** Séquence de la protéine et du cDNA du gène nanog poulet.
**Figure 18** **:** Séquence de la protéine et du cDNA du gène gcnf de poulet.

### Exemples de Réalisation

### Exemple 1 : Obtention de cellules souches embryonnaires in vitro,

Les cellules souches embryonnaires de poulet sont obtenues *in vitro* à partir d'oeufs embryonnés. Deux approches sont utilisées pour obtenir des populations de cellules souches embryonnaires en prolifération *in vitro.*

Dans un premier aspect, les cellules sont obtenues dans des conditions similaires à celles précédemment décrites (Pain et al., 1996). Brièvement, les oeufs embryonnés non incubés sont nettoyés et cassés. Le jaune est séparé du blanc et l'embryon est prélevé à l'aide d'une pipette Pasteur ou d'un anneau de papier tel que décrit par Carscience et al., 1993. Les cellules ainsi obtenues sont déposées sur un tapis nourricier inactivé dans un milieu de prolifération contenant les éléments essentiels à la prolifération des cellules comme le sérum de veau foetal, des acides aminés et des cytokines et facteurs de croissance.

Dans un autre aspect de l'invention, les cellules sont ensemencées directement dans une boite de plastique non traité culture cellulaire et destinée à un usage de culture bactériologique. Dans ce dernier cas, les cellules sont mises en légère agitation. Il se forme des agrégats de cellules souches qui prolifèrent après quelques jours en suspension.

Après quelques jours, les cellules souches embryonnaires forment de petits amas compacts qui sont dissociés à l'aide d'enzymes protéolytiques et sont ensemencées dans de nouvelles boites de cultures pour une amplification in vitro.

Après quelques passages, la culture devient plus homogène et le type cellulaire prépondérant est la cellule souche embryonnaire de poulet (CESC). Ces cellules CESC sont caractérisées par les différents marqueurs décrits préalablement, notamment la présence de l'activité Alcaline phosphatase, l'activité télomérase endogène et leur réactivité vis à vis d'anticorps spécifiques (Pain et al., 1996).

Afin de caractériser les cellules CESC obtenues en culture, une observation en microscopie électronique a été réalisée. Elle indique que les cellules isolées présentent de très nombreuses microvillosités à leur surface, ce qui n'a jamais été présenté ou décrit pour une cellule CESC. Que celles-ci disparaissent dés que les cellules sont en amas ou interagissent avec d'autres cellules homologues ou hétérologues (cellules du feeder). Que les différentes structures et ultrastructures reconnues par ME indiquent un métabolisme important (ribosomes, réticulum, mitochondries nombreuses) une forte activité transcriptionnelle par la présence d'un nucléole important. Que ces cellules CESC présentent des dimensions de 7 à 15 µm en moyenne avec un noyau relativement homogène en taille plus ou moins sphérique de 4 à 8 µm en moyenne. L'espace intracellulaire des cellules en amas est très restreint, car les cellules sont particulièrement jointives.

### Exemple 2 : Obtention de cellules germinales in vitro

Chez l'oiseau, les acteurs moléculaires du déterminisme des cellules germinales ne sont pas encore bien identifiés. Ce déterminisme demeure un processus relativement peu documenté. Le stade de développement de l'embryon au moment de la ponte est le stade IX-XI selon la table de développement proposée par Eyal Giladi & Kovak (1976). A ce stade, l'embryon comprend déjà de 40 000 à 60 000 cellules selon les estimations et est composé de deux feuillets, l'épiblaste et l'hypoblaste. A ce stade, la présence de cellules positives pour le gène vasa (Tsukenawa et al., 2000) indiquent que des cellules germinales sont déjà déterminées. Différentes publications ont fait état de l'obtention in vitro de cellules souches germinales (EG) à partir de la mise en culture des cellules de la crête germinale de l'embryon de poulet. On peut citer notamment les travaux de Ha et al., 2002 ; Park et al., 2003 à partir d'embryon de stade 28 selon la table de développement proposée par Hamburger& Hamilton (1951) correspondant environ à 5-6 jours de développement.

Dans un autre aspect de l'invention, les cellules de l'embryon sont prélevées comme décrit dans l'exemple 1, mais elles sont ensemencées dans une boite de culture sans tapis nourricier. La boite de culture a été au préalable traitée au moins 1 heure ou davantage avec une solution de blanc d'oeuf ou d'une autres solution riche en protéine sérique, comme de l'albumine sérique. Cette solution de blanc peut être du blanc d'oeuf directement prélevé ou dilué avec du PBS. De préférence, une solution diluée entre 5 et 10 fois est utilisée. La solution contient donc les composants principaux de l'albumen de l'oeuf, comme l'ovalbumine, la conalbumine, les ovomucoïdes, l'ovomucine et le lysozyme, ainsi que les autres composants plus minoritaires. Une solution d'ovalbumine ou de conalbumine purifiée peut être également utilisée.

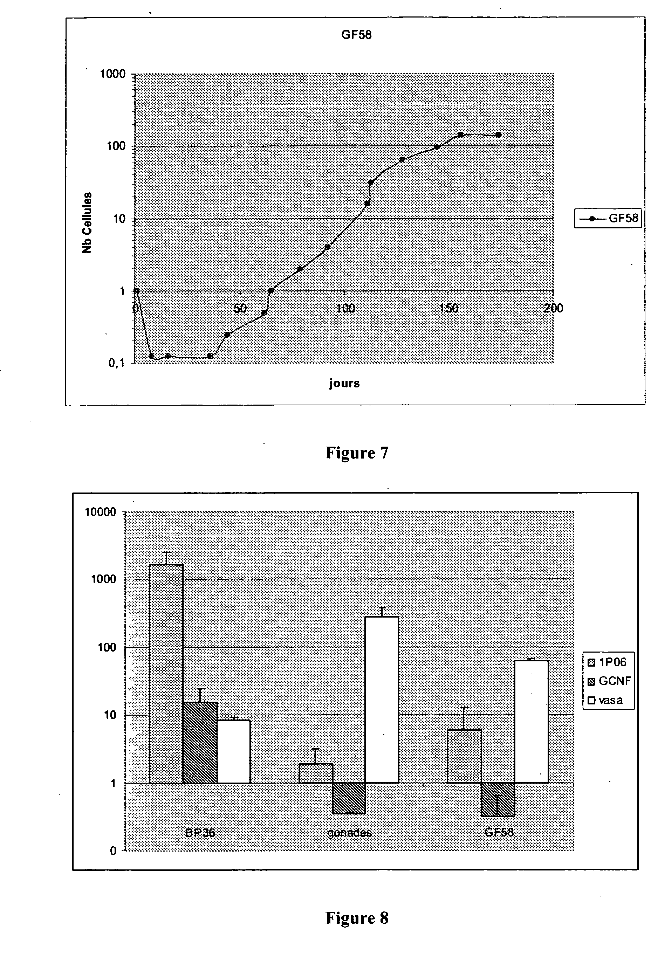

Dans un aspect de l'invention, des cellules de blastoderme de stade IX-XIII sont ensemencées en boite de culture dans des conditions de culture qui favorise la multiplication des cellules germinales 'vasa' positives. Ainsi, les boites ont été traitées au sérum pour réaliser un 'coating' équivalent à une matrice extracellulaire destinées à favoriser l'adhésion et la non différenciation des cellules mais sans addition de tapis nourricier 'feeder' inactivé. Après quelques jours, des foyers de cellules apparaissent qui présentent une prolifération lente. Ce processus a perduré et les cellules appelées GF58 ont continué à proliférer pendant au moins 200 jours. Cette prolifération, bien que particulièrement lente est néanmoins continue sous la forme de quelques foyers compacts de cellules. Les passages sont délicats, car il est difficile de dissocier les cellules. Une 'courbe' de croissance a été réalisée (Figure7), basée sur les coefficients de dilution lors des passages des cellules, car il apparaît impossible de compter les cellules individuellement à chaque (rare) passage.

Ces cellules présentent une morphologie particulière et prolifèrent en petits amas au centre d'un foyer de cellules adhérentes et pavimenteuses, à gros noyau. La taille des foyers s'élargit très lentement.

Des tests pour remettre les cellules en présence de 'feeder' sont apparus catastrophiques, les cellules ne résistant pas à cet environnement. Des essais d'addition de BMP-4, FGF-4 dans le milieu de culture n'ont pas eu d'effets négatifs sur la prolifération et n'ont pas entraîné de modification morphologique majeure. Une faible stimulation serait observée.

Au passage 7 (après 79 jours de culture), les cellules présentent une forte activité alcaline phosphatasse endogène et sont positives pour les anticorps SSEA-1 et EMA-1. A ce même passage 7, quelques cellules ont été prélevées pour une analyse au niveau moléculaire. Les résultats (Figure 8) indiquent que le profil d'expression des gènes 1P06, gcnf et vasa est plus proche de celui des gonades que des cellules CESC.

Ce résultat très important supporte l'idée du maintien de cellules germinales vasa positives après 80 jours de cultures, obtenues à partir de la mise en culture de cellules de blastoderme de stade X. Ces cellules sont des cellules germinales maintenues in vitro.

### Exemple 3 : Obtention de cellules germinales par tri cellulaire de gonades embryonnaires

Afin d'obtenir des cellules germinales purifiées en quantité utilisable pour une analyse moléculaire, une approche de tri par FACS est initiée. L'approche s'appuie sur la présence d'une protéine membranaire, le récepteur ABC transporteur Bcrp1/ABCG2, dont l'expression a été initialement identifiée dans les cellules souches hématopoiétiques (Goodell et al., 1997) Cette molécule est décrite comme un marqueur de cellules souches hématopoiétiques. Cette molécule semble assurer un rôle physiologique de détoxification et est responsable du transport actif du Hoëchst hors de la cellule, quand cette drogue est mise en présence de la cellule. Cette propriété semble spécifique des cellules souches et est actuellement mise à profit pour identifier des 'SP cells' ou 'side population', d'après leur caractéristique lors d'un tri cellulaire dans différents tissus (Zhou et al., 2001). De façon intéressante, les cellules germinales de souris semblent présenter cette propriété (Lassalle et al., 2004).

En première approche, le niveau d'expression du messager bcrp1 est plus élevé dans les cellules germinales de poulet que dans les cellules souches CESC (cf infra). Ce gène devient ainsi un marqueur supplémentaire pour identifier une cellule souche chez le poulet et une cellule germinale.

Afin de tester le rôle de cette molécule comme marqueur dans les cellules CESC et les cellules germinales de poulet, des études de fractionnement de gonades embryonnaires de poulet ont été entreprises. Les gonades embryonnaires d'embryons males de 15 à 21 jours de développement sont prélevées après décapitation de l'embryon et placées dans la glace. Les gonades sont placées dans du milieu HBSS 1X (GIBCO ref 14175-046) complet contenant 20mM Hepes pH 7.2, 1.2mM MgSO4 7H₂O, 1.3mM CaCl₂ 2H₂O, 6.6mM sodium pyruvate, 0.05% lactate et 1% du mélange d'antibiotique penicilline Streptamycine. Les gonades sont dissociées mécaniquement par passage successif dans une aiguille de 18G1/2 puis 20G 1/2 jusqu'à l'obtention d'une suspension monocellulaire qui est alors filtrée sur une membrane de nylon 100µM (Falcon 352360), comptée par numération cellulaire avec du bleu trypan et centrifugée 10 min à 400g à 4°C. Le culot est repris dans du milieu HBSS complet.

Une partie des cellules (de 10⁵ à 2x10⁶ cellules) est soumise dans du HBSS complet à un traitement au Vérapamil (V4629 SIGMA) ou à la drogue Ko143 (Lassalle et al., 2004, Allen et al. 2002) aux concentrations respectives de 75µg/ml et de 200nM/ml finale. Les cellules sont placées 20 min à l'étuve 37,5°C 7.5% CO₂. A l'issue de cette incubation, les cellules sont rincées avec du PBS IX, centrifugées pendant 10 min à 400g et le culot repris dans 1 ml de HBSS complet. Les cellules contrôles non traitées au Vérapamil ou au Ko143 sont placées dans la glace.

Les cellules traitées au Vérapamil, au Ko143 ou non traitées sont reprises dans du HBSS complet à la concentration de 1 à 2.10⁶ cellules/ml puis incubées avec le Hoechst 33342 (réf B2261, Sigma) à la concentration de 5µg/ml finale pendant 45 min dans un incubateur à 37,5°C 7.5% CO₂. A l'issue de cette incubation, les cellules sont rincées avec du PBS 1X, centrifugées 10 min à 400g et le culot est repris dans 1ml HBSS complet additionné de 0.5%SVF. L'ensemble est alors placé dans la glace. L'iodure de propidium est ajouté extemporanément à la concentration de 2µg/ml avant l'analyse FACS

Un profil de répartition en FACS montre la présence de différentes populations de cellules en présence ou en absence des différentes drogues. On peut ainsi définir deux populations principales, p3g (R1) et p4g (R2). La population appelée p3g est la plus sensible à l'action des drogues connues pour inhiber le canal abcg2, notamment le Ko143.

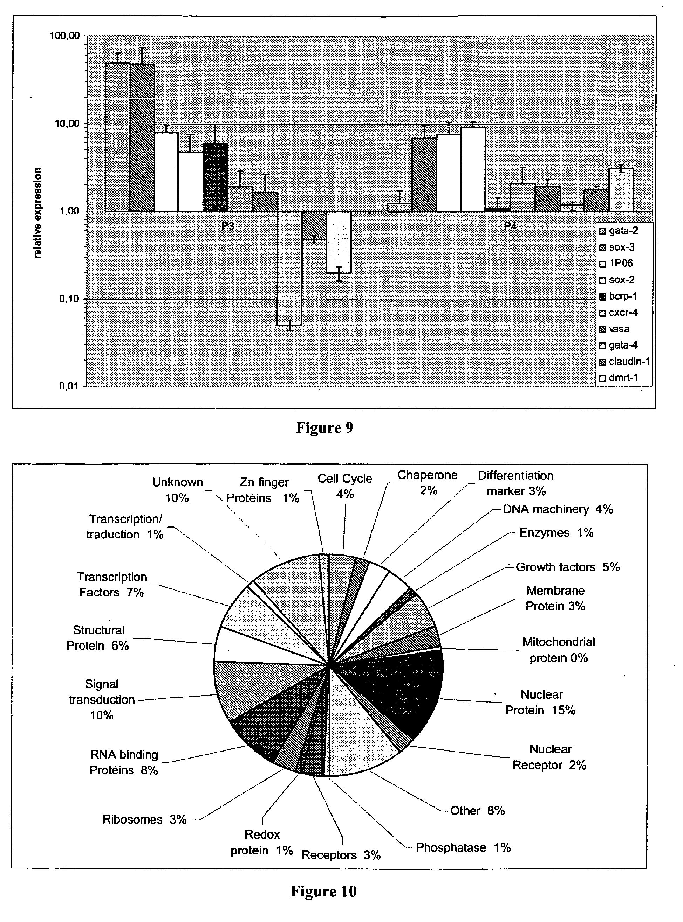

Les populations p3g et p4g ont été triées par FACS et les RNA de ces cellules analysés en Q-PCR (Figure 9). Sur les quelques gènes testés, il est possible d'identifier la fraction p3g comme étant enrichie en cellules germinales., car seule cette fraction est enrichie en bcrp-1 (abcg2) ; au contraire la fraction p4g se distingue par un enrichissement en dmrt-1 et gata-4, marqueurs caractéristiques des cellules de soutien.

La détection de marqueurs de cellules germinales plus exprimés dans cette sous population p3g confirme l'intérêt de cette méthode d'enrichissement pour obtenir des cellules germinales purifiées.

### Exemple 4 : Identification des gènes impliqués dans la pluripotence des cellules souches embryonnaires aviaires (CESC).

Afin d'identifier les gènes impliqués dans le maintien de la pluripotence des cellules CES, différentes stratégies complémentaires de criblage permettent de caractériser au niveau transcriptionnel les cellules CESC. Parmi les approches, on peut citer le criblage différentiel de banques d'expression, le criblage *in silico* des banques de données, le criblage de puces à ADN spécifiques du poulet et le criblage de banques SAGE. En utilisant ces différentes approches, une liste de gènes candidats est définie. Le niveau d'expression de ces gènes est testé par une approche de PCR semi quantitative et quantitative. Les 800 différents gènes testés se répartissent dans différentes classes (Figure 10).

Les messagers (mRNA) sont extraits des cellules CESC en prolifération à l'aide de kit RNA-Easy (Qiagen, Réf 74104). A l'issue de la préparation sur colonne, les RNAs sont récupérés dans 50ul d eau RNase free, dosés et conservés précipités dans l'éthanol à -80°C. Une réaction de reverse transcription avec une amorce oligodT. est réalisée 3' avec la reverse transcriptase Superscript II (Invitrogen, Carlsbad) à42°C pendant 1 h avec 2µg de RNA totaux issus de cellules CESC indifférenciées en prolifération. Le produit de RT est dilué 100 fois dans l'eau millipore et une réaction de PCR quantitative est lancée en plaque avec l'appareil Mx3500P (*Stratagene*). Le mélange réactionnel de 25 µl dans l'eau comprend 2 µl du produit de RT dilué, 12,5 µl du Mix Quantitect SYBRGreen (Qiagen, réf 204245), 10 pM de chaque amorce. Après une dénaturation de 15 min, le mélange est soumis à 40 cycles composés de 30 s d'une phase de dénaturation à 95°C, de 30 s d'une phase d'hybridation à 55°C et de 30 s d'une phase d'élongation à 72°C. Deux ou trois essais indépendants sont réalisés pour chaque gène et la moyenne est portée comme valeur relative d'expression.

Afin de valider les données obtenues en PCR quantitative, deux méthodes complémentaires sont utilisées, la courbe de dissociation (melting curve) et le calcul de l'efficacité de la PCR par la méthode du standard interne. Cette dernière analyse permet d'évaluer si chaque molécule de DNA est bien amplifiée spécifiquement à chaque cycle de PCR. La courbe de dissociation informe sur le nombre d'amplicons de nature différente présents dans les tubes à la fin de l'amplification et permet de tester la spécificité du couple d'amorces utilisé, pour lequel un seul amplicon doit être formé. Cette courbe permet également de détecter une éventuelle contamination par de l'ADN génomique sous réserve que les amorces soient choisies entre deux exons d'un gène. Cette courbe est réalisée à la fin des 40 cycles de PCR quantitative par un dernier cycle qui comprend une dénaturation de 1 min à 95 °C , une hybridation de 1 min à 55°C puis une montée en température de 55°C à 95°C avec une lecture de la fluorescence toutes les 0,2s. Ainsi, à 55°C, les fragments d'ADN amplifiés au cours des 40 cycles de PCR s'hybrident et la fluorescence mesurée sera alors à son maximum. En augmentant la température très progressivement la température à laquelle les deux brins d'ADN se séparent est mesurée très précisément ce qui provoque une chute de la fluorsecence, le fluorochrome n'étant incorporé que dans l'ADN bicaténaire. Cette température est fonction de la taille et de la composition en base de l'amplicon. Si un produit non spécifique est présent, la chute de la fluorescence présentera 2 paliers correspondant chacun à un amplicon. Seuls sont validés les couples d'amorces et les expériences présentant un seul palier. En parallèle, une réaction de reverse transcription (RT) est réalisée sur 2 ug de RNA afin de valider l'efficacité de la PCR selon les conditions décrites précédemment. Une gamme de dilution en série de ce produit de RT est réalisée et des quantités arbitraires sont portées dans les tableaux de données proposées par le logiciel d'analyse fourni avec l'appareil de PCR quantitative (Stratagène, MXP 3500). Pour chaque dilution, une PCR quantitative est réalisée avec le couple d'amorce à tester et le début de l'amplification exponentielle de l'amplicon est obtenu à un cycle particulier de la PCR. Cette valeur Ct obtenue pour chaque dilution permet d'obtenir une droite Y = aX + b ou Y est la valeur de Ct pour un point donné, X est la quantité initiale de matériel pour chaque dilution et a représente la pente de la droite. L'efficacité E de la PCR est alors donnée par la formule E = 10^{(1/-a)}. Cette efficacité doit être comprise entre 1,8 et 2,2 pour que le couple d'amorce et les conditions de PCR soient validés. Une valeur de 2 correspond à une amplification de 100%.

Afin de permettre une comparaison des variations des niveaux d'expression des différents gènes, il est nécessaire de comparer ces niveaux à celui d'un gène exprimé de façon constante entre les échantillons et les conditions expérimentales. Le gène choisi dans la plupart des expériences menées est le gène RS17 (X07257), gène codant pour une protéine ribosomique. Il a en effet été montré que le niveau d'expression de ce gène ne variait que peu ou pas du tout en fonction de l'état des différentes cellules étudiées. Ainsi, le différentiel observé entre deux valeurs Ct témoigne d'une modulation du niveau d'expression du gène d'intérêt et n'est pas du à une variation dans la quantité de matériel de départ. Le calcul du niveau relatif est donné par la formule : D = 2^((CtX1-CtX2)/(CtR1-CtR2)) dans laquelle :
CtX1 représente le Ct du gène X pour le type cellulaire 1
CtX2 représente le Ct du gène X pour le type cellulaire 2
CtR1 représente le Ct du gène contrôle RS17 pour le type cellulaire 1
CtR2 représente le Ct du gène contrôle RS17 pour le type cellulaire 2.

Le niveau d'expression relatif des gènes par rapport à leur niveau d'expression dans un fibroblaste embryonnaire met en évidence 3 catégories de gènes selon l'intensité de l'expression. On peut identifier des gènes surexprimés à des niveaux très importants et dont le niveau relatif d'expression par rapport à des fibroblastes embryonnaires est supérieur à 100 fois. Ces gènes pourraient être considérés comme 'spécifiques' des cellules CESC. La classe des gènes dont le niveau relatif d'expression est compris entre 10 et 100 comprend actuellement une douzaine de gènes. Enfin, on peut distinguer la classe des gènes dont le niveau relatif d'expression est compris entre 2 et 10 fois par rapport à celui des fibroblastes embryonnaires.

### Exemple 5: Suivi de l'expression de gènes au cours de la différenciation des cellules CESC

Certains des gènes identifiés au cours du criblage semblent présenter une expression spécifique des cellules CESC. Le différentiel d'expression entre les fibroblastes embryonnaires, les cellules CESC et les cellules CEGC indiquent que le niveau d'expression des gènes varie en fonction du type cellulaire. Il apparaît également essentiel de caractériser les variations de niveaux d'expression au cours de la différenciation des cellules CESC.

Des expériences de cinétiques d'induction ont été réalisées, notamment par ensemencement des cellules dans le milieu de différenciation sans facteurs de croissance ni cytokines en présence ou en absence d'inducteur comme l'acide rétinoïque à 10 ⁻⁷M. Les cellules stimulées sont prélevées à différents temps et les niveaux d'expression des gènes sont analysés par QRT-PCR comme décrit précédemment (cf exemple 4). L'analyse des variations de l'expression de certains gènes révèlent des cinétiques différentes selon les gènes.

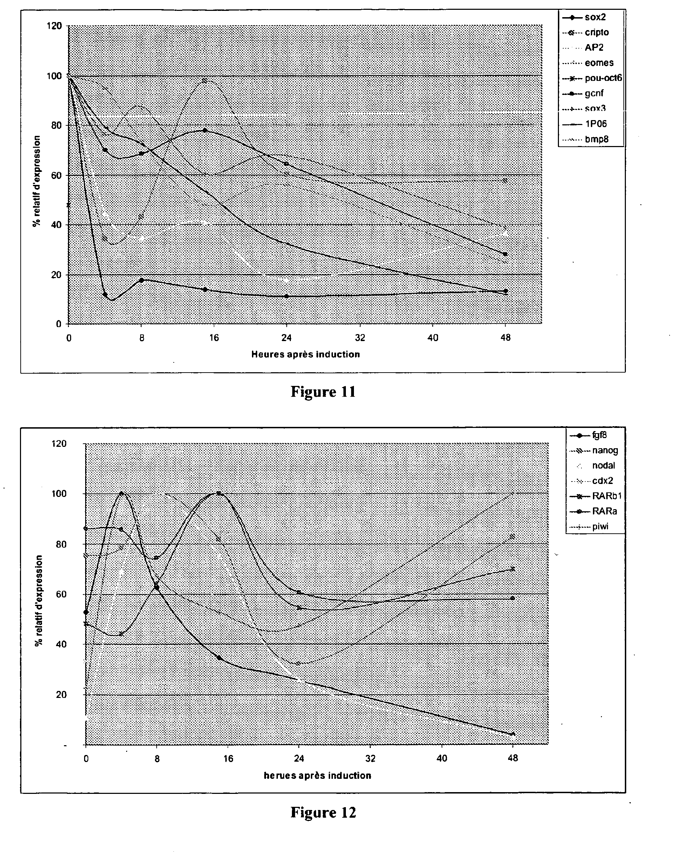

On peut distinguer 3 grandes classes de gènes selon la cinétique des variations de leur niveau d'expression. Dans la classe 1, on trouve les gènes pour lesquels le niveau d'expression est maximum avant induction et qui s'effondre très rapidement avec l'addition d'acide rétinoique dans le milieu de culture (Figure 11). Dans cette classe, on trouve les gènes sox-2, cripto, AP, eomes, oct-6. genf, sox-3, 1P06, GTT3, RARg2, BMP-8, gata-4, etc...

Dans la classe 2, on trouve les gènes pour lesquels l'optimum d'expression est atteint ente 4 et 8 h après l'induction de la différenciation (Figure 12), comme les gènes fFGF-8, nanog, nodal, cdx-2. RARb1, RARa, piwi, TERT, etc...

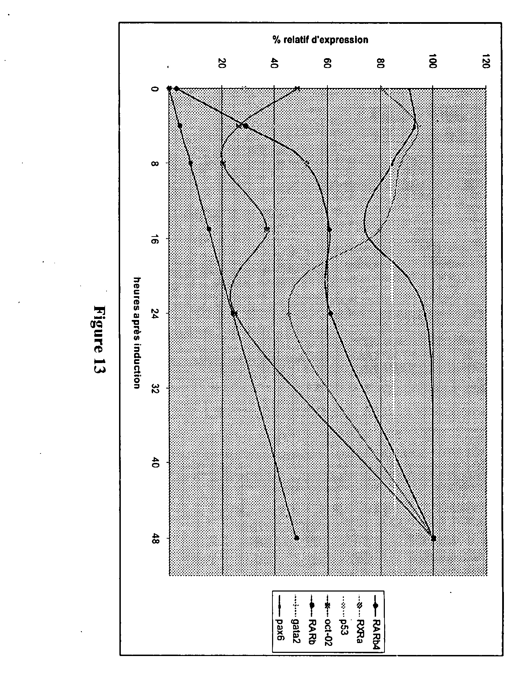

Dans la classe 3, on trouve les gènes pour lesquels, le maximum d'induction est obtenu à partir de 24 après le début de l'induction de la différenciation (Figure 13), en particulier, les gènes piwi, p53. TERT, RXRa, Oct-2, RARb, gata-2, pax-6, etc...

Dans ces successions de variations et d'induction, on notera également la rapidité de variation de certains de ces acteurs comme les gènes cripto/.nodal et FGF-8/BMP-8 dont les variations sont opposées.

### Exemple 6 : Identification des gènes exprimés dans les cellules de blastoderme de stade IX-XII

Les cellules de blastoderme de stade IX à XII sont prélevées comme décrit précédemment (Pain et al., 1996 FR N°) Au regard de la présence de vitellus résiduel, même après préparation et lavages extensifs des embryons, une double méthode de préparation des RNA à partir du culot de ces cellules de blastoderme est réalisée. Le culot cellulaire est congelé et les RNA sont en première étape extraits par une méthode au Trizol (Tri Reagent (Invitrogen cat 15596-018) à raison de 5 ml pour 1 x 10⁶ cellules environ. Après une homogénéisation à la pipette, le mélange est centrifugé 10 min à 400 g à 4 °C puis 200 µl de chloroforme par 1 ml de solution est ajouté. L'ensemble est émulsionné, décanté à température ambiante et l'opération répétée plusieurs fois. Le mélange est alors centrifugé 15 min à 400 g à 4°C et la phase aqueuse transférée dans un nouveau tube. 500 µl d'isopropanol par ml de mélange est ajouté. L'ensemble est alors précipité par centrifugation 15 min à 400g à 4°C, le culot est lavé à l'éthanol 70°C, séché et repris dans 200 à400 µl de tampon de lyse du kit d'extraction RNA kit Quiagen. Cette deuxième étape de préparation / purification des RNA est alors suivie selon les instructions de fournisseur. Cette double extraction donne de meilleurs résultats de reproductibilité dans l'analyse par QRT-PCR du niveau d'expression des gènes.

Les conditions de détection par QRT-PCR sont identiques à celles décrites précédemment (cf. exemple 4)

Le niveau relatif d'expression des gènes dans les cellules de blastoderme de stade X indique que certains gènes sont également très fortement exprimés dans ces cellules comparativement aux fibroblastes embryonnaires.

### Exemple 7 : Identification des gènes impliqués dans la physiologie des cellules germinales aviaires (CGSC).

Afin d'identifier les gènes impliqués dans l'émergence et le maintien des cellules germinales du poulet les stratégies de criblage utilisées pour les cellules CESC ont également été appliquées au niveau des cellules de gonades embryonnaires de poulet.

De façon analogue à l'approche utilisée pour l'étude des gènes des cellules CESC, le niveau d'expression de gènes a été analysé dans les cellules germinales aviaires. La liste des gènes exprimés dans ces cellules permet de mettre en évidence des gènes très fortement exprimés dans ces cellules (ratio de surexpression relatif supérieur à 100 et des gènes dont le niveau d'expression relatif est compris entre 100 et 10. Une grande partie des gènes présente une expression plus modeste avec un ratio compris entre 2 et 10.

### Exemple 8: Analyse de l'expression comparative des gènes entre les différents types cellulaires

En comparant les niveaux relatifs d'expression entre les cellules CESC, les cellules CEGC et les cellules de blastoderme de stade IX-XII, on peut obtenir différentes listes de gènes qui apparaissent comme des gènes marqueurs caractéristiques des différents types cellulaires. (les résultats brut sont donnés en annexe).

### 8.1 Avec les cellules de blastoderme de stade IX-XII

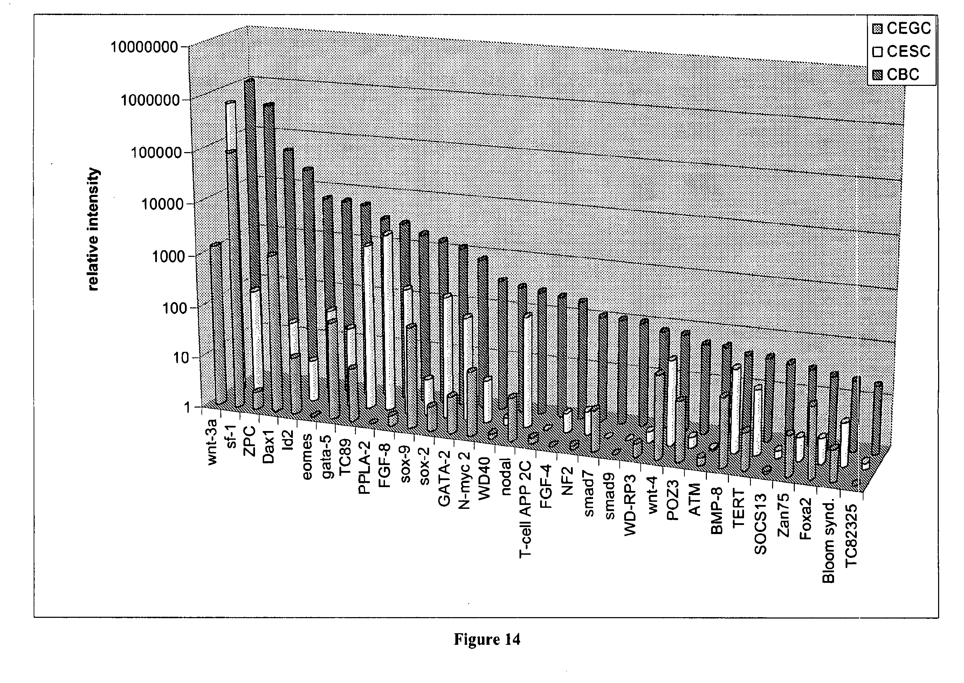

En combinant les résultats précédents des différents profils d'expression, une combinatoire d'expression propre à chaque type cellulaire peut être définie. Ainsi, en comparant les niveaux d'expression entre les cellules de blastoderme du stade X avec les cellules CESC in vitro, les cellules de blastoderme de stade X sur-expriment (Figure 14) :
- les facteurs de croissance comme fgf-8, tgf-b2, fgf-4, wnt-14,
- les acteurs de la signalisation comme smad-9, cis-1, smad-7, id2, smad-4,
- les facteurs de transcription comme sf-1, gata-5, sox-9, sox-2,
- des gènes de structure comme WD40, WDRP-3, le transporteur abcg2,
- des gènes régulateurs comme N-myc2, poz-3, atm.
- d'autres gènes moins caractérisés comme la phosphatase T-cell APP2C, ssb-1, nf-2,
- le gène zpc qui apparaît très spécifique de ce stade de développement.

Cette même comparaison entre les cellules de blastoderme de stade X et les cellules CEGC de gonades embryonnaires indiquent une sur-expression (Figure 14) :
- les facteurs de croissance comme fgf-8, fgf-4, wnt-3a, wnt-11, tgf-b2,
- les acteurs de la signalisation comme id2, smad-9, socs-1, cis-1, smad-7,
- les facteurs de transcription comme eomes, sox-3, sox-2, 1P06, gata-2, gata-5, pax-6, fbx-15b, sox-9,
- des gènes de structure comme WD40, la claudine-1, l'astacine, WDRP-3, abcg2,
- des gènes régulateurs comme atm, N-myc-2, poz-2, poz-3,
- d'autres gènes moins caractérisés comme ens-1, la phosphatase T-cell APP2C, TC893, nf-2, ssb-1, TC823,
- le gène zpc qui apparaît très spécifique de ce stade de développement.

Ainsi, la combinatoire permettant de définir le mieux le stade X comprend les gènes les plus exprimés entre les deux situations:
- _fgf-4, tgf-b2,
- id-2, smad-7, smad-9, snoN, SOCS13,
- Cxcr-4,
- gata-5, N-Cor1,
- atm, bloom syndrome, enx-1, rad54b, rbm6, ssb-1,
- nf-2, N-myc2, pml, T-cell APP2C, WDRP-3,
- cpe1738, cwf16, TC82325, TC87, zan75,
- Zpc.

Pour les cellules de blastoderme de stade X, on peut ainsi noter la présence de nombreux acteurs de signalisation comme les facteurs fgf-4 et fgf-8, les gènes id-2, SOCS13, différentes membres de la famille smad, incluant snoN, agissant pour transmettre les signaux intracellulaires. Même forte, la présence de ces acteurs n'est pas exclusivement spécifique.

### 8.2 Les cellules CESC

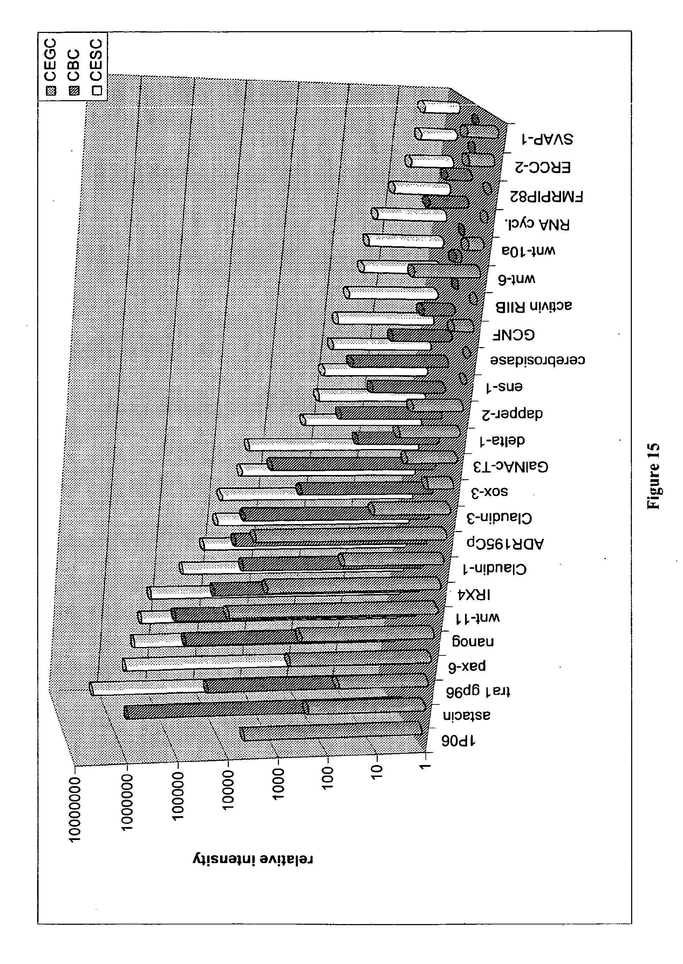

De façon identique, les cellules CESC sur-expriment par rapport aux cellules de blastoderme de stade X (Figure 15)
- les facteurs de croissance comme wnt-10, bmp-5, dapper-1,
- les acteurs de la signalisation comme brap, TGF-RII,
- les facteurs de transcription comme gcnf, TR-a, pax-6, 1 P06, irx-4,
- des gènes de structure comme l'émerine, l'astacine, la claudine-3, la LAMP lectine, svap-1, dorfin,
- des gènes marqueurs comme tra-1, ch-tog, musashi,
- d'autres gènes moins caractérisés comme FTT synthase, CG182, la maturase K, RNA cyclase, FMRPI182.

Et par rapport aux cellules de gonades embryonnaires CEGC (Figure 15) :
- les facteurs de croissance comme wnt-3a, wnt-10, fgf-8, bmp-8, wnt-11, fgf-4, dapper-2,
- les acteurs de la signalisation comme activin RII, brap,
- les facteurs de transcription comme nanog, eomes, sox-3, 1P06, pax-6, gcnf, sox-2, gata-2, fbx-15b, irx-4,
- des gènes de structure comme l'astacine, la claudine-3, la claudine-1,
- des gènes marqueurs comme tra-1, ens-1,
- d'autres gènes moins caractérisés comme TC896, la maturase K, RNA cyclase, FMRPI182.

Ainsi, la combinatoire permettant de définir le mieux les cellules CESC en prolifération comprend les gènes:
- wnt-10a, wnt-11, dapper-1,
- 1 P06, pax-6, gcnf, irx-4, sox-3,
- l'astacine, la claudine-3,
- tra- 1,
- 1P08-A09, slc38a, FMRPI182.

Pour les cellules CESC en prolifération, on peut mentionner la forte proportion de facteurs de transcription dans la liste caractéristique de ces cellules. Notamment, les gènes nanog, 1P06 (oct-3/4- cf infra) et gcnf ont été isolés, clonés et étudiés chez le poulet pour la première fois au cours de cette étude.

De nouveaux gènes ont également été identifiés comme la maturase, le gène de fonction inconnue FMRPI182, dont l'expression est liée à la prolifération, le gène slc38-2 (sans doute un membre de la famille des transporteurs de solutés), 1P08-A09 etc.

### 4.3 Les cellules CEGC

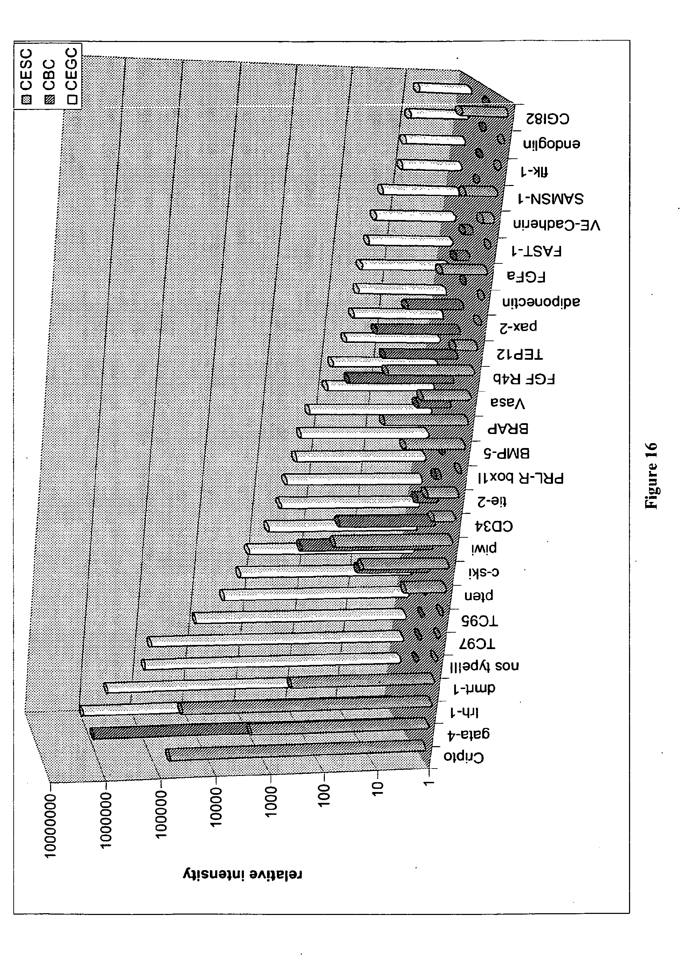

Les cellules de gonades embryonnaires, avec la réserve de l'utilisation du tissu entier (cf supra) sur-expriment par rapport aux cellules de blastoderme de stade X les gènes (Figure 16) :
- les facteurs de croissance comme bmp-5, dkk-3,
- les acteurs de la signalisation comme le récepteur endogline, TGF-RII, FGF-R, pten, wisp-1, smad-3, FGF-R, smarcd-3,
- les facteurs de transcription comme pax-2,
- des gènes de structure comme l'émerine, VE-cadherine-2, HSPb7,
- des gènes marqueurs comme tie-2, piwi, musashi,
- d'autres gènes moins caractérisés comme CG1182, samsn-1, flj00188.

Et par rapport aux cellules CESC en prolifération, les gènes (Figure 16) :
- les facteurs de croissance comme wnt-14, bmp-5, dkk-3,
- les acteurs de la signalisation comme le récepteur endogline, PRL-Rbox1, wisp-1, pten, cis-1, smad-7, smad-9, smad-4, TGF-RII, c-ski, smarcd-3,
- les facteurs de transcription comme lhx-9, sf-1, gata-4, pax-2, dax-1, sox-9,
- des gènes de structure comme abcg-2, VE-cadherine-2,
- des gènes marqueurs comme tie-2, dmrt-1, piwi,
- d'autres gènes moins caractérisés comme TC95, KHKoc-1, flj00188, tep12, samsn-1,
- des gènes marqueurs des cellules germinales comme vasa, piwi.

Ainsi, la combinatoire permettant de définir le mieux les cellules CEGC de gonades embryonnaires comprend les gènes:
- BMP-21K, BMP-5, dkk3,
- Endoglin, FGF R, TGF RII, Wisp-1,
- Gata-4, lhx-9,
- Rab40B, smad3, Smarcd3,
- TC95408, TC97694, FLJ00188,
- tie2, tob.

Ces combinatoires ne sont qu'indicatives et peu de gènes apparaissent très spécifiques d'un seul type cellulaire.

Certains des gènes identifiés présentent des homologues dans d'autres espèces dont les profils d'expression sont restreints aux tissus reproducteurs comme deadend, vasa, wisp-1, tep12, etc...En comparant les profils d'expression communs entre les cellules de blastoderme de stade X et les cellules CEGC (deux types cellulaires avec une compétence germinale), on peut identifier une combinatoire de gènes dont l'expression est faible voir quasi absente des cellules CESC. Cette perte ou cette forte diminution de marqueurs de cellules germinales pourrait être une conséquence de la mise en culture des cellules de blastoderme de stade X. Mais aucune indication n'est possible quant à l'expression de ces gènes pour une condition nécessaire et/ou suffisante à une compétence germinale. Les gènes sont :
- wnt-14,
- Brinp, cis-1, c-ski, pr1-Rbox11, pten, sef, smad-4, smad-5,
- Abcg2, clock, dmrt-1, 1fng, morf,
- Dax-1, pax-2, sf-1, sox-9,
- Deadend, poz-3, slug, Ve-cadhérine-2,
- Emx-2, fragilis4, TC136, tep12, tsc-22,
- Tudor, vasa.

De façon analogue, même avec des variations dans leur niveau d'expression, des gènes sont communs aux cellules CESC et CEGC à l'exclusion relative des cellules de blastoderme de stade X ; Ces gènes sont :
- cripto, BRAP, TGF RIII,
- CGI82, ch-tog, ddx-25, Dorfm, emerin,
- ERCC-2, KHKoc1, LAMP lectine,
- Maturase, musashi, piwi, SAMSN-1, SVAP-1, TC86990,
- TR-alpha.

La même analyse est également réalisée pour les gènes exprimés de façon commune entre les cellules CESC et les cellules de blastoderme de stade X à l'exclusion relative des cellules CEGC/ Ces gènes sont :
- nodal, wnt-3a, wnt-11
- Ras like
- drg-1, ens-1, ews-1
- Eomes, Fbx15b, gata-2, jsd3, pax-6, sox-2, sox-3
- Ddx-28, Mago, nestin, POZ2
- Arg NmethylASE, PP2447, RNA cyclase
- A14TS, CES-c32, TC893, Translin
- Claudin-1, syntaxin 16, WD40, WDFYP-2.

Une analyse des différents gènes qui apparaissent sur-exprimés dans l'une ou l'autre des situations permet de proposer différents modèles pour le maintien de la pluripotence in vitro, le contrôle de la compétence germinale in vivo et in vitro ainsi que l'impact des conditions de culture sur le niveau d'expression de gènes particuliers.

### Cytosquelette

Au niveau des gènes du cytosquelette et qui assurent le maintien de la morphogénèse des cellules CESC, seul un nombre restreint de gènes présentent un différentiel d'expression important intéressant. Notamment un gène présentant un motif WD, le gène WDRP-2 (TC190198) dont le motif impliqué dans les interactions protéines-protéines au niveau du cytosquelette est plus exprimé dans les cellules CESC que dans les CEGC. Le gène WD40 (TC189537) apparaît assez spécifique des cellules de blastoderme de stade IX-XII. On notera que le gène de la beta-catenine est fortement exprimé dans les deux systèmes, suggérant que sa régulation est plus vraisemblablement effectuée au niveau protéique. A l'inverse, le gène Claudin-1 (TC189974) impliqué dans les 'gap junction' et le maintien de fortes associations cellules-cellules est fortement exprimé dans les cellules CESC. Cette observation rejoint l'analyse réalisée par microscopie électronique des cellules CESC pour lesquelles les jonctions intra-cellulaires sont particulièrement importantes et bien structurées. On détecte également l'astacine (TC 221952), enzyme métalloprotéase qui intervient dans le contrôle de la matrice extracellulaire des cellules permettant leur mouvement et les interactions potentielles avec leur environnement.

### Facteurs de croissance

Les cellules CESC et les cellules GESC expriment à des niveaux variables différents membres des grandes familles de facteurs de croissances et de cytokines. Ainsi, de nombreux facteurs de croissance des familles TGF/BMP, de la famille des FGF, de la famille des facteurs de récepteurs tyrosine kinase, de la famille des cytokines dont le signal est transmis par la voie gp130 et de la famille wnt sont exprimés de façon spécifique soit dans les cellules CESC soit dans les cellules GESC.

Parmi les différents acteurs testés, les cellules CESC expriment ainsi les facteurs nodal, FGF-8, wnt-3a, wnt-10a, BMP-8 et FGF-4 beaucoup plus fortement que les cellules CEGC. Ces dernières sont notamment caractérisées par une sur-expression des facteurs wnt-14, TGF-b2 et BMP-5.

On notera que l'expression de nodal a été impliquée dans un ensemble de régulation complexe de la pluripotence comme mentionné pour les cellules ES murines et humaines (Besser, 2004, Vallier et al., 2004).

Le gène cripto, retrouvé à la fois dans les cellules GESC et les cellules CESC est mentionné comme un élément essentiel et une 'signature' des cellules ES humaines (Bhattacharya et al., 2004).

Au niveau de la souris, l'expression du FGF-8 a été impliquée dans la prolifération des PGC, quand celles ci sont parvenues après leur migration dans les gonades (Kawase et al., 2004). La forte expression de FGF-8 dans les cellules CESC pourrait être un bon indicateur d'une 'nature' PGC de ces cellules, mais le niveau beaucoup plus élevé que dans les cellules GESC pourrait être le signe d'un dérèglement de la voie d'activation de ce facteur. De la même façon, le FGF-4 apparaît être exprimé environ 5 x plus dans les cellules CESC que dans les cellules GESC. On notera que le FGF-4 a été identifié chez la souris comme une cible privilégiée du facteur de transcription Oct-3/4 (Dailey et al., 1994 ; Ambrosetti et al., 2000) et est un acteur essentiel dans les cellules MESC à la fois in vitro et in vivo (Yuan et al., 1995, Feldman et al., 1995, Wilder et al., 1997).

La situation des deux facteurs BMP est également particulière notamment par la forte implication des facteurs BMP-4 et BMP-8 dans le contrôle de la pluripotence des cellules MESC (Ying et al., 2001) et dans l'émergence et le maintien des cellule germinales murines *in vitro* (Hubner et al., 2003 ; Geijsen et al., 2004) et *in vivo* (Saitou et al., 2002). La destruction de l'expression du BMP-4 par perte de fonction chez la souris aboutit à la mort précoce de l'embryon (8-9 jours de développement) par l'absence de formation des dérivés mésodermiques (Winnier et al., 1995). Dans ces embryons, aucune PGC n'est détectée (Lawson et al., 1999). Cette absence ne peut être compensée par le BMP-8. Le BMP-4 est lui même nécessaire à l'induction précoce à partir de l'épiblaste. La destruction de l'expression du gène BMP-8b a pour conséquence une diminution de la prolifération des cellules germinales et une diminution de l'entrée des cellules en méiose (Zhao et al., 1996, Zhao et al., 1998).

Pour le gène sdf-1, l'expression du messager de ce facteur est corrélée avec celle de son récepteur, CXCR4, également détectée de façon spécifique dans les gonades, même si les cellules positives dans l'ensemble du tissu ne sont pas identifiées. Les cellules CESC ne présenteraient pas cette propriété d'exprimer le facteur ou le récepteur assurant le guidage des cellules germinales car ces deux acteurs sont fortement impliqués dans le contrôle de la migration des cellules germinales, comme cela a été montré à la fois chez le poisson zèbre (Doitsidou et al., 2002, Knaut et al., 2003), la souris (Ara et al., 2003) et plus récemment chez le poulet (Stebler et al., 2004). Il serait ainsi possible de modifier le tropisme des cellules CESC en exprimant par exemple le récepteur CXCR4 dans les cellules CESC pour obtenir une colonisation gonadique plus efficace. La forte expression de CXCR-4 dans les cellules de blastoderme de stade IX-XII est un élément important dans le contrôle de la migration des cellules au sein de l'embryon précoce.

Un autre acteur important est le gène deadend, impliqué dans ce processus de colonisation germinale (Weidinger et al., 2003) et dont la mutation *ter* compromet le développement des cellules germinales (Youngren et al., 2005). Ce gène est plus exprimé dans les cellules de gonades que dans les cellules CESC.

Les niveaux d'expression des autres facteurs et récepteurs testés ne sont pas aussi spécifiques des seules cellules CESC ou des cellules GESC.

### Récepteurs membranaires

Les messagers des récepteurs aux TGFb/BMP et aux FGF sont détectés dans l'ensemble des types cellulaires testés y compris les CEF. Si l'importance physiologique de ces variations n'est pas connue, il ne semble pas en première approche qu'un réel déficit de signalisation soit observable au niveau de ces récepteurs, sous réserve que le niveau des RNA détectés soit un bon indicateur du niveau protéique. On détecte néanmoins fortement exprimés dans les cellules CESC le récepteur de l'activine IIB (U31223), sans doute en relation avec la forte expression de nodal et de cripto dans ces cellules.

Les cellules CEGC expriment les récepteurs FGF-R (U48395), TGF-RII (AF202991) et TGF-RIII (L01121) dans de fortes proportions comparativement aux cellules CESC. Les profils d'expression des différents gènes des familles des récepteurs tyrosine kinase ne semblent pas être très variables, même si le nombre de gènes analysés n'est pas exhaustif.

De façon notable, les niveaux d'expression de c-kit, récepteur au SCF est très faible dans les cellules CESC, ce qui contraste avec l'expression observée dans les cellules des gonades embryonnaires. Or, l'importance du SCF dans le maintien de la lignée germinale est également démontrée. Ce niveau de c-kit serait ainsi un bon indicateur de la nature germinale des cellules, même si le récepteur c-kit est exprimé dans de nombreux autres tissus (hématopoièse, cellules nerveuses, ....).

Au niveau de la voie de signalisation du LIF, on retrouve exprimés le récepteur LIF-R et la protéine gp130, ceux ci sont également détectés dans les fibroblastes (Duong et al., 2002; Geissen et al., 1998). Le facteur LIF aviaire, récemment cloné (Horiuchi et al., 2004) est quant à lui détecté uniquement dans les fibroblastes, ce qui est conforme à la production de cette cytokine dans les autres espèces et ce qui suggère que les cellules CESC ne semblent pas présenter de production autocrine dans les conditions de culture utilisées.

### Signalisation

Au niveau des voies de signalisation, la plupart des acteurs des voies d'activation des récepteurs tyrosine kinase, des récepteurs TGFb/BMP et de la voie gp130 jak/STAT sont exprimés de façon généralement ubiquitaire sans grande variation entre les différents types cellulaires. Néanmoins, quelques observations sont à faire. Le gène PTEN (BM486819) est trouvé fortement exprimé dans les cellules CEGC ainsi des gènes de la famille à domaine POZ comme POZ3 (TC216137). Des gènes comme CIS-1 (TC216000), SSB-1 (TC210358), SOCS13 (TC217588), etc.. ; sont impliqués dans les signalisations par les différents récepteurs ainsi que de nombreux gènes impliqués dans la voie des GTPases. Les gènes Smad-3 (TC187835), Smad-6 et smad-7 (TC194136), connus pour leur action régulatrice de l'action des TGF/BMP sont également plus exprimés dans les cellules CEGC que dans les cellules CESC, même si on retrouve une forte expression dans les CEF. Les gènes Smad-1 (Tremblay et al., 2001) et Smad-5 (TC209770)(Chang et al., 2001) connus pour être fortement impliqués dans le déterminisme des cellules germinales chez la souris sont respectivement plus exprimés dans les cellules CESC et dans les CEGC. Une expression dans les fibroblastes est également détectée. Le gène Smad-4 (TC207213) est préférentiellement exprimé dans les CEGC et n'est pas ou peu détectable dans les CESC et dans les CEF. Ce gène Smad-4 apparaît dans beaucoup de situations comme un gène clef de la signalisation des membres de la famille TGFb/BMP, car indispensable au contrôle transcriptionnel des gènes dépendants de ces facteurs. Parmi les nombreux défauts liés à l'inactivation du gène smad-4 chez la souris, on trouve une absence de formation des PGC (Chu et al., 2004) très similaire à celle observée dans les mutants BMP-4 (Lawson et al., 1999) et BMP-8b (Ying et al., 2000 ; Loebel et al., 2003). Les gènes c-ski (TC219247) et SNoN (TC215069) présentent également des différentiels d'expression spécifiques et participent à ces contrôles de prolifération et de différenciation de ces cellules dans la voie germinale. Les niveaux d'expression de ces différents acteurs de la transmission du signal de la famille TGF/BMP peuvent représenter des indicateurs précieux de la nature 'cellule souche' ou 'cellule germinale' des cellules étudiées. Toute variation dans les niveaux d'expression pourra être mise à profit pour mieux cerner les évolutions induites par les différentes manipulations des gènes candidats. Ces différents gènes eux mêmes, notamment Smad-4, sont des candidats très importants dans le contrôle du potentiel germinal d'une cellule CES. Il sera donc particulièrement judicieux de tester l'effet ou la destruction de ces différents gènes à la fois *in vitro* et *in vivo.*

Le gène ras-like (TC201177) présente également une expression plus spécifique dans les cellules CESC que dans les cellules CEGC ainsi que dans les cellules de blastoderme de stade IX-XII. Le gène orthologue murin a été identifié récemment comme un acteur majeur de la prolifération des cellules MESC (Takahashi et al., 2003) et pourrait être responsable du caractère 'tumorigène de certaines lignées cellulaires par la voie P13 kinase. Mais les cellules MESC mutantes pour ce gène sont capables de coloniser la lignée germinale et aucune conséquence sur la descendance ne se manifeste jusqu'à présent. Le niveau d'expression dans les cellules germinales n'est pas documenté et une inhibition d'expression dans les cellules MESC a un effet négatif sur la vitesse de prolifération. Une hypothèse pourrait être que la diminution nécessaire de la vitesse de prolifération (via l'inhibition de ce gène) serait un préalable à la différenciation des PGCs.

Les autres gènes testés ne semblent pas présenter un déséquilibre majeur dans leur niveau d'expression. La régulation de l'activité d'autres acteurs de la signalisation comme les protéines Stat, MAP kinase, erk, etc.... est sans doute plus effectuée au niveau protéique, notamment par les mécanismes d'activation liées aux phosphorylations /déphosphorylations. Cela a été bien démontré pour la protéine STAT-3 dans les cellules MESC.

Au niveau des facteurs des protéines nucléaires, la situation apparaît complexe, au regard du nombre important de gènes candidats. Pour les facteurs de transcription, schématiquement, différentes familles de gènes semblent particulièrement impliquées comme celle des récepteurs nucléaires d'hormones (HNR), celle des gènes sox et des gènes gata.

L'implication de certains des gènes est directement liée au contrôle épigénétique de l'expression des gènes. La connaissance des mécanismes épigénétiques au cours du développement du poulet ou dans le modèle aviaire se limite aux découvertes pionnières des mécanismes d'expression des gènes de globine, incluant l'identification de séquences isolatrices (Felsenfeld, 1993) et à des études sur le contrôle transcriptionnel du gène du lysosyme (Kontaraki et al., 2000). Par analogie avec les autres systèmes mammifères ou eucaryotes inférieurs, différents gènes poulet impliqués dans le contrôle épigénétique de l'expression des gènes ont été identifiés. Parmi ceux pour lesquels un différentiel fort d'expression est observé, on trouve le gène dmrt-1 plus fortement exprimé dans les gonades embryonnaires et les gènes DCSM, enx-1 1 plus exprimés dans les cellules de blastodermes. L'expression du gène dmrt-1 pourrait être spécifique des cellules de Sertoli comme dans le modèle murin (Lei et al., 2004), mais la détection de ce gène dans les fractions des cellules germinales module le commentaire. D'autres gènes présentent des profils d'expression intéressants mais sont également exprimés dans les fibroblastes.

### Gènes de la télomérase

Les cellules souches et les cellules souches embryonnaires présentent une activité télomérase intrinsèque, en particulier, les cellules CESC (Pain et al., 1996). Le gène de la télomérase poulet vient d'être cloné (Delany et al., 2004 ; Swanberg et al., 2004) et le niveau d'expression dans les cellules CESC apparaît important.

### Gènes de la famille sox

Pour les gènes sox, l'expression du gène sox-9 (U12533) est plus spécifique des cellules CEGC, ce qui semble conforme à l'importance du rôle de ce gène dans la formation testiculaire de la souris (Chaboissier et al., 2004, Vidal et al., 2001), mais pourrait être exprimé préférentiellement dans les cellules de soutien. La conservation du rôle de ce gène entre les mammifères et les oiseaux est évoquée (Morais da Silva et al., 1996) et ce gène serait une cible directe du récepteur nucléaire sf-1 (Sekido et al., 2004) (NM 205077), acteur également trouvé fortement exprimé dans les CEGC en présence de Dax-1 (AF202991)

Parmi les gènes les plus spécifiques des cellules CESC, on mentionnera les deux gènes sox-2 (U12532) et sox-3 (U12467). Il a été démontré de fortes relations d'associations des protéines SOX-2 et OCT-3/4 chez la souris au niveau de certains promoteurs (Miyagi et al., 2004 ; Nishimoto et al., 1999), notamment au niveau du promoteur du FGF-4 (Dailey et al., 1994; Ambrosetti et al., 2000). Le gène 1P06, homologue aviaire du gène Oct-3/4 mammifère (cf infra exemple 9) est très fortement exprimé dans les cellules CESC et apparaît comme un des meilleurs marqueurs de ces cellules.

### Gènes de la famille gata

Les gènes gata montrent également des profils d'expression particuliers. Le gène gata-4 ( U11887) est exprimé de façon préférentielle dans les cellules de gonade embryonnaires, expression peut être liée à la présence des cellules de Sertoli (Imai et al., 2004, Lavoie et al., 2004). Les gènes gata-2 (X56930) et gata-5 (U11888) sont plus exprimés dans les cellules CESC, avec notamment une forte sur-expression du gène gata-5 dans les cellules de blastodermes. Le messager du gène gata-2 a été détecté dans les cellules germinales (Siggers et al., 2002) et au niveau de l'embryon précoce de poulet (Sheng et al., 1999). L'action pléiotropique des gènes gata est modulée et contrôlée par l'association avec différents partenaires au niveau d'éléments de réponse proches et/ou communs dans les promoteurs des gènes cibles. Ainsi, si un des partenaires privilégiés dans le système hématopoiétique est un des gènes de la famille ets, les combinaisons sont différentes dans d'autres systèmes de prolifération et de différenciation. Ainsi des éléments de réponse aux gènes gata sont identifiés avec des éléments de réponse aux BMP (les éléments BRE) dans le promoteur du gène smad-7 (Benchabane et al., 2004). Ce gène smad-7 est également plus exprimé dans les cellules de gonade embryonnaires que dans les cellules CESC.

Les gènes de la famille Id apparaissent comme des acteurs incontournables du contrôle de l'action des BMP. Le gène Id2 semble plus exprimé dans les cellules de gonades embryonnaires et surtout dans els cellules de blastoderme que dans les cellules CESC. Ce gène pourrait être au centre de la régulation de la prolifération/ différenciation des cellules CESC, dans une approche similaire au modèle murin (Ying et al., 2003, Kowanetz et al., 2004).

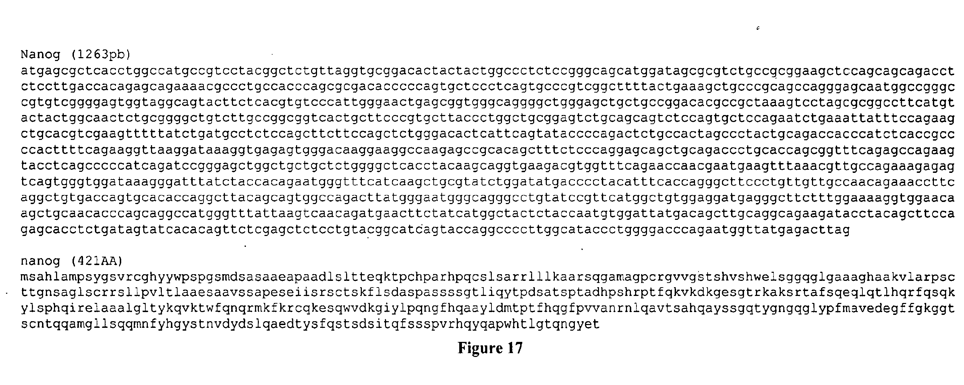

Dans ces différents processus décrits dans la littérature, on retrouve fréquemment le partenaire YY1 (Kurisaki et al., 2003) (trouvé également fortement exprimé dans les cellules CESC, mais dont le niveau d'expression est relativement ubiquitaire) et un acteur de la famille NKx comme dans le cas de la différenciation cardiaque (Lee et al., 2004). Or, le gène nanog, actuellement identifié exclusivement chez les mammifères appartient à cette famille de gène Nkx (Chambers et al., 2003). Par recherche d'homologie au niveau de la séquence publiée du génome de poulet, le gène nanog poulet a été identifié (Figure 17). La fonction de cet acteur est encore largement inconnue dans le système souris. La protéine ENS-1, identifiée dans l'approche de piégeage de gène (Acloque et al., 2004) présente également un profil d'expression particulièrement intéressant avec une très forte sur-expression dans les cellules CESC et une disparition rapide de son messager quand les cellules sont induites à différencier (Acloque et al.,, 2001).

Si l'homologue du gène utf-1 connu pour être une cible du complexe Oct-3/4/sox-2 (Nishimoto et al., 1999) n'a pas encore été identifié chez le poulet, on mentionnera que dans des expériences de transfection, les promoteurs des gènes oct-3/4, sox-2 et utf-1 souris sont spécifiquement activés dans les cellules CESC suggérant que les mécanismes de régulation de l'expression de ces différents acteurs sont conservés et fonctionnels dans les cellules aviaires CESC (cf exemple 12). Cette transactivation n'est pas détectée dans un fibroblaste embryonnaire de poulet.

### Récepteurs nucléaires d'hormones

Pour les récepteurs nucléaires d'hormones, le gène dax-1 apparaît très frotement surexprimé dans les cellules de blastodermes. Ce gène est connu pour avoir un rôle négatif (Swain et al., 1998) mais essentiel (Meeks et al., 2003) sur la différenciation germinale, notamment par son action antagoniste sur celle du gène sf-1 (Crawford et al., 1998). Ce gène dax-1 pourrait être lui même contrôlé positivement par sf-1 et négativement par COUP-TF (Yu et al., 1998). L'expression de dax-1 serait également dépendante dans certains systèmes de l'activation par wnt-4 (Mizusaki et al., 2003). Or wnt-4 est un des facteurs trouvés plus exprimé dans les gonades que dans les cellules CESC. Les gènes codant pour les protéines associées aux HNR sont également représentés et présentent des différentiels particuliers, comme le gène N-Cor1 (TC201157).

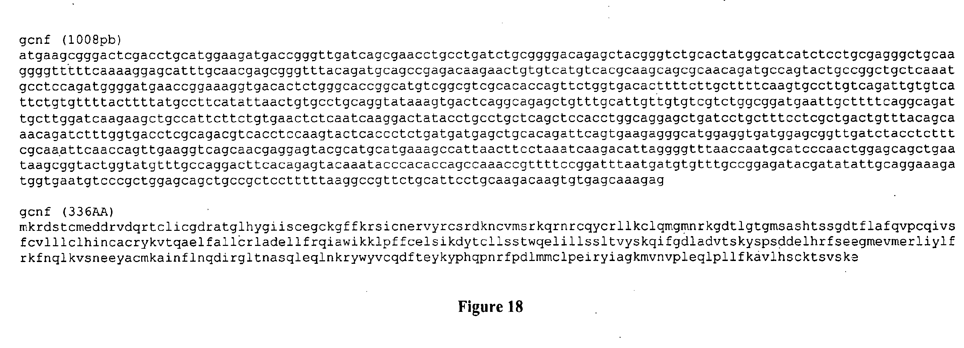

Le gène gcnf dont nous avons déterminée l'intégralité de la séquence (Figure 18) est trouvée très fortement et spécifiquement exprimé dans les cellules CESC et son action antagoniste de sf-1 au niveau du promoteur du gène Oct-3/4 est documentée chez la souris (Furhmann et al., 2001).

Le gène sf-1 est trouvé presque exclusivement exprimé dans les cellules de gonades embryonnaires gonades. Or, sf-1 et lrh sont deux homologues de FTZ-F1, dont le rôle dans le déterminisme des gonades et dans le développement précoce est documenté (Kudo et al., 1997 ; Fayard et al., 2004). Un niveau de régulation supplémentaire et un lien possible entre les actions des récepteurs nucléaires d'hormones et les autres partenaires, (comme sox-9 par exemple) pourraient se faire via la sumoylation. L'importance de cette modification post traductionnelle dans le contrôle de la transcription et de l'intégrité du génome ne cesse de croître (Muller et al., 2004 ; Seeler et al., 2003). La sumoylation modifie à la fois la répartition cellulaire et les interactions entre facteurs (Chen et al., 2004 ; Komatsu et al., 2004). D'autres récepteurs connus pour être impliqués dans le déterminisme des cellules germinales de souris comme ERR (Mitsunaga et al., 2004) ne semblent pas présenter dans le système étudié de fortes variations de leur niveau d'expression.

Les récepteurs RAR, RXR, TR, sont plus particulièrement impliqués au cours de la différenciation des cellules. Leur cinétique d'expression et les variations de leur niveau d'expression ont été analysées dans l'exemple 5.

### RNA binding proteins

Parmi les protéines impliquées dans le contrôle de la transcription de gènes dans les cellules germinales, on retrouve des protéines qui fixent les RNA, les 'RNA binding proteins'. Ces protéines ont notamment été identifiées chez la drosophile et interviennent dans le contrôle de la polarité des embryons (Huynh et al., 2004a) et du déterminisme des cellules germinales. Dans une autre espèce modèle, C. elegans, l'hypothèse de la préformation des cellules germinales repose également et schématiquement sur l'existence et le rôle de ces protéines. Mais, dans ces deux modèles, le contrôle de l'activation par phosphorylation de la RNA polymerase **II,** activation nécessaire à la transcription, n'est pas réalisé par ces 'RNA binding proteins' (Seydoux et la., 1997). Au niveau des mammifères, il a été montré que quelques uns de ces gènes sont également essentiels au maintien de la lignée germinale chez la souris (Wang et al., 2004 ; Tsuda et al., 2003 ; Tanaka et al., 2000). Mais l'origine des cellules germinales murines semble plus liée à un phénomène d'induction par différents acteurs liés à l'environnement cellulaire tridimensionnel et à l'action de facteurs comme les BMP-4 et BMP-8. L'ensemble de ces inductions contrôle au final les niveaux d'expression de différents acteurs majeurs comme Oct-3/4 (Saitou et al., 2003). Cette origine inductive semble renforcée par l'obtention *in vitro* de cellules germinales à partir de la différenciation de cellules MESC de souris (Hubner et al., 2003, Geijsen, et al., 2004, Toyooka et al., 2003).

Au niveau du poulet, la théorie de la préformation a été revivifiée par l'identification de cellules vasa positives dès les stades précoces de l'embryon de poulet (Extavour et al., 2003 ; Tsukenawa et al., 2000). Dans cette hypothèse, le rôle de certaines 'RNA binding proteins' pourrait être primordial.

Sur la soixantaine de gènes de cette famille dont le niveau d'expression a été testé, on détecte les gènes bruno (AB050497), tudor (TC213378), nanos (TC223629), vasa (AB004836) comme beaucoup plus fortement exprimés dans les cellules CEGC que dans les CESC, mais aussi dans les cellules de blastoderme, correspondant sans doute à la présence de cellules compétentes au niveau germinale dans ces cellules. Les cellules CESC sont positives pour le gène vasa, mais présentent une très faible contribution à la lignée germinale dés qu'elles sont maintenues en culture. Aussi, le niveau d'expression relatif apparaît être un élément essentiel à cette compétence germinale. Certains comme le gène nanos présente une spécificité d'expression quasi exclusive pour les cellules CEGC. Le gène mago présente une expression plus importante dans les cellules de blastoderme comparativement aux deux types cellulaires CESC et CEGC. On mentionnera également les gènes caractérisée par une 'Dead box', motif caractéristique des protéines RNA helicase capables de lier les RNA (Rocak et al., 2004). Le gène de la ddx 28 (TC211748) est bien exprimé dans les cellules CESC ainsi que l'homologue de la RNA cyclase (TC214888) De nouvelles molécules homologues de protéines de drosophile et/ou de C. elegans sont encore à identifier, notamment les homologues de oskar, pumilio, aubergine, maelstrom etc.... Au niveau des mammifères, le rôle de ces 'RNA binding' protéines dans le contrôle de la nature des cellules germinales n'est pas connu, mais il semble, d'après les résultats obtenus chez la drosophile, que toute perturbation de l'activité de ces gènes conduit à une perte de la lignée germinale. Les protéines semblent agir sur le contrôle de la localisation cytoplasme/noyau des messagers qu'ils reconnaissent et donc sur l'efficacité de traduction de ces messagers cibles (Huynh et al., 2004 ; Yano et al., 2004 ; Hachet et al., 2004, Palacios et al., 2004). Les mécanismes d'action de ces protéines et de leur RNA ne cessent de se complexifier, notamment par une régulation possible du niveau d'expression de ces messagers par les RNAi et le complexe RISC 'RNA-induced Silencing complex' (Tomari et al., 2004).

Le gène *piwi,* impliqué dans les divisions asymétriques et dans le maintien de la lignée germinale de nombreuses espèces (Cox et al., 1998 ; Kuromochi et al., 2004) est plus exprimé dans les cellules CEGC que dans les CESC. Les cellules de blastoderme apparaissent exprimé moins piwi que les cellules CESC et CEGS. Ce gène piwi appartient à la famille des protéines Argonaute et est un des composants essentiels du complexe RISC. Il contient un domaine PAZ qui pourrait lier les RNA et agir comme une endonucléase au niveau des mRNA dans le duplex de dégradation siRNA/mRNA (Song et al., 2004 ; Tahbaz et al., 2004). Par ailleurs, le niveau d'expression du gène *dicer,* qui assure la production des siRNA et qui a également été identifié chez le poulet (Fukagawa et al., 2004) semble équivalent dans les cellules CESC et CEGC.

### Autres gènes

Parmi les autres gènes qui présentent de forts différentiels d'expressions elon les types cellulaires comparés, on peut noter la présence de tra-1 (NM 204289), spécifique des cellules CESC et des cellules de blastodermes, le gène ens-1, décrit précédemment (Acloque et al ;, 2001), comme un marqueur ds cellules CESC, le gène fbx15 et fbx15b (Tokuzawa et al ;, 2003), gènes à boite T-box impliqués dans la physiologie des cellules MESC, mais dont le rôle demeure inconnu, le gène à homeobox iroquois IRX4, de la famille des facteurs de transcription Nkx (Houweling et al., 2001). De nombreux autres gènes présentent un différentiel d'expression.

### Gènes inconnus

Des gènes dont la fonction est actuellement complètement inconnue présentent un différentiel d'expression entre les différents types cellulaires testés. On mentionnera ainsi les gènes TC976 (TC227369), TC954 (TC203410), FLJ00188 (TC 196697) sur-exprimés dans les cellules CEGC, les gènes TC869 (TC192927), 1P08-A09 (TC189639), TC823 (TC209084), slc38a2 (TC187360), TC896 (TC196399), FPPsynthase (TC211235), TC874 (TC193590) et CES-c32 (TC83694) sur-exprimés dans les cellules CESC. On mentionnera les gènesCPE1738 (TC214741) plus exprimés dans les cellules de blastodermes. Le gène TC976 présente une homologie partielle avec la protéine ASB-6 de souris contenant un motif ankyrin et une boite SOCS (Suppressor of Cytokine signaling') (Wilcox et al., 2004, Kim et al., 2004). Ces gènes ASB sont souvent exprimés dans les cellules germinales et le gène ASB14 est trouvé exprimé préférentiellement dans els cellules CESC et les cellules CEGC mais peu dans les cellules de blastodermes. Une des hypothèses de la fonction de ces gènes est que le domaine SOCS interviendrait dans la compartimentation germinale spécifique de certains messagers et favoriserait leur dégradation dans le soma, (DeRenzo et al., 2003). Ce mécanisme existerait en parallèle et peut être de façon complémentaire à celui exercé par les 'RNA binding protein'.

### Références

- Acloque et al., (2001) Mech Dev. 103 : 79-91.
- Acloque et al., (2004) Nucleic Acids Res. 32 : 2259-71.
- Aida M et al. (2004) Cell 119 : 109-20.
- Allen JD et al. (2002) C. Mol Cancer Ther. 1 : 417-425.
- Ambrosetti DC et al. (2000) J Biol Chem. 275 : 3387-97.
- Anderson R et al. (2000) Mech Dev. 91 : 61-8.
- Ara T et al. (2003) Proc Natl Acad Sci USA. 100 : 5319-23.
- Arai F et al. (2004) Cell 118 : 149-61.
- Barth JL et al. (1998) Exp Cell Res. 238 : 430-8.
- Benchabane H, Wrana JL. (2003) Mol Cell Biol. 23 : 6646-61.
- Bcsser D. (2004) J Biol Chem. 279 : 45076-84.
- Bhattacharya et al., (2004). Blood 103 : 2956-64.
- Biniszkiewicz D et al. (2002) Mol Cell Biol. 22 : 2124-2135.
- Blackwell TK. (2004) Curr Biol. 2004 Mar 23;14(6):R229-30.
- Blanpain C et al. (2004) Cell 118 : 635-48.
- Boeuf H et al. (1997) J Cell Biol. 138 : 1207-17.
- Buaas FW et al. (2004) Nat Genet. 36 : 647-52.
- Cai J et al.( 2004) Exp Hematol. 32 : 585-598.
- Carsience RS et al. (1993) Development 117 : 669-75.
- Cartwright P et al. (2005) Development 132 : 885-96.
- Castrillon DH er al. (2000) Proc Natl Acad Sci USA. 97 : 9585-90.
- Chaboissier MC et al. (2004) Development 131: 1891-901.
- Chambers 1 et al. (2003) Cell 113 : 643-55.
- Chambers 1, Smith A.(2004) Oncogene 23 : 7150-60.
- Chang 11, Matzuk MM. (2001) Mech Dev. 104 : 61-7.
- Charge SB, Rudnicki MA. (2004) Physiol Rev. 84 : 209-238.
- Chen WY et al. (2004) J Biol Chem. 279 : 38730-5.
- Chu GC et al. (2004) Development 131 : 3501-12.
- Cossu G, Bianco P. (2003) Curr Opin Genet Dev. 13 : 537-42.
- Costoya JA et al. (2004) Nat Genet. 36 :653-9.
- Coumoul X et al. (2004) Nucleic Acids Res. 32 : 85.
- Cox DN et al. (1998) Genes Dev. 12 : 3715-27.
- Crawford PA et al. (1998) Mol Cell Biol. 18 : 2949-56.
- Daheron L et al. (2004) Stem Cells. 22 : 770-778.
- Dailey L et a. (1994) Mol Cell Bio. 14 : 7758-69.
- Damiola F et al. (2004) Oncogene 23 : 7628-43.
- Dani C et al. (1998) Dev Biol. 1998 Nov 1;203(1):149-62.
- De Felici M et al. (1993) Dev Biol. 157 : 277-80.
- Delany ME, Daniels LM.(2004) Gene 339:61-9.
- DeRenzo C et al. (2003) Nature 424 : 685-9.
- Doitsidou M et al. (2002) Cell 111 : 647-59.
- Duong CV et al. (2002) Development 129 : 1387-96.
- Evans MJ, Kaufman MH. (1981) Nature 292 : 154-6.
- Extavour CG, Akam M. (2003) Development 130 : 5869-84.
- Eyal Giladi & Kovak (1976).
- Fabioux C et al. (2004) Biochem Biophys Res Commun. 320 : 592-8.
- Fayard E et al. (2004) Trends Cell Biol. 14 : 250-60.
- Feldman B et al. (1995) Science 267 : 246-9.
- Felsenfeld G. (1993) Gene. 135 : 119-24.
- Fuhrmann G et al. (2001) Dev Cell. 1: 377-87.
- Fujiwara Y et al. (1994) Proc Natl Acad Sci USA. 91 : 12258-62.
- Fukagawa T et al. (2004) Nat Cell Biol. 6 : 784-91.
- Furhmann Get al., (2001) Dev Cell. 1 :377-87.
- Galli R et al. (2000) Nat Neurosci. 3 : 986-991.
- Gaudet F et al. (2004) Mol Cell Biol. 24 : 1640-168.
- Geijsen N et al. (2004) Nature 427 : 148-54.
- Geissen M et al. (1998) Development 125 : 4791-801.
- Ginis 1 et al. (2004) Dev Biol. 269 : 360-380.
- Goodell MA et al. (1997) Nat Med. 3 : 1337-45.
- Grabarek JB et al. (2003) Biotechniques. 2003 Apr; 34(4) : 734-6, 739-44.
- Gu P et al., (2005) Mol Cell Biol. 25 : 8507-19.
- Ha JY et al., (2002) ; Theriogenology.58 : 1531-9.
- Hachet O, Ephrussi A. (2004) Nature 428 : 959-63.
- Hamada H et al. (2005) Cancer Sci. 96 : 149-156.
- Hamburger& Hamilton (1951.)
- Hattori N et al. (2004) Genome Res. 14 : 1733-1740.
- Hogan B et al. (1994). Isolation, Culture and Manipulation of Embryonic Stem cells. In Manipulating the Mouse Embryo. A laboratory Manual, Second Edition, pp253-291. CSHL Press.
- Hong Y et al. (1996) Mech Dev. 60 : 33-44.
- Hong Y et al. (2004) Mech Dev. 121 : 933-43.
- Horiuchi H et al. (2004) J Biol Chem. 279 : 24514-20.
- Houweling AC et al. (2001) Mech Dev. 107 : 169-174.
- Hubner K et al. (2003) Science300 : 1251-6.
- Humphrey RK et al. (2004) Stem Cells 22 : 522-30.
- Huynh JR et al. (2004 Dev Cell. 6: 625-35.
- Imai T et al. (2004) Mol Cell Endocrinol. 214 : 107-15.
- Ivanova NB et al. (2002) Science 298 : 601-4.
- Jiang Y et al. (2002) Exp Hematol. 30 : 896-904.
- Jiang Y et al. (2002) Nature 418 : 41-49.
- Karagenc L et al. (1996) Dev Genet. 19 : 290-301.
- Karagenc L, (2000). Poult Sci. 79 : 80-5.
- Katahira T, Nakamura H. (2003) Dev Growth Differ. 45 : 361-7.
- Kawase E et al. (2004) Mol Reprod Dev. 68 : 5-16.
- Kehler J et al. (2004) EMBO Rep. 5.
- Kim KS et al. (2004) Zygote 12 : 151-6.
- Knaut H et al. (2002) Curr Biol. 12 : 454-66.
- Knaut H et al. (2003) Nature 421: 279-82.
- Komatsu T et al. (2004) Mol Endocrinol. 18 : 2451-62.
- Komiya T et al. (1994) Dev Biol. 162 : 354-63.
- Kontaraki J et al. (2000) Genes Dev. 14 : 2106-22.
- Koshimizu U et al. (1995) Dev Biol. 168 : 683-5.
- Kowanetz M et al., (2004) Mol Cell Biol. 24 : 4241-54.
- Krovel AV, Olsen LC. (2004) Dev Biol. 271 : 190-7.
- Kudo T, Sutou S. (1997) Gene 197 : 261-8.
- Kunath T et al. (2003) Nat Biotechnol. 2003 May;21 (5):559-61. Epub 2003 Apr 7.
- Kuramochi-Miyagawa S et al. (2004) Development 131 : 839-49.
- Kurisaki K et al. (2003) Mol Cell Biol. 23: 4494-510.
- Kuromochi et al., 2004.
- Lakshmipathy U, Verfaillie C. (2005) Blood Rev. 19 : 29-38.
- Lasko PF, Ashburner M. (1988) Nature 335 : 611-7.
- Lassalle B et al. (2004) Development 131 : 479-487.
- Lassalle B et al. (2004) Development 131 : 479-87.
- Lavoie HA et al. (2004) Mol Cell Endocrinol. 227 : 31-40.
- Lawson KA et al. (1999) Genes Dev. 13 :424-36.
- Lee KH et al. (2004) Development 131 : 4709-23.
- Lei N, Heckert LL. (2004) Mol Cell Biol. 24 : 377-88.
- Levavasseur F et al. (1998) Mech Dev. 74 : 89-98.
- Lickert H et al. (2005) Development. 2005 Jun; 132(11):2599-609. Epub 2005 Apr 27.
- Lin T et al. (2005) Nat Cell Biol. 7:165-71.
- Loebel DA et al., 2003 Dev Biol. 264 :1-14. Review.
- Martin GR. (1981) Proc Natl Acad Sci USA. 78 : 7634-8.
- Matsuda T et al. (1999) EMBO J. 18 : 4261-9.
- Mayhall EA et al. (2004) Curr Opin Cell Biol. 16 : 713-720.
- Meeks JJ et al. (2003) Nat Genet. 34 : 32-3.
- Metzger D et al. (1995) Proc Natl Acad Sci USA. 92 : 6991-5.
- Minasi MG et al. (2002) Development 129 : 2773-2283.
- Mitsui K et al. (2003) Cell 113 : 631-42.
- Mitsunaga K et al. (2004) Mech Dev. 121 : 237-46.
- Miyagi S et al. (2004) Mol Cell Biol. 24 : 4207-20.
- Mizusaki H et al. (2003) Mol Endocrinol 17 : 507-19.
- Mochizuki K et al. (2001) Dev Genes Evol. 211 : 299-308.
- Morais da Silva S et al. (1996) Nat Genet. 14 : 62-8.
- Mukhopadhyay M et al. (2003) Development 130 : 495-505.
- Muller S et al. (2004) Oncogene 23 : 1998-2008.
- Nakamura H et al. (2004) Mech Dev. 121 : 1137-43.
- Nichols J et al. (1996) Mech Dev. 1996 Jul;57(2):123-31.
- Nichols J et al. (2001) Development 128(12):2333-9.
- Nishimoto M et al. (1999) Mol Cell Biol. 19 : 5453-65.
- Nishimoto M et al. (1999) Mol Cell Biol. 19: 5453-65.
- Niwa H et al. (1998) Genes Dev. 12 : 2048-60.
- Niwa H et al. (2000) Nat Genet. 24 : 372-6.
- Niwa H et al. (2002) Mol Cell Biol. 22 : 1526-36.
- Ohinata Y, (2005). Nature 436 : 207-13.
- Okumura-Nakanishi S et al. (2005) J Biol Chem. 280 : 5307-5317.
- Pain B et al. (1996) Development 122 : 2339-48.
- Pain Bet al., (1999) Cells Tissues Organs. 165 : 212-9. Review.
- Palacios IM et al. (2004) Nature 427 : 753-7.
- Park TS et al., (2003) Mol Reprod Dev. 65 : 389-95.
- Pekarik V et al. (2003) Nat Biotechnol. 21 : 93-6. Erratum in: Nat Biotechnol. 21 : 199.
- Petitte JN et al. (1990) Development 108 : 185-9.
- Ramalho-Santos M et al. (2002) Science 298 : 597-600.
- Raz R et al. (1999) Proc Natl Acad Sci USA. 96 : 2846-551.
- Reik W et al. (2001) Science 293 : 1089-1093.
- Robertson EJ. (1987). Embryo derived stem cells. In teratocarcinomas and Embryonic Stem Cells : a Practical Approach (Ed E.J. Robertson), pp71-112. Oxford IRL Press.
- Rocak S, Linder P. (2004) Nat Rev Mol Cell Biol. 5 : 232-41.
- Saitou M et al. (2002) Nature 418 : 293-300.
- Schisa JA et al. (2001) Development 128 : 1287-98.
- Seale P et al. (2004) Dev Biol. 275 : 287-300.
- Seeler JS, Dejean A.(2003) Nat Rev Mol Cell Biol. 4 : 690-9.
- Sekido R et al. (2004) Dev Biol. 274 : 271-9.
- Seydoux G, Dunn MA. (1997) Development 124 : 2191-201.
- Shamblott MJ et al. (1998). Proc Natl Acad Sci USA. 95 : 13726-31. Erratum in: Proc Natl Acad Sci U S A 96: 1162.
- Sheng G, Stem CD. (1999) Mech Dev. 87 : 213-6.
- Shibata N et al. (1999) Dev Biol. 206 : 73-87.
- Shiota K et al. (2002) Genes Cells 7 : 961-969.
- Shizuru JA et al. (2005) Annu Rev Med.;56:509-38.
- Siggers P et al. (2002) Mech Dev. 111: 159-62.
- Song JJ et al. (2004) Science 305 : 1434-7.
- Song X et al. (2002) Science 296 : 1855-7.
- Song X et al. (2004) Development 131: 1353-64.
- Soodeen-Karamath S, Gibbins AM. (2001) Mol Reprod Dev. 58 : 137-48.
- Stebler J et al. (2004) Dev Biol. 272 : 351-61.
- Sumi T et al. (2004) Stem Cells 22 : 861-72.
- Swain A et al. (1998) Nature 391 : 761-7.
- Swanberg SE et al. (2004) Dev Dyn. 231: 14-21.
- Tada Tet al., (1998) Dev Genes Evol.207 : 551-61.
- Tahbaz N et al. (2004) EMBO Rep.5 : 189-94.
- Takahashi K et al. (2003) Nature 423 : 541-5.
- Tanaka SS et al. (2000) Genes Dev. 14 : 841-53.
- Thomson JA et al. (1995) Proc Natl Acad Sci USA. 92 : 7844-8.
- Thomson JA et al. (1998) Science 282 : 1145-7. Erratum in: Science 282 : 1827.
- Thoraval P et al. (1994) Poult Sci.73 : 1897-905.
- Thoraval P et al. (1994) Poult Sci.73 :1897-905.
- Tokuzawa Y et al. (2003) Mol Cell Biol. 23 : 2699-2708.
- Tomari Y et al. (2004) Cell 116 : 831-41.
- Toyooka Y et al. (2003) Proc Natl Acad Sci USA. 100 : 11457-62.
- Tremblay KD et al. (2001) Development 128 : 3609-21.
- Tsuda M et al. (2003) Science 301 : 1239-41.
- Tsunekawa N et al. (2000) Development 127 : 2741-S0.
- Vallier L et al. (2004) et al. Dev Biol. 275 : 403-21.
- Velculescu VE et al. (1995) Science 270 : 484-7.
- Vidal VP et al. (2001) Nat Genet. 28 : 216-7.
- Wagers AJ, Weissman IL. (2004) Cell 116 : 639-648.
- Wang Z, Lin H. (2004) Science 303 : 2016-9.
- Weidinger G et al. (2003) Curr Biol. 13 : 1429-34.
- Wilcox A et al. (2004) J Biol Chem. 279 : 38881-8.
- Wilder PJ et al. (1997) Dev Biol. 192 : 614-29.
- Winnier G et al. (1995) Genes Dev. 9: 2105-16.
- Wurmser AE et al. (2004) Science 304 : 1253-5.
- Xie T, Spradling AC. (2000) Science 290 : 328-30.
- Yamashita YM et al. (2003) Science 301 : 1547-50.
- Yano T et al. (2004) Dev Cell. 6 : 637-48.
- Yeom YI et al. (1996) Development 122 : 881-94.
- Ying QL et al. (2003) Cell 115 : 281-92.
- Ying Y et al. (2000) Mol Endocrinol. 14 : 1053-63.
- Ying Yet al., (20041) Proc Natl Acad Sci U S A.98 :7858-62.
- Yoon C et al. (1997) Development 124 : 3157-65.
- Yoshida K et al. (1994) Mech Dev. 1994 Feb;45(2):163-71.
- Yoshimizu T et al. (1999) Dev Growth Differ. 41 : 675-84.
- Youngren KKet al., (2005) Nature 435 : 360-4.
- Yu RN et al. (1998) Mol Endocrinol. 12 : 1010-22.
- Yuan H et al. (1995) Genes Dev. 9 : 2635-45.
- Zhang et al. (2003) Nature 425 : 836-41.
- Zhao GQ et al. (1996) Genes Dev. 10 : 1657-69.
- Zhao GQ et al. (1998) Development 125; 1103-12
- Zhou S et al. (2001) Nat Med. 7 : 1028-34.

## Revendications

1. Combinaison de marqueurs permettant de caractériser des cellules aviaires de type StX (blastoderme de stade X) ou des cellules souches aviaires, comprenant au moins deux marqueurs **caractérisée en ce que** l'un des marqueurs est spécifique du gène *Ip06,* et l'autre marqueur est choisi parmi :
a. un marqueur d'un gène cible préférentiellement exprimé dans les cellules StX choisi parmi les gènes suivants : *2contig58, 60S-L14, ATM, Bloom syndrome, Bteb4, CD9, CHD Helicase, Clock, Cwf16 (FLJ10374), CXCR4, Dnmt2, Enx1 (Ho-zeste 2), Eomes, EWS, Fgf4, Gata55, Hojl, N-AGN6P deacetylase, N-Cor1, NF2, p53, Pml,Rbm6, Sa2, Sa3, Sara. Scye1, Sef, S1, SnoN, Socs13, Ssb1, TC87479, T-cell APP 2C, TGF-beta2, WD40*/*FYVE-D protein 2, WD-RP3. Zan75. Zpc,* ou
b. un marqueur d'un gène cible préférentiellement exprimé dans les cellules souches choisi parmi les gènes suivants : *1P08-A09, Activin RIIB. Astacin, Claudin3,D apper1, Dorfin, FPP synthase (Fps), GalNAc-T3,Gcnf, Hspb7, Irx4, Lmx,Pax6, Slc38a2, Sox3,Ttral gp96, Wnt10a,Wwnt11.*

2. Combinaison selon la revendication 1, **caractérisée en ce que** l'autre marqueur d'un gène cible préférentiellement exprimé dans les cellules StX (de blastoderme de stade X) est choisi parmi les marqueurs des gènes suivants: *Atm, CXCR4, Eomes, Fgf4, Gata5, N-AGN6P deacetylase. NF2, Socs13, Sssbl, TC87479, T-cell APP 2C, TGF-beta2, WD-RP3. ZPC* et leurs combinaisons.

3. Combinaison selon l'une des revendications 1 ou 2, **caractérisé en ce qu'**elle comprend au moins un marqueur du gène *1p06* et un marqueur du gène *ZPC.*

4. Combinaison selon la revendication 1, **caractérisée en ce que** l'autre marqueur d'un gène cible préférentiellement exprimé dans les cellules souches est choisi parmi les marqueurs des gènes suivants : *Activin RIIB, Aastacin, Claudin3,Dapperl, FPP synthase (Fps), GalNAc-T3, Gcnf, Lmx, Pax,Ttral gp96, Wnt10a* et leurs combinaisons.

5. Combinaison selon la revendication 4, **caractérisé en ce qu'**elle comprend au moins un marqueur du gène *1P06* et un marqueur du gène *Tra1.*

6. Combinaison selon l'une des revendications 1 à 5 **caractérisé en ce que** les marqueurs sont choisis parmi les anticorps venant se lier de manière spécifique aux produits d'expression d'un gène cible, ARNm, ADNc ou polypeptide, ou un fragment de ces derniers, et les fragments d'acide nucléique capables de s'hybrider de manière spécifique aux ARNm exprimés par lesdits gènes cibles ou aux ADNc correspondants, ou des fragments de ces derniers.

7. Combinaison selon l'une des revendications 1 à 6, **caractérisée en ce qu'**elle est assemblée sur un même support, de préférence un support normé.

8. Combinaison selon la revendication 7, **caractérisée en ce qu'**elle se présente sous la forme d'une matrice à ADN, comprenant un support sur lequel sont disposés, préférentiellement de manière normée, les fragments d'acide nucléique susceptibles de s'hybrider avec les gènes cibles.

9. Combinaison selon la revendication 8, **caractérisée en ce qu'**elle est disposée sur une puce à ADN.

10. Puce à ADN, **caractérisée en ce qu'**elle comprend une combinaison de marqueurs selon l'une des revendications 1 à 9.

11. Procédé de caractérisation d'une cellule aviaire comprenant les étapes suivantes :
- l'analyse de l'expression des gènes exprimés dans ladite cellule, en comparant les niveaux relatifs d'expression d'au moins deux marqueurs, l'un étant spécifique du gène 1p06 et l'autre étant choisi parmi :
a. un marqueur d'un gène cible préférentiellement exprimé dans les cellules StX choisi parmi les gènes suivants : *2contig58. 60S-L14, ATM, Bloom syndrome, Bteb4, CD9, CHD Helicase, Clock, Cwf16 (FLJ10374), CXCR4, Dnmt2, Enx1 (Ho-zeste* 2), *Eomes, EWS, Fgf4, Gata55. Hoj1, N-AGN6P deacetylase, N-Cor1, NF2, p53, Pml,Rbm6, Sa2, Sa3, Sara, Scye1, Sef, S1, SnoN, Socs13, Ssb1, TC87479, T-cell APP 2C, TGF-beta2, WD40*/*FYVE-D protein 2, WD-RP3. Zan75, Zpc,* ou
b. un marqueur d'un gène cible préférentiellement exprimé dans les cellules souches choisi parmi les gènes suivants : *IP08-A09, Activin RIIB, Astacin, Claudin3,D apper1, Dorfin, FPP synthase (Fps), GalNAc-T3,Gcnf, Hspb7, Irx4, Lmx,Pax6, Slc38a2, Sox3,Ttra1 gp96, Wnt10a, Wnt11,*
- la caractérisation du phénotype des cellules analysées entre les deux types suivants: cellules aviaires de type StX ou cellules souches aviaires.

12. Procédé de culture de cellules aviaires comprenant la culture de cellules StX ou de cellules souches aviaires préalablement caractérisées au moyen d'un procédé selon la revendication 11 et isolées, dans un milieu de culture approprié.

13. Procédé d'obtention de cellules germinales aviaires, **caractérisé en ce qu'**il comprend une étape de culture de cellules StX obtenues selon la revendication 12, dans un milieu de culture approprié sans adjonction de tapis nourricier « feeder » inactivé.

## Claims

1. Combination of markers allowing StX-type avian cells (X-stage blastoderm) or avian stem cells to be characterized, including at least two markers **characterized in that** one of the markers is specific to the 1p06 gene, and the other marker is chosen from:
a. a marker of a target gene preferentially expressed in StX cells chosen from the. following genes: 2contig58, 60S-Ll4, ATM, Bloom syndrome, Bteb4, CD9, CHD Helicase, Clock, Cwf16 (FLJ10374), CXCR4, Dnmt2, Enx1 (Ho-zeste 2), Eomes, EWS, Fgf4, Gata55, Hoj1, N-AGN6P deacetylase, N-Cor1, NF2, p53, Pml, Rbm6, Sa2, Sa3, Sara, Scye1, Sef, S1, SnoN, Socs13, Ssb1, TC87479, T-cell APP 2C, TGF-beta2, WD40/FYVE-D protein 2, WD-RP3, Zan75, and Zpc, or
b. a marker of a target gene preferentially expressed in stem cells chosen from the following genes: 1P08-A09, Activin RIIB, Astacin, Claudin3, D apper1, Dorfin, FPP synthase (Fps), GalNAc-T3, Gcnf, Hspb7, Irx4, Lmx, Pax6, Slc38a2, Sox3, Ttra1 gp96, Wnt10a, and Wwnt11.

2. Combination according to claim 1, **characterized in that** the marker of a target gene preferentially expressed in StX cells (X-stage blastoderm) is chosen from the markers of the following genes: Atm, CXCR4, Eomes, Fgf4, Gata5, N-AGN6P deacetylase, NF2, Socs13, Sssbl, TC87479, T-cell APP 2C, TGF-beta2, WD-RP3, ZPC and combinations thereof.

3. Combination according to one of claims 1 or 2, **characterized in that** it includes at least one marker of the 1p06 gene and one marker of the ZPC gene.

4. Combination according to claim 1, **characterized in that** the other marker of a target gene preferentially expressed in the stem cells is chosen from the markers of the following genes: Activin RIIB, Aastacin, Claudin3, Dapper1, FPP synthase (Fps), GalNAc-T3, Gcnf, Lmx, Pax, Ttra1 gp96, Wnt10a and combinations thereof.

5. Combination according to claim 4, **characterized in that** it includes at least one marker of the 1P06 gene and one marker of the Tra1 gene.

6. Combination according to one of claims 1 to 5, **characterized in that** the markers are chosen from the antibodies binding specifically to the expression products of a target gene, mRNA, cDNA or polypeptide, or a fragment thereof, and nucleic acid fragments capable of hybridizing specifically with the mRNA expressed by said target genes or the corresponding cDNA, or fragments thereof.

7. Combination according to one of claims 1 to 6, **characterized in that** it is assembled on a same support, preferably on a standard support.

8. Combination according to claim 7, **characterized in that** it is in the form of a DNA matrix, including a support on which the nucleic acid fragments capable of hybridizing with the target genes are arranged, preferably in a standard manner.

9. Combination according to claim 8, **characterized in that** it is arranged on a DNA chip.

10. DNA chip, **characterized in that** it includes a combination of markers according to one of claims 1 to 9.

11. Process for characterizing an avian cell including the following steps:
- the analysis of the expression of genes expressed in said cell, by comparing the relative expression levels of at least two markers, one being specific to the 1P06 gene and the other being chosen from:
a. a marker of a target gene preferentially expressed in StX cells chosen from the following genes: 2contig58, 60S-L14, ATM, Bloom syndrome, Bteb4, CD9, CHD Helicase, Clock, Cwf16 (FLJ10374), CXCR4, Dnmt2, Enx1 (Ho-zeste 2), Eomes, EWS, Fgf4, Gata55, Hoj1, N-AGN6P deacetylase, N-Cor1, NF2, p53, Pml, Rbm6, Sa2, Sa3, Sara, Scye1, Sef, S1, SnoN, Socs13, Ssb1, TC87479, T-cell APP 2C, TGF-beta2, WD40/FYVE-D protein 2, WD-RP3, Zan75, and Zpc, or
b. a marker of a target gene preferentially expressed in stem cells chosen from the following genes: 1P08-A09, Activin RIIB, Astacin, Claudin3, D apper1, Dorfin, FPP synthase (Fps), GalNAc-T3, Gcnf, HSpb7, Irx4, Lmx, Pax6, Slc38a2, Sox3, Ttra1 gp96, Wnt10a, and Wnt11,
- the characterization of the phenotype of the cells analyzed between the following two types: StX-type avian cells or avian stem cells.

12. Process for culturing avian cells including the culture of StX cells or avian stem cells previously characterized using a process according to claim 11 and isolated, in a suitable culture.

13. Process for obtaining avian germ cells, **characterized in that** it includes a step of culturing StX cells obtained according to claim 12, in a suitable culture medium without the addition of an inactivated "feeder" layer.

## Patentansprüche

1. Kombination von Markern, welche erlaubt, Vogelzellen vom Typ StX (Blastoderm des Stadiums X) oder Vogelstammzellen zu charakterisieren, welche mindestens zwei Marker umfasst, **dadurch gekennzeichnet, dass** einer der Marker für das Gen *1p06* spezifisch ist und der andere Marker ausgewählt ist aus:
a. einem Marker eines Zielgens, das bevorzugt in den StX-Zellen exprimiert wird, ausgewählt unter den folgenden Genen: *2contig58, 60S-L14, ATM, Bloom syndrome, Bteb4, CD9, CHD Helicase, Clock, Cwf16 (FLJ10374), CXCR4, Dnmt2, Enx1 (Ho-zeste* 2), *Eomes, EWS, Fgf4, Gata55, Hoj1, N-AGN6P deacetylase, N-Cor1, NF2, p53, Pml, Rbm6, Sa2, Sa3, Sara, Scye1, Sef, S1, SnoN, Socs13, Ssb1, TC87479, T-cell APP 2C, TGF-beta2, WD40*/*FYVE-D protein 2, IND-RP3, Zan75, Zpc,* oder
b. einem Marker eines Zielgens, das bevorzugt in den Stammzellen exprimiert wird, ausgewählt unter den folgenden Genen: *1P08-A09, Activin RIIB, Astacin, Claudin3, D apper1, Dorfin, FPP synthase (Fps), GalNAc-T3, Gcnf, Hspb7, Irx4, Lmx, Pax6, Slc38a2, Sox3, Ttra1 gp96, Wnt10a, Wwnt11.*

2. Kombination nach Anspruch 1, **dadurch gekennzeichnet, dass** der andere Marker eines Zielgens, das bevorzugt in den StX-Zellen (Blastoderm-Stadium X-Zellen) exprimiert wird, ausgewählt ist unter den Markern der folgenden Gene: *Atm, CXCR4, Eomes, Fgf4, Gata5, N-AGN6P deacetylase, NF2, Socs13, Sssb1, TC87479, T-cell APP 2C, TGF-beta2, WD-RP3, ZPC* und deren Kombinationen.

3. Kombination nach einem der Ansprüche 1 oder 2, **dadurch gekennzeichnet, dass** sie mindestens einen Marker des Gens *1p06* und einen Marker des Gens ZPC umfasst.

4. Kombination nach Anspruch 1, **dadurch gekennzeichnet, dass** der andere Marker eines Zielgens, das bevorzugt in den Stammzellen exprimiert wird, ausgewählt ist unter den Markern der folgenden Gene: *Activin RIIB, Aastacin, Claudin3, Dapper1, FPP synthase (Fps), GalNAc-T3, Gcnf Lmx, Pax, Ttra1 gp96, Wnt10a* und deren Kombinationen.

5. Kombination nach Anspruch 4, **dadurch gekennzeichnet, dass** sie mindestens einen Marker des Gens *1P06* und einen Marker des Gens *Tra1* umfasst.

6. Kombination nach einem der Ansprüche 1 bis 5, **dadurch gekennzeichnet, dass** die Marker unter den Antikörpern, die sich auf spezifische Weise an die Expressionsprodukte eines Zielgens, einer mRNA, einer cDNA oder ein Polypeptid binden werden, oder einem Fragment von diesen Letzteren und den Nukleinsäurefragmenten, die in der Lage sind, auf spezifische Weise mit den durch die Zielgene exprimierten mRNAs oder mit den entsprechenden cDNAs zu hybridisieren, oder Fragmenten von diesen Letzteren ausgewählt sind.

7. Kombination nach einem der Ansprüche 1 bis 6, **dadurch gekennzeichnet, dass** sie auf ein und demselben Träger, vorzugsweise einem normierten Träger zusammengestellt ist.

8. Kombination nach Anspruch 7, **dadurch gekennzeichnet, dass** sie in Form einer DNA-Matrix vorliegt, welche einen Träger, auf welchem vorzugsweise auf normierte Weise die Nukleinsäurefragmente, die in der Lage sind, mit den Zielgenen zu hybridisieren, angeordnet sind, umfasst.

9. Kombination nach Anspruch 8, **dadurch gekennzeichnet, dass** sie auf einem DNA-Chip angeordnet ist.

10. DNA-Chip, **dadurch gekennzeichnet, dass** er eine Kombination von Markern nach einem der Ansprüche 1 bis 9 umfasst.

11. Verfahren zur Charakterisierung einer Vogelzelle, welches die folgenden Schritte umfasst:
- die Analyse der Expression der Gene, die in der Zelle exprimiert werden, indem man die relativen Expressionsniveaus von mindestens zwei Markern vergleicht, wobei der eine für das Gen 1 p06 spezifisch ist und der andere ausgewählt ist unter:
a. einem Marker eines Zielgens, das bevorzugt in den StX-Zellen exprimiert wird, ausgewählt unter den folgenden Genen: *2contig58, 60S-L14, ATM, Bloom syndrome, Bteb4, CD9, CHD Helicase, Clock, Cwf16 (FLJ10374), CXCR4, Dnmt2, Enx1 (Ho-zeste* 2), *Eomes, EWS, Fgf4, Gata55, Hoj1, N-AGN6P deacetylase, N-Cor1, NF2, p53, Pml, Rbm6, Sa2, Sa3, Sara, Scye1, Sef, S1, SnoN, Socs13, Ssb1, TC87479, T-cell APP 2C, TGF-beta2, WD40*/*FYVE-D protein 2, WD-RP3, Zan75, Zpc,* oder
b. einem Marker eines Zielgens, das bevorzugt in den Stammzellen exprimiert wird, ausgewählt unter den folgenden Genen: *1P08-A09, Activin RIIB, Astacin, Claudin3, D apper1, Dorfin, FPP synthase (Fps), GalNAc-T3, Gcnf Hspb7, Irx4, Lmx, Pax6, Slc38a2, Sox3, Ttra1 gp96, Wnt10a, Wnt11,*
- die Charakterisierung des Phänotyps der analysierten Zellen zwischen den zwei folgenden Typen: Vogelzellen vom Typ StX oder Vogelstammzellen.

12. Verfahren zum Kultivieren von Vogelzellen, welches die Kultivierung von StX-Zellen oder von Vogelstammzellen, die vorab mittels eines Verfahrens nach Anspruch 11 charakterisiert und isoliert worden sind, in einem geeigneten Kulturmedium umfasst.

13. Verfahren zum Gewinnen von Vogelkeimzellen, **dadurch gekennzeichnet, dass** es einen Schritt einer Kultivierung von StX-Zellen, die gemäß Anspruch 12 erhalten worden sind, in einem geeigneten Kulturmedium ohne Hinzufügung einer inaktivierten "Feeder"-Nährschicht ("Feeder-Layer") umfasst.


### SEGUENCE LlSTING

<110> INRA, CNRS, ENS Lyon, Vivalis
<120> Combinai son de marqueurs de cellules aviaires
<130> 242486 D23927
<150> FR 06/02592 2006-03-24
   <151>
<160> 376
<170> Patent Inversion 3. 3
<210> 1
   <211> 1263
   <212> DNA
   <213> Gallus gallus
<400> 1
   atgagcgctc acctggccat gccgtcctac ggctctgtta ggtgcggaca ctactactgg 60
   ccctctccgg gcagcatgga tagcgcgtct gccgcggaag ctccagcagc agacctctcc 120
   ttgaccacag agcagaaaac gccctgccac ccagcgcgac acccccagtg ctccctcagt 180
   gcccgtcggc ttttactgaa agctgcccgc agccagggag caatggccgg gccgtgtcgg 240
   ggagtggtag gcagtacttc tcacgtgtcc cattgggaac tgagcggtgg gcaggggctg 300
   ggagctgctg ccggacacgc cgctaaagtc ctagcgcggc cttcatgtac tactggcaac 360
   tctgcggggc tgtcttgccg gcggtcactg cttcccgtgc ttaccctggc tgcggagtct 420
   gcagcagtct ccagtgctcc agaatctgaa attatttcca gaagctgcac gtcgaagttt 480
   ttatctgatg cctctccagc ttcttccagc tctgggacac tcattcagta taccccagac 540
   tctgccacta gccctactgc agaccaccca tctcaccgcc ccacttttca gaaggttaag 600
   gataaaggtg agagtgggac aaggaaggcc aagagccgca cagctttctc ccaggagcag 660
   ctgcagaccc tgcaccagcg gtttcagagc cagaagtacc tcagccccca tcagatccgg 720
   gagctggctg ctgctctggg gctcacctac aagcaggtga agacgtggtt tcagaaccaa 780
   cgaatgaagt ttaaacgttg ccagaaagag agtcagtggg tggataaagg gatttatcta 840
   ccacagaatg ggtttcatca agctgcgtat ctggatatga cccctacatt tcaccagggc 900
   ttccctgttg ttgccaacag aaaccttcag gctgtgacca gtgcacacca ggcttacagc 960
   agtggccaga cttatgggaa tgggcagggc ctgtatccgt tcatggctgt ggaggatgag 1020
   ggcttctttg gaaaaggtgg aacaagctgc aacacccagc aggccatggg tttattaagt 1080
   caacagatga acttctatca tggctactct accaatgtgg attatgacag cttgcaggca 1140
   gaagatacct acagcttcca gagcacctct gatagtatca cacagttctc gagctctcct 1200
   gtacggcatc agtaccaggc cccttggcat accctgggga cccagaatgg ttatgagact 1260
   tag 1263
<210> 2
   <211> 420
   <212> PRT
   <213> Gallus gallus
<400> 2
   Met Ser Ala His Leu Ala Met Pro Ser Tyr Gly Ser Val Arg Cys Gly
   1 5 10 15
   His Tyr Tyr Trp Pro Ser Pro Gly Ser Met Asp Ser Ala Ser Ala Ala
   20 25 30
   Glu Ala Pro Ala Ala Asp Leu Ser Leu Thr Thr Glu Gln Lys Thr Pro
   35 40 45
   Cys His Pro Ala Arg His Pro Gln Cys Ser Leu Ser Ala Arg Arg Leu
   50 55 60
   Leu Leu Lys Ala Ala Arg Ser Gln Gly Ala Met Ala Gly Pro Cys Arg
   65 70 75 80
   Gly Val Val Gly Ser Thr Ser His Val Ser His Trp Glu Leu Ser Gly
   85 90 95
   Gly Gln Gly Leu Gly Ala Ala Ala Gly His Ala Ala Lys Val Leu Ala
   100 105 110
   Arg Pro Ser Cys Thr Thr Gly Asn Ser Ala Gly Leu Ser Cys Arg Arg
   115 120 125
   Ser Leu Leu Pro Val Leu Thr Leu Ala Ala Glu Ser Ala Ala Val Ser
   130 135 140
   Ser Ala Pro Glu Ser Glu Ile Ile Ser Arg Ser Cys Thr Ser Lys Phe
   145 150 155 160
   Leu Ser Asp Ala Ser Pro Ala Ser Ser Ser Ser Gly Thr Leu Ile Gln
   165 170 175
   Tyr Thr Pro Asp Ser Ala Thr Ser Pro Thr Ala Asp His Pro Ser His
   180 185 190
   Arg Pro Thr Phe Gln Lys Val Lys Asp Lys Gly Glu Ser Gly Thr Arg
   195 200 205
   Lys Ala Lys Ser Arg Thr Ala Phe Ser Gln Glu Gln Leu Gln Thr Leu
   210 215 220
   His Gln Arg Phe Gln Ser Gln Lys Tyr Leu Ser Pro His Gln Ile Arg
   225 230 235 240
   Glu Leu Ala Ala Ala Leu Gly Leu Thr Tyr Lys Gln Val Lys Thr Trp
   245 250 255
   Phe Gln Asn Gln Arg Met Lys Phe Lys Arg Cys Gln Lys Glu Ser Gln
   260 265 270
   Trp Val Asp Lys Gly Ile Tyr Leu Pro Gln Asn Gly Phe His Gln Ala
   275 280 285
   Ala Tyr Leu Asp Met Thr Pro Thr Phe His Gln Gly Phe Pro Val Val
   290 295 300
   Ala Asn Arg Asn Leu Gln Ala Val Thr Ser Ala His Gln Ala Tyr Ser
   305 310 315 320
   Ser Gly Gln Thr Tyr Gly Asn Gly Gln Gly Leu Tyr Pro Phe Met Ala
   325 330 335
   Val Glu Asp Glu Gly Phe Phe Gly Lys Gly Gly Thr Ser Cys Asn Thr
   340 345 350
   Gln Gln Ala Met Gly Leu Leu Ser Gln Gln Met Asn Phe Tyr His Gly
   355 360 365
   Tyr Ser Thr Asn Val Asp Tyr Asp Ser Leu Gln Ala Glu Asp Thr Tyr
   370 375 380
   Ser Phe Gln Ser Thr Ser Asp Ser Ile Thr Gln Phe Ser Ser Ser Pro
   385 390 395 400
   Val Arg His Gln Tyr Gln Ala Pro Trp His Thr Leu Gly Thr Gln Asn
   405 410 415
   Gly Tyr Glu Thr
   420
<210> 3
   <211> > 1008
   <212> DNA
   <213> Gallus gallus
<400> 3 <210> 4

   <211> 336
   <212> PRT
   <213> Gallus gallus
<400> 4
<210> 5
   <211> 21
   <212> DNA
   <213> Artificial
<220>
   <223> PCR primer
<400> 5
   acat gt gccc tcttattgca c 21
<210> 6
   <211> 21
   <212> DNA
   <213> Artificial
<220>
   <223> PCR primer
<400> 6
   aaact ccaca ccatcatcag c 21
<210> 7
   <211> 21
   <212> DNA
   <213> Artificial
<220>
   <223> PCR primer
<400> 7
   atctttctgc ccgt aggt gt t 21
<210> 8
   <211> 21
   <212> DNA
   <213> Artificial
<220>
   <223> PCR pr i rrer
<400> 8
   tgctgcccac ttctcatttat 21
<210> 9
   <211> 21
   <212> DNA
   <213> Artificial
<220>
   <223> PCR primer
<400> 9
   at cct t ccca caccagagac t 21
<210> 10
   <211> 21
   <212> DNA
   <213> Artificial
<220>
   <223> PCR primer
<400> 10
   gagct cat gt cagct t cat c c 21
<210> 11
   <211> 21
   <212> DNA
   <213> Artificial
<220>
   <223> PCR pr i rrer
<400> 11
   gcaaaactct cgggaaaatc t 21
<210> 12
   <211> 21
   <212> DNA
   <213> Artificial
<220>
   <223> PCR primer
<400> 12
   gcatcttctggtgttgtccat 21
<210> 13
   <211> 21
   <212> DNA
   <213> Artificial
<220>
   <223> PCR primer
<400> 13
   ctcgacttcg acaaccagaa g 21
<210> 14
   <211> 21
   <212> DNA
   <213> Artificial <220>
   <223> PCR primer
<400> 14
   tgcatcacactgttctcaagg 21
<210> 15
   <211> 21
   <212> DNA
   <213> Artificial
<220>
   <223> PCR primer
<400> 15
   tccattttgc tctgaactgc t 21
<210> 16
   <211> > 21
   <212> DNA
   <213> Artificial
<220>
   <223> PCR primer
<400> 16
   ctctgtttct gcggatagcacc 21
<210> 17
   <211> 21
   <212> DNA
   <213> Artificial
<220>
   <223> PCR primer
<400> 17
   taaagaacct gaaggggagg a 21
<210> 18
   <211> 21
   <212> DNA
   <213> Artificial
<220>
   <223> PCR primer
<400> 18
   ctcttctattcgtcgccactg 21
<210> 19
   <211> 20
<212> DNA
   <213> Artifcial
<220>
   <223> PCR primer
<400> 19
   tgcagttcaa caaccagctc 20
<210> 20
   <211> 20
   <212> DNA
   <213> Artificial
<220>
   <223> PCR primer
<400> 20
   gtcagttctgtgccgtgaga 20
<210> 21
   <211> 21
   <212> DNA
   <213> Artificial
<220>
   <223> PCR pr i rrer
<400> 21
   ctccccatat tccgcttcta c 21
<210> 22
   <211> 21
   <212> DNA
   <213> Artificial
<220>
   <223> PCR primer
<400> 22
   tcagctcctgtaggttctcc a 21
<210> 23
   <211> 21
   <212> DNA
   <213> Artificial
<220>
   <223> PCR primer
<400> 23
   caggaataat cgggaatggat 21
<210> 24
   <211> 21
   <212> DNA
   <213> Artificial
<220>
   <223> PCR primer
<400> 24
   caggacattt ccgaagtacc a 21
<210> 25
   <211> 20
   <212> DNA
   <213> Artificial
<220>
   <223> PCR primer
<400> 25
   t t ggcat t cc caat t ct agg 20
<210> 26
   <211> 20
   <212> DNA
   <213> Artificial
<220>
   <223> PCR primer
<400> 26
   gaaaggcccc ttttggtaag 20
<210> 27
   <211> 20
   <212> DNA
   <213> Artificial
<220>
   <223> PCR primer
<400> 27
   aacaggtttc ctggatgtcg 20
<210> 28
   <211> 20
   <212> DNA
   <213> Artificial
<220>
   <223> PCR primer
<400> 28 20
   tttggatccc tctctggatg 20
<210> 29
   <211> 21
   <212> DNA
   <213> Artificial
<220>
   <223> PCR primer
<400> 29
   acacttgttc gcagagcagt t 21
<210> 30
   <211> 21
   <212> DNA
   <213> Artificial
<220>
   <223> PCR primer
<400> 30
   gccacctcaa t aagggt t ag g 21
<210> 31
   <211> 21
   <212> DNA
   <213> Artificial
<220>
   <223> PCR primer
<400> 31
   cagccaccat caagt acaac a 21
<210> 32
   <211> 21
   <212> DNA
   <213> Artificial
<220>
   <223> PCR primer
<400> 32
   ctgctgggaa t aagt gct ct g 21
<210> 33
   <211> 21
   <212> DNA
   <213> Artificial
<220>
   <223> PCR primer
<400> 33
   aaccgataca gtctgctgga a 21
<210> 34
   <211> 21
   <212> DNA
   <213> Artificial
<220>
   <223> PCR primer
<400> 34
   agttgtttgg caggaggatct 21
<210> 35
   <211> 21
   <212> DNA
   <213> Artificial
<220>
   <223> PCR primer
<400> 35
   gtgggctcta ccacaaaat g a 21
<210> 36
   <211> 21
   <212> DNA
   <213> Artificial
<220>
   <223> PCR primer
<400> 36 21
   tgcctttggt aatgtttttc g 21
<210> 37
   <211> 21
   <212> DNA
   <213> Artificial
<220>
   <223> PCR primer
<400> 37 21
   atggccagta gtccctctgt t 21
<210> 38
   <211> 21
   <212> DNA
   <213> Artificial
<220>
   <223> PCR primer
<400> 38 21
   caatgccgta gtatgcagga t 21
<210> 39
   <211> 21
   <212> DNA
   <213> Artificial
<220>
   <223> PCR primer
<400> 39 21
   cactatgacc tttggcagag c 21
<210> 40
   <211> 21
   <212> DNA
   <213> Artificial
<220>
   <223> PCR primer
<400> 40 21
   aatgcaccat tcaaaacaag c 21
<210> 41
   <211> 21
   <212> DNA
   <213> Artificial
<220>
   <223> PCR primer
<400> 41 21
   cct cct aacc aaggagcatt c 21
<210> 42
   <211> 21
   <212> DNA
   <213> Artificial
<220>
   <223> PCR primer
<400> 42
   ct gagat gga cct ggat gaa a 21
<210> 43
   <211> 21
   <212> DNA
   <213> Artificial
<220>
   <223> PCR primer
<400> 43
   actaccctca ggggttcttc a 21
<210> 44
   <211> 21
   <212> DNA
   <213> Artificial
<220>
   <223> PCR primer
<400> 44
   cctcaaacac aagacccaaa a 21
<210> 45
   <211> 21
   <212> DNA
   <213> Artificial
<220>
   <223> PCR primer
<400> 45
   ccgtggccgt ctataagaaat 21
<210> 46
   <211> 21
   <212> DNA
   <213> Artificial
<220>
   <223> PCR pr i rrer
<400> 46
   ccagtgtaag gatggtgagg a 21
<210> 47
   <211> 21
   <212> DNA
   <213> Artificial
<220>
   <223> PCR primer
<400> 47
   acctcgccag ccataactaa t 21
<210> 48
   <211> 21
   <212> DNA
   <213> Artificial
<220>
   <223> PCR primer
<400> 48
   tctccttcat ggggatcttc t 21
<210> 49
   <211> 21
   <212> DNA
   <213> Artificial
<220>
   <223> PCR primer
<400> 49
   tggaacaggg actatcctcc t 21
<210> 50
   <211> 21
   <212> DNA
   <213> Artificial
<220>
   <223> PCR primer
<400> 50
   gctccaaagc tgaaagaagg t 21
<210> 51
   <211> 21
   <212> DNA
   <213> Artificial
<220>
   <223> PCR primer
<400> 51
   aagaaaacaa agccagcaac a 21
<210> 52
   <211> 21
   <212> DNA
   <213> Artificial
<220>
   <223> PCR primer
<400> 52
   atctcttggg ctccctcatt a 21
<210> 53
   <211> 21
   <212> DNA
   <213> Artificial
<220>
   <223> PCR primer
<400> 53
   atgcacaaaa agtggcaaat c 21
<210> 54
   <211> 21
   <212> DNA
   <213> Artificial
<220>
   <223> PCR primer
<400> 54
   agttcgggca caatctacct t 21
<210> 55
   <211> 21
   <212> DNA
   <213> Artificial
<220>
   <223> PCR primer
<400> 55
   gttcccacat gcttgtcact t 21
<210> 56
   <211> 21
   <212> DNA
   <213> Artificial
<220>
   <223> PCR primer
<400> 56
   aaagatttgg atgcgtttgt g 21
<210> 57
   <211> 21
   <212> DNA
   <213> Artificial
<220>
   <223> PCR primer
<400> 57
   ccggatgcag act cat t gt a t 21
<210> 58
   <211> 21
   <212> DNA
   <213> Artificial
<220>
   <223> PCR primer
<400> 58
   tcatctttgc aggcttcaag t 21
<210> 59
   <211> 21
   <212> DNA
   <213> Artificial
<220>
   <223> PCR primer
<400> 59
   aattcagtgggaaagcatgtg 21
<210> 60
   <211> 21
   <212> DNA
   <213> Artificial
<220>
   <223> PCR primer
<400> 60
   ttcagatttgcaaagggattg 21
<210> 61
   <211> 21
   <212> DNA
   <213> Artificial
<220>
   <223> PCR primer
<400> 61
   aacctatgatggacccaggac 21
<210> 62
   <211> 21
   <212> DNA
   <213> Artificial
<220>
   <223> PCR primer
<400> 62
   cagaataagcatcgccagaag 21
<210> 63
   <211> 21
   <212> DNA
   <213> Artificial
<220>
   <223> PCR primer
<400> 63
   taagcgtttagacccccttgt 21
<210> 64
   <211> 21
   <212> DNA
   <213> Artificial
<220>
   <223> PCR primer
<400> 64
   atgatccttcccttgtcgtct 21
<210> 65
   <211> 21
   <212> DNA
   <213> Artificial
<220>
   <223> PCR primer
<400> 65
   cagattgtgtgcaactcctca 21
<210> 66
   <211> 21
   <212> DNA
   <213> Artificial
<220>
   <223> PCR primer
<400> 66
   tcgacgttcttcacttttgct 21
<210> 67
   <211> 21
   <212> DNA
   <213> Artificial
<220>
   <223> PCR primer
<400> 67
   gtggaatagggaggagtctgg 21
<210> 68
   <211> 21
   <212> DNA
   <213> Artificial
<220>
   <223> PCR primer
<400> 68
   ggtgataaacacggaaagcaa 21
<210> 69
   <211> 21
   <212> DNA
   <213> Artificial
<220>
   <223> PCR primer
<400> 69
   tgccagtaccctacaaacctg 21
<210> 70
   <211> 21
   <212> DNA
   <213> Artificial
<220>
   <223> PCR primer
<400> 70
   gctacggctgctaatgcatac 21
<210> 71
   <211> 21
   <212> DNA
   <213> Artificial
<220>
   <223> PCR primer
<400> 71
   tgctgcagtatagggcttgtt 21
<210> 72
   <211> 21
   <212> DNA
   <213> Artificial
<220>
   <223> PCR primer
<400> 72
   gacgaggagtcagagtgcaac 21
<210> 73
   <211> 21
   <212> DNA
   <213> Artificial
<220>
   <223> PCR primer
<400> 73
   tgtatctgtcccgtgccttac 21
<210> 74
   <211> 21
   <212> DNA
   <213> Artificial
<220>
   <223> PCR primer
<400> 74
   ttttgagcttgcgttccttta 21
<210> 75
   <211> 21
   <212> DNA
   <213> Artificial
<220>
   <223> PCR primer
<400> 75
   tgcacgaagactctttccact 21
<210> 76
   <211> 21
   <212> DNA
   <213> Artificial
<220>
   <223> PCR primer
<400> 76
   caaagcagcaagaatcctga c 21
<210> 77
   <211> 21
   <212> DNA
   <213> Artificial
<220>
   <223> PCR primer
<400> 77
   gctgtctgtgctctgctcttt 21
<210> 78
   <211> 21
   <212> DNA
   <213> Artificial
<220>
   <223> PCR primer
<400> 78
   ttgaccccattcagttagcac 21
<210> 79
   <211> 21
   <212> DNA
   <213> Artificial
<220>
   <223> PCR primer
<400> 79
   atacctggttgctccgaaagt 21
<210> 80
   <211> 21
   <212> DNA
   <213> Artificial
<220>
   <223> PCR primer
<400> 80
   ccaccacatttcaagcagttt 21
<210> 81
   <211> 21
   <212> DNA
   <213> Artificial
<220>
   <223> PCR primer
<400> 81
   ccacataccactgaggctgtt 21
<210> 82
   <211> 21
   <212> DNA
   <213> Artificial
<220>
   <223> PCR primer
<400> 82
   accatgaactgcagtgtttcc 21
<210> 83
   <211> 21
   <212> DNA
   <213> Artificial
<220>
   <223> PCR primer
<400> 83
   tccctttgcatccacactat 21
<210> 84
   <211> 21
   <212> DNA
   <213> Artificial
<220>
   <223> PCR primer
<400> 84
   gcttcaccaccatttttgaga 21
<210> 85
   <211> 21
   <212> DNA
   <213> Artificial
<220>
   <223> PCR primer
<400> 85
   gcttctgcaggtcatcatctc 21
<210> 86
   <211> 21
   <212> DNA
   <213> Artificial
<220>
   <223> PCR primer
<400> 86
   gcgttgagctatccctctctt 21
<210> 87
   <211> 21
   <212> DNA
   <213> Artificial
<220>
   <223> PCR primer
<400> 87
   caatcttggcagagtgagagg 21
<210> 88
   <211> 21
   <212> DNA
   <213> Artificial
<220>
   <223> PCR primer
<400> 88
   cgtcctcatggaggtaagaca 21
<210> 89
   <211> 21
   <212> DNA
   <213> Artificial
<220>
   <223> PCR primer
<400> 89
   ctggcacagatcttcaaggag 21
<210> 90
   <211> 21
   <212> DNA
   <213> Artificial <220>

   <223> PCR primer
<400> 90
   acatcacgaccagttggagac 21
<210> 91
   <211> 21
   <212> DNA
   <213> Artificial
<220>
   <223> PCR primer
<400> 91
   caaggtgtacgactccatgct 21
<210> 92
   <211> 21
   <212> DNA
   <213> Artificial
<220>
   <223> PCR primer
<400> 92
   gtagaaatcccggatgatggt 21
<210> 93
   <211> 21
   <212> DNA
   <213> Artificial
<220>
   <223> PCR primer
<400> 93
   ttacagctagcacggacgttt 21

<210> 94
   <211> 21
   <212> DNA
   <213> Artificial
<220>
   <223> PCR primer
<400> 94
   ttggaaaccaagtcattctgg 21
<210> 95
   <211> 21
   <212> DNA
   <213> Artificial
<220>
   <223> PCR primer
<400> 95
   ggcggagtaaccctatctgtc 21
<210> 96
   <211> > 21
   <212> DNA
   <213> Artificial
<220>
   <223> PCR primer
<400> 96
   gtggtatccacagctgcattt 21
<210> 97
   <211> 21
   <212> DNA
   <213> Artificial
<220>
   <223> PCR primer
<400> 97
   catctgttggagctcttcctg 21
<210> 98
   <211> 21
   <212> DNA
   <213> Artificial
<220>
   <223> PCR primer
<400> 98
   ccaaagcagtccaggtaatca 21
<210> 99
   <211> 21
   <212> DNA
   <213> Artificial
<220>
   <223> PCR primer
<400> 99
   attgctggcaagaatagctga 21
<210> 100
   <211> 21
   <212> DNA
   <213> Artificial
<220>
   <223> PCR primer
<400> 100
   tgaaaagagacctccagcaaa 21
<210> 101
   <211> 21
   <212> DNA
   <213> Artificial
<220>
   <223> PCR primer
<400> 101
   gtttgccaggacttcacagag 21
<210> 102
   <211> 20
   <212> DNA
   <213> Artificial
<220>
   <223> PCR primer
<400> 102
   cgggacattcaccatctttc 20
<210> 103
   <211> 21
   <212> DNA
   <213> Artificial
<220>
   <223> PCR primer
<400> 103
   gctctggagtggatgaacttg 21
<210> 104
   <211> 21
   <212> DNA
   <213> Artificial
<220>
   <223> PCR primer
<400> 104
   gtgcacctcaatctggttgtt 21
<210> 105
   <211> 21
   <212> DNA
   <213> Artificial
<220>
   <223> PCR primer
<400> 105
   aggactcgagcaaggagtttc 21
<210> 106
   <211> 21
   <212> DNA
   <213> Artificial
<220>
   <223> PCR primer
<400> 106
   gtctgttgagctgagcgaact 21
<210> 107
   <211> 21
   <212> DNA
   <213> Artificial
<220>
   <223> PCR primer
<400> 107
   tctggactgcaaagagcattt 21
<210> 108
   <211> 21
   <212> DNA
   <213> Artificial
<220>
   <223> PCR primer
<400> 108
   gctcaaacca aaccaaaaca a 21
<210> 109
   <211> 21
   <212> DNA
   <213> Artificial
<220>
   <223> PCR primer
<400> 109
   cgaagccctt gagaaagagt t 21
<210> 110
   <211> 21
   <212> DNA
   <213> Artificial
<220>
   <223> PCR primer
<400> 110
   actactgctgatggggatgtg 21
<210> 111
   <211> 21
   <212> DNA
   <213> Artificial
<220>
   <223> PCR primer
<400> 111
   ctccacttcaaatgccagtgt 21
<210> 112
   <211> > 21
   <212> DNA
   <213> Artificial
<220>
   <223> PCR primer
<400> 112
   gccaaacatggcttgataaaa 21
<210> 113
   <211> 21
   <212> DNA
   <213> Artificial
<220>
   <223> PCR primer
<400> 113
   tgttcgcttccgagtcttaaa 21
<210> 114
   <211> 21
   <212> DNA
   <213> Artificial
<220>
   <223> PCR primer
<400> 114
   cctttccgtaggaacaaaacc 21
<210> 115
   <211> 21
   <212> DNA
   <213> Artificial
<220>
   <223> PCR primer
<400> 115
   tgcagggacagaagaagaaga 21
<210> 116
   <211> 21
   <212> DNA
   <213> Artificial
<220>
   <223> PCR primer
<400> 116
   tgtgacccataatcccaacat 21
<210> 117
   <211> 21
   <212> DNA
   <213> Artificial
<220>
   <223> PCR primer
<400> 117
   agagtgaggctgcacaacaac 21
<210> 118
   <211> 21
   <212> DNA
   <213> Artificial
<220>
   <223> PCR primer
<400> 118
   atgagagtggccccatagaac 21
<210> 119
   <211> 21
   <212> DNA
   <213> Artificial
<220>
   <223> PCR primer
<400> 119
   ataacatccagccgtcaagtg 21
<210> 120
   <211> 21
   <212> DNA
   <213> Artificial <220>
   <223> PCR primer
<400> 120
   agtttttgcttctgggatggt 21
<210> 121
   <211> 21
   <212> DNA
   <213> Artificial
<220>
   <223> PCR primer
<400> 121
   gagcagaaccactacgacagc 21
<210> 122
   <211> 21
   <212> DNA
   <213> Artificial
<220>
   <223> PCR primer
<400> 122
   cctggaactggtcataggtga a 21
<210> 123
   <211> 21
   <212> DNA
   <213> Artificial
<220>
   <223> PCR primer
<400> 123
   gacgatccag agttcagcaa c 21
<210> 124
   <211> 21
   <212> DNA
   <213> Artificial <220>

   <223> PCR primer
<400> 124
   aggttgtttttgctggtgatg 21
<210> 125
   <211> 21
   <212> DNA
   <213> Artificial
<220>
   <223> PCR primer
<400> 125
   tgtgcttagggaatgtgaag 21
<210> 126
   <211> 21
   <212> DNA
   <213> Artificial
<220>
   <223> PCR primer
<400> 126
   caatgcttttcacactcagca 21
<210> 127
   <211> 21
   <212> DNA
   <213> Artificial
<220>
   <223> PCR primer
<400> 127
   ggacgtttccattcagacaaa 21
<210> 128
   <211> 21
   <212> DNA
   <213> Artificial
<220>
   <223> PCR primer
<400> 128
   agctttgagtcattgcgttgt 21
<210> 129
   <211> 21
   <212> DNA
   <213> Artificial
<220>
   <223> PCR primer
<400> 129
   acgtggtgttcctctacatgc 21
<210> 130
   <211> 21
   <212> DNA
   <213> Artificial
<220>
   <223> PCR primer
<400> 130
   gaaggcctccttacagctctc 21
<210> 131
   <211> 19
   <212> DNA
   <213> Artificial
<220>
   <223> PCR primer
<400> 131
   agatctgcaaacccgtgct 19
<210> 132
   <211> 21
   <212> DNA
   <213> Artificial
<220>
   <223> PCR primer
<400> 132
   tcgagaaacaaccttctgctc 21
<210> 133
   <211> 21
   <212> DNA
   <213> Artificial
<220>
   <223> PCR primer
<400> 133
   aaaacccagcatacacactgc 21
<210> 134
   <211> 21
   <212> DNA
   <213> Artificial
<220>
   <223> PCR primer
<400> 134
   aaggttcaccttcttcgggta 21
<210> 135
   <211> 21
   <212> DNA
   <213> Artificial
<220>
   <223> PCR primer
<400> 135
   cgtctgatttggttgtggact 21
<210> 136
   <211> 21
   <212> DNA
   <213> Artificial
<220>
   <223> PCR primer
<400> 136
   ttcatggctgaccctacaaac 21
<210> 137
   <211> 21
   <212> DNA
   <213> Artificial
<220>
   <223> PCR primer
<400> 137
   caccttctacccgtggctaat 21
<210> 138
   <211> 21
   <212> DNA
   <213> Artificial
<220>
   <223> PCR primer
<400> 138
   cgcttgaactttgtccttctg 21
<210> 139
   <211> 21
   <212> DNA
   <213> Artificial
<220>
   <223> PCR primer
<400> 139
   gagaagaacg ccacacactt c 21
<210> 140
   <211> 21
   <212> DNA
   <213> Artificial
<220>
   <223> PCR primer
<400> 140
   tgttgacctccagctctgtct 21
<210> 141
   <211> 21
   <212> DNA
   <213> Artificial
<220>
   <223> PCR primer
<400> 141
   at cagact ac ccat ccccat c 21
<210> 142
   <211> 21
   <212> DNA
   <213> Artificial
<220>
   <223> PCR primer
<400> 142
   gggagtcttctcctgtcatcc 21
<210> 143
   <211> 21
   <212> DNA
   <213> Artificial
<220>
   <223> PCR pr i rrer
<400> 143
   ctcaccatgtggatgaaggat 21
<210> 144
   <211> 21
   <212> DNA
   <2.13> Artificial
<220>
   <223> PCR primer
<400> 144
   acgatgagggtgtagttcacg 21
<210> 145
   <211> 21
   <212> DNA
   <213> Artificial
<220>
   <223> PCR primer
<400> 145
   gtctctctcactcgggctttt 21
<210> 146
   <211> 21
   <212> DNA
   <213> Artificial
<220>
   <223> PCR primer
<400> 146
   tgtctgaggactgagctggat 21
<210> 147
   <211> 21
   <212> DNA
   <213> Artificial
<220>
   <223> PCR primer
<400> 147
   gtgctatccctacccaaaagc 21
<210> 148
   <211> 21
   <212> DNA
   <213> Artificial
<220>
   <223> PCR primer
<400> 148
   ggcttggctgtcaacatacat 21
<210> 149
   <211> 21
   <212> DNA
   <213> Artificial
<220>
   <223> PCR primer
<400> 149
   tgagaaaagagggcattcaga 21
<210> 150
   <211> 21
   <212> DNA
   <213> Artificial
<220>
   <223> PCR primer
<400> 150
   gcaggatgaattgaagatcca 21
<210> 151
   <211> 21
   <212> DNA
   <213> Artificial <220>

   <223> PCR pr i rrer
<400> 151
   gcagaaccacagggtatcaga 21
<210> 152
   <211> 21
   <212> DNA
   <213> Artificial
<220>
   <223> PCR primer
<400> 152
   tcgagcacacattcccttaac 21
<210> 153
   <211> 21
   <212> DNA
   <213> Artificial
<220>
   <223> PCR primer
<400> 153
   cccttttgcaaggagaatacc 21
<210> 154
   <211> 21
   <212> DNA
   <213> Artificial
<220>
   <223> PCR primer
<400> 154
   gctggtctctgtccatgtgat 21
<210> 155
   <211> 21
   <212> DNA
   <213> Artificial
<220>
   <223> PCR primer
<400> 155
   cagtatggtgctggtgtttgg 21
<210> 156
   <211> 21
   <212> DNA
   <213> Artificial
<220>
   <223> PCR primer
<400> 156
   catacatgtgtcccccactct 21
<210> 157
   <211> 21
   <212> DNA
   <213> Artificial
<220>
   <223> PCR primer
<400> 157
   cacgctcacaggacagtgtta 21
<210> 158
   <211> 21
   <212> DNA
   <213> Artificial
<220>
   <223> PCR primer
<400> 158
   gttgtcatcagatgcctgga t 21
<210> 159
   <211> 21
   <212> DNA
   <213> Artificial
<220>
   <223> PCR primer
<400> 159
   gggacagggaaatctgaagag 21
<210> 160
   <211> 21
   <212> DNA
   <213> Artificial
<220>

   <223> PCR primer
<400> 160
   ccttgcgtgtaatttttgcat 21
<210> 161
   <211> 21
   <212> DNA
   <213> Artificial
<220>
   <223> PCR primer
<400> 161
   taccactggagccaatttcac 21
<210> 162
   <211> 21
   <212> DNA
   <213> Artificial
<220>
   <223> PCR primer
<400> 162
   cagggggaacttattcagagg 21
<210> 163
   <211> 21
   <212> DNA
   <213> Artificial
<220>
   <223> PCR primer
<400> 163
   gatgggtttgctgagatgtgt 21
<210> 164
   <211> 21
   <212> DNA
   <213> Artificial
<220>
   <223> PCR primer
<400> 164
   gcccctttagagaccattttg 21
<210> 165
   <211> 21
   <212> DNA
   <213> Artificial
<220>
   <223> PCR primer
<400> 165
   atgcccagtctaggaaatcgt 21
<210> 166
   <211> > 21
   <212> DNA
   <213> Artificial
<220>
   <223> PCR primer
<400> 166
   ctccagtgtgggttctgatgt 21
<210> 167
   <211> 21
   <212> DNA
   <213> Artificial
<220>
   <223> PCR primer
<400> 167
   acatcaacaccaagtgcatca 21
<210> 168
   <211> 21
   <212> DNA
   <213> Artificial
<220>
   <223> PCR primer
<400> 168
   ggcgt act ca cacat ct cca t **2**1
<210> 169
   <211> 21
   <212> DNA
   <213> Artificial
<220>
   <223> PCR primer
<400> 169
   acacccagctgttcaccatag 21
<210> 170
   <211> 21
   <212> DNA
   <213> Artificial
<220>
   <223> PCR primer
<400> 170
   ctgaagcaggagcagctctaa 21
<210> 171
   <211> 21
   <212> DNA
   <213> Artificial
<220>
   <223> PCR primer
<400> 171
   tcaacgagttcgaccaatacc 21
<210> 172
   <211> 21
   <212> DNA
   <213> Artificial
<220>
   <223> PCR primer
<400> 172
   gctcagctgctctgttttgat 21
<210> 173
   <211> 21
   <212> DNA
   <213> Artificial
<220>
   <223> PCR primer
<400> 173
   tggaggaagtcagggaagaat 21
<210> 174
   <211> 21
   <212> DNA
   <213> Artificial
<220>
   <223> PCR primer
<400> 174
   agatctggggatgaggtgtct 21
<210> 175
   <211> 21
   <212> DNA
   <213> Artificial
<220>
   <223> PCR primer
<400> 175
   gacaagcaggattacccacaa 21
<210> 176
   <211> 21
   <212> DNA
   <213> Artificial
<220>
   <223> PCR primer
<400> 176
   ctgggcatcgaacattaggta 21
<210> 177
   <211> 21
   <212> DNA
   <213> Artificial
<220>
   <223> PCR primer
<400> 177
   gtcaggtagccacaagacca 21
<210> 178
   <211> 21
   <212> DNA
   <213> Artificial
<220>
   <223> PCR primer
<400> 178
   gaccatgccctgtcttcagta 21
<210> 179
   <211> 21
   <212> DNA
   <213> Artificial
<220>
   <223> PCR primer
<400> 179
   cttgacgtagcagagggaaaa 21
<210> 180
   <211> 21
   <212> DNA
   <213> Artificial
<220>
   <223> PCR primer
<400> 180
   aagctttgcagagtcaagtgc 21
<210> 181
   <211> 21
   <212> DNA
   <213> Artificial
<220>
   <223> PCR primer
<400> 181
   gctggacccttctctctctgt 21
<210> 182
   <211> 21
   <212> DNA
   <213> Artificial
<220>
   <223> PCR primer
<400> 182
   ccgttacagcgagatgtcatt 21
<210> 183
   <211> 21
   <212> DNA
   <213> Artificial
<220>
   <223> PCR primer
<400> 183
   aaccgtctcattgacacaagg 21
<210> 184
   <211> 20
   <212> DNA
   <213> Artificial
<220>
   <223> PCR primer
<400> 184
   accttcaggc tttcttgctt 20
<210> 185
   <211> 21
   <212> DNA
   <213> Artificial
<220>
   <223> PCR primer
<400> 185 atccaggggtcaggaagtcta 21
<210> 186
   <211> 21
   <212> DNA
   <213> Artificial
<220>
   <223> PCR primer
<400> 186
   acccttgaggcagtttttcat 21
<210> 187
   <211> 21
   <212> DNA
   <213> Artificial
<220>
   <223> PCR primer
<400> 187
   caaaagaaag agcagccaat g 21
<210> 188
   <211> 21
   <212> DNA
   <213> Artificial
<220>
   <223> PCR primer
<400> 188
   cccatcgtacttgcaggagta 21
<210> 189
   <211> 21
   <212> DNA
   <213> Artificial
<220>
   <223> PCR primer
<400> 189
   aatgagcacc ctcttcgaaa t 21
<210> 190
   <211> 21
   <212> DNA
   <213> Artificial
<220>
   <223> PCR primer
<400> 190
   tgacgctctg gacctcttaaa 21
<210> 191
   <211> 21
   <212> DNA
   <213> Artificial
<220>
   <223> PCR primer
<400> 191
   aggccgaggaagaaattatga 21
<210> 192
   <211> 21
   <212> DNA
   <213> Artificial
<220>
   <223> PCR primer
<400> 192
   actgcaggag gt ggt at t cc t 21
<210> 193
   <211> 21
   <212> DNA
   <213> Artificial
<220>
   <223> PCR primer
<400> 193
   gccaagagaa aaaggcagag t 21
<210> 194
   <211> 21
   <212> DNA
   <213> Artificial
<220>
   <223> PCR primer
<400> 194
   tccgttgagg acacatccta c 21
<210> 195
   <211> 21
   <212> DNA
   <213> Artificial
<220>
   <223> PCR primer
<400> 195
   cccagtcctg tcctgttcat a 21
<210> 196
   <211> 21
   <212> DNA
   <213> Artificial
<220>
   <223> PCR primer
<400> 196
   cggtctcgta cgtctaacag c **2**1
<210> 197
   <211> 21
   <212> DNA
   <213> Artificial
<220>
   <223> PCR primer
<400> 197
   acaaat t gga ccatgtggtg t 21
<210> 198
   <211> 21
   <212> DNA
   <213> Artificial
<220>
   <223> PCR primer
<400> 198
   gtccttatac tgccggtcct c 21
<210> 199
   <211> 21
   <212> DNA
   <213> Artificial
<220>
   <223> PCR primer
<400> 199
   ttctttgagc cagtctttgg a 21
<210> 200
   <211> 21
   <212> DNA
   <213> Artificial
<220>
   <223> PCR primer
<400> 200
   cagggggt cc ttaaacaaga g 21
<210> 201
   <211> 21
   <212> DNA
   <213> Artificial
<220>
   <223> PCR primer
<400> 201
   gccacctccg tcctttacta c 21
<210> 202
   <211> 21
   <212> DNA
   <213> Artificial
<220>
   <223> PCR primer
<400> 202
   tttacatcca cgtctgggaa g 21
<210> 203
   <211> 21
   <212> DNA <213> Artificial
<220>
   <223> PCR primer
<400> 203
   tcttctacaa gcagcccatg t 21
<210> 204
   <211> 21
   <212> DNA
   <213> Artificial
<220>
   <223> PCR primer
<400> 204
   gt cct caat gacaccgacag t 21
<210> 205
   <211> 21
   <212> DNA
   <213> Artificial
<220>
   <223> PCR primer
<400> 205
   atggacgaaa tgcctgtagt g 21
<210> 206
   <211> 21
   <212> DNA
   <213> Artificial
<220>
   <223> PCR primer
<400> 206
   tgacgtccag tttttccattc 21
<210> 207
   <211> 21
   <212> DNA
   <213> Artificial
<220>
   <223> PCR primer
<400> 207
   ccccttgtca gttacaggtc a 21
<210> 208
   <211> 21
   <212> DNA
   <213> Artificial
<220>
   <223> PCR primer
<400> 208
   gtttccttgg aatttgcctt c 21

<210> 209
   <211> 21
   <212> DNA
   <213> Artificial
<220>
   <223> PCR primer
<400> 209
   gactgctttg tcaacctcag c 21
<210> 210
   <211> 21
   <212> DNA
   <213> Artificial
<220>
   <223> PCR primer
<400> 210
   gctgttgcag aagatgagga c 21
<210> 211
   <211> 20
   <212> DNA
   <213> Artificial
<220>
   <223> PCR primer
<400> 211
   cactgacaac cctgatggtg 20
<210> 212
   <211> 20
   <212> DNA
   <213> Artificial
<220>
   <223> PCR primer
<400> 212
   tggcactggc atatgtagga 20
<210> 213
   <211> 21
   <212> DNA
   <213> Artificial
<220>
   <223> PCR primer
<400> 213
   ttgaccaaat ttccattgag g 21
<210> 214
   <211> 21
   <212> DNA
   <213> Artificial
<220>
   <223> PCR primer
<400> 214
   acagaagaac cccagaccag t 21
<210> 215
   <211> 20
   <212> DNA
   <213> Artificial
<220>
   <223> PCR primer
<400> 215
   caccagtcaggacccaaagt 20
<210> 216
   <211> 20
   <212> DNA
   <213> Artifcial
<220>
   <223> PCR primer
<400> 216
   ggggatgaaa cctttttggt 20
<210> 217
   <211> 21
   <212> DNA
   <213> Artificial
<220>
   <223> PCR primer
<400> 217
   gggacttcct caagtggtag g 21
<210> 218
   <211> 21
   <212> DNA
   <213> Artificial
<220>
   <223> PCR primer
<400> 218
   acgtggaagg aagaagtgga t 21
<210> 219
   <211> 21
   <212> DNA
   <213> Artificial
<220>
   <223> PCR primer
<400> 219
   acaagaaaat caatgcgatg g 21
<210> 220
   <211> 21
   <212> DNA
   <213> Artificial
<220>
   <223> PCR primer
<400> 220
   gttgttctcc agcacgatct c 21
<210> 221
   <211> 21
   <212> DNA
   <213> Artificial
<220>
   <223> PCR primer
<400> 221
   ccagagaggc ctagaaagga a 21
<210> 222
   <211> 21
   <212> DNA
   <213> Artificial
<220>
   <223> PCR primer
<400> 222
   tcctctgctg cactgaagatt 21
<210> 223
   <211> 21
   <212> DNA
   <213> Artificial
<220>
   <223> PCR primer
<400> 223
   aaacgagaca gctccatcag a 21
<210> 224
   <211> 21
   <212> DNA
   <213> Artificial
<220>
   <223> PCR primer
<400> 224
   ctgccctaag tttacccaag g 21
<210> 225
   <211> 21
   <212> DNA
   <213> Artificial
<220>
   <223> PCR primer
<400> 225
   acaagaacga cgtcatgatcc c **2**1
<210> 226
   <211> 21
   <212> DNA
   <213> Artificial,
<220>
   <223> PCR primer
<400> 226
   cgtggtaaag gagatgtgct c 21
<210> 227
   <211> 20
   <212> DNA
   <213> Artificial
<220>
   <223> PCR primer
<400> 227
   agctgcacgt cgaagttttt 20
<210> 228
   <211> 20
   <212> DNA
   <213> Artificial
<220>
   <223> PCR primer
<400> 228
   ttccttgtcc cactctcacc 20
<210> 229
   <211> 21
   <212> DNA
   <213> Artificial
<220>
   <223> PCR primer
<400> 229
   acatgatggt ggactttgag c 21
<210> 230
   <211> 21
   <212> DNA
   <213> Artificial
<220>
   <223> PCR primer
<400> 230
   gtacagcatg gagaggggac t 21
<210> 231
   <211> 21
   <212> DNA
   <213> Artificial
<220>
   <223> PCR primer
<400> 231
   gtcttcgatg ccatgttcaa t 21
<210> 232
   <211> 21
   <212> DNA
   <213> Artificial <220>

   <223> PCR primer
<400> 232
   gt ct gct cgg aggttcttct t 21
<210> 233
   <211> 21
   <212> DNA
   <213> Artificial
<220>
   <223> PCR primer
<400> 233
   agcaagcagc aaagcagata g 21
<210> 234
   <211> 21
   <212> DNA
   <213> Artificial
<220>
   <223> PCR primer
<400> 234
   aactgaaccg tcctttgacc t 21
<210> 235
   <211> 21
   <212> DNA
   <213> Artificial
<220>
   <223> PCR primer
<400> 235
   taacccagtc ggattgctat g 21
<210> 236
   <211> 21
   <212> DNA
   <213> Artificial
<220>
   <223> PCR primer
<400> 236
   ctggattttg gagacatcga a 21
<210> 237
   <211> 21
   <212> DNA
   <213> Artificial
<220>
   <223> PCR primer
<400> 237
   ccaagaccaa atgcaagtca t 21
<210> 238
   <211> 21
   <212> DNA
   <213> Artificial
<220>
   <223> PCR primer
<400> 238
   aatgcactgc aatgtttttc c 21
<210> 239
   <211> 22
   <212> DNA
   <213> Artificial
<220>
   <223> PCR primer
<400> 239
   tacctaactg gcatagagac ct 22
<210> 240
   <211> 19
   <212> DNA
   <213> Artificial
<220>
   <223> PCR primer
<400> 240
   tcctgtggaa gacaatgga 19
<210> 241
   <211> 21
   <212> DNA
   <213> Artificial
<220>
   <223> PCR primer
<400> 241
   tttcagctct tggacctttc a 21
<210> 242
   <211> 21
   <212> DNA
   <213> Artificial
<220>
   <223> PCR primer
<400> 242
   ttttcaagtt cttccgcttga 21
<210> 243
   <211> 21
   <212> DNA
   <213> Artificial
<220>
   <223> PCR primer
<400> 243
   gtttctgcga cacctgagaa g 21
<210> 244
   <211> 21
   <212> DNA
   <213> Artificial
<220>
   <223> PCR primer
<400> 244
   ttcacatgca gcaagattga g 21
<210> 245
   <211> 21
   <212> DNA
   <213> Artificial <220>
   <223> PCR primer
<400> 245
   gcagagaaaa gggaaaaagg a 21
<210> 246
   <211> 21
   <212> DNA
   <213> Artificial
<220>
   <223> PCR primer
<400> 246
   tttcctaggg aggggtatga a 21
<210> 247
   <211> 21
   <212> DNA
   <213> Artificial
<220>
   <223> PCR primer
<400> 247
   tagatggaga gcgtcaggaa a 21
<210> 248
   <211> 21
   <212> DNA
   <213> Artificial
<220>
   <223> PCR primer
<400> 248
   catgactcag gaacacggt t 21
<210> 249
   <211> 21
   <212> DNA
   <213> Artificial
<220>
   <223> PCR primer
<400> 249
   caacgcatccctaatttctg a 21
<210> 250
   <211> 21
   <212> DNA
   <213> Artificial
<220>
   <223> PCR primer
<400> 250
   acggacagac caaaagaggt t 21
<210> 251
   <211> 20
   <212> DNA
   <213> Artificial
<220>
   <223> PCR primer
<400> 251
   gggaaatgat ggacaaaacg 20
<210> 252
   <211> 20
   <212> DNA
   <213> Artificial
<220>
   <223> PCR primer
<400> 252
   ggcagtcgtttcaagagagg 20
<210> 253
   <211> 21
   <212> DNA
   <213> Artificial
<220>
   <223> PCR primer
<400> 253
   cccaatagaa ggggcataga g 21
<210> 254
   <211> 21
   <212> DNA
   <213> Artificial
<220>
   <223> PCR primer
<400> 254
   ct t ggt aact gcgggaatac a 21
<210> 255
   <211> 21
   <212> DNA
   <213> Artificial
<220>
   <223> PCR primer
<400> 255
   t ct cct caac ctgaagaacg a 21
<210> 256
   <211> 21
   <212> DNA
   <213> Artificial
<220>
   <223> PCR primer
<400> 256
   cttggcaacc atttgaggta a 21
<210> 257
   <211> 21
   <212> DNA
   <213> Artificial
<220>
   <223> PCR primer
<400> 257 21
   gtgcagacaa tggaggacat t 21
<210> 258
   <211> 21
   <212> DNA
   <213> Artificial
<220>
   <223> PCR primer
<400> 258 21
   tcttggttgt tggtttcttg g 21
<210> 259
   <211> 21
   <212> DNA
   <213> Artificial
<220>
   <223> PCR primer
<400> 259 21
   tcaggattct tgctgctttg t 21
<210> 260
   <211> 21
   <212> DNA
   <213> Artificial
<220>
   <223> PCR primer
<400> 260
   ggatggtaga gcccttggta g 21
<210> 261
   <211> 21
   <212> DNA
   <213> Artificial
<220>
   <223> PCR primer
<400> 261
   ccaagcagct acgattctgt c 21
<210> 262
   <211> 21
   <212> DNA
   <213> Artificial
<220>
   <223> PCR primer
<400> 262
   ggattccctg gtagctttgt c 21
<210> 263
   <211> 21
   <212> DNA
   <213> Artificial
<220>
   <223> PCR primer
<400> 263
   ggctggtttg tgtttgacat t 21
<210> 264
   <211> 21
   <212> DNA
   <213> Artificial
<220>
   <223> PCR primer
<400> 264
   ccatgaatgg ctgttttgac t 21
<210> 265
   <211> 21
   <212> DNA
   <213> Artificial
<220>
   <223> PCR primer
<400> 265
   aggagaagag cacactgacc a 21
<210> 266
   <211> 21
   <212> DNA
   <213> Artificial
<220>
   <223> PCR primer
<400> 266
   gtgtccagat agctgccaat g 21
<210> 267
   <211> 21
   <212> DNA
   <213> Artificial
<220>
   <223> PCR primer
<400> 267
   cagtgcaaag acctgaggaa g 21
<210> 268
   <211> 21
   <212> DNA
   <213> Artificial
<220>
   <223> PCR primer
<400> 268
   cttcagtcag gatggaagca g 21
<210> 269
   <211> 21
   <212> DNA
   <213> Artificial
<220>
   <223> PCR primer
<400> 269
   ccttgccaac atctctctct g 21
<210> 270
   <211> 21
   <212> DNA
   <213> Artificial
<220>
   <223> PCR primer
<400> 270
   caagcgagag tccattttct g 21
<210> 271
   <211> 21
   <212> DNA
   <213> Artificial
<220>
   <223> PCR primer
<400> 271
   t ct t at tact ggagcgcctg a 21
<210> 272
   <211> 21
   <212> DNA
   <213> Artificial
<220>
   <223> PCR primer
<400> 272
   caggatggag agagtttgct g 21
<210> 273
   <211> 21
   <212> DNA
   <213> Artificial
<220>
   <223> PCR primer
<400> 273
   ctggaagagc ttggatgtct g 21
<210> 274
   <211> 21
   <212> DNA
   <213> Artificial
<220>
   <223> PCR primer
<400> 274
   tcctctgcct gtcttactgg a 21
<210> 275
   <211> 21
   <212> DNA
   <213> Artificial
<220>
   <223> PCR primer
<400> 275
   atctgattgc acagagccag t 21
<210> 276
   <211> 21
   <212> DNA
   <213> Artificial
<220>
   <223> PCR pr i rrer
<400> 276
   accctgatgt cattgcagaa c 21
<210> 277
   <211> 21
   <212> DNA
   <213> Artificial
<220>
   <223> PCR primer
<400> 277
   ggacctggaa caggttttct c 21
<210> 278
   <211> 21
   <212> DNA
   <213> Artificial
<220>
   <223> PCR primer
<400> 278
   agcaactgca tgtccattac c 21
<210> 279
   <211> 21
   <212> DNA
   <213> Artificial
<220>
   <223> PCR primer
<400> 279
   tcaggaaagc cagaacagaa a 21
<210> 280
   <211> 21
   <212> DNA
   <213> Artificial
<220>
   <223> PCR primer
<400> 280
   agacagctga ggaaggtgtg g a 21
<210> 281
   <211> 21
   <212> DNA
   <213> Artificial
<220>
   <223> PCR primer
<400> 281
   acacttgttc gcagagcagt t 21
<210> 282
   <211> 21
   <212> DNA
   <213> Artificial
<220>
   <223> PCR pr i rrer
<400> 282
   gccacctcaa t aagggt t ag g 21
<210> 283
   <211> 21
   <212> DNA
   <213> Artificial
<220>
   <223> PCR primer
<400> 283
   cctgaatgct cttttcagtgc 21
<210> 284
   <211> 21
   <212> DNA
   <213> Artificial
<220>
   <223> PCR primer
<400> 284
   cgct t ct cac cactgaccta c 21
<210> 285
   <211> 21
   <212> DNA
   <213> Artificial
<220>
   <223> PCR primer
<400> 285
   tcagacctga tccagagcct a 21
<210> 286
   <211> 21
   <212> DNA
   <213> Artificial
<220>
   <223> PCR primer
<400> 286
   cattgcaaca ccagtgacat c 21
<210> 287
   <211> 21
   <212> DNA
   <213> Artificial
<220>
   <223> PCR primer
<400> 287
   gcgaaggaga ggctcaatac t 21
<210> 288
   <211> 21
   <212> DNA
   <213> Artificial
<220>
   <223> PCR primer
<400> 288
   ggctgtgatt ccacgataaa a 21
<210> 289
   <211> 21
   <212> DNA
   <213> Artificial
<220>
   <223> PCR primer
<400> 289
   atcaataccc tggaaccctt g 21
<210> 290
   <211> 21
   <212> DNA
   <213> Artificial
<220>
   <223> PCR primer
<400> 290
   gct cat cagc atcaaatcca t 21
<210> 291
   <211> 20
   <212> DNA
   <213> Artificial
<220>
   <223> PCR primer
<400> 291
   ggtgtgcgga gataaggtgt 20
<210> 292
   <211> 20
   <212> DNA
   <213> Artificial
<220>
   <223> PCR primer
<400> 292
   gctcgcacag cttctagctt 20
<210> 293
   <211> 21
   <212> DNA
   <213> Artificial
<220>
   <223> PCR primer
<400> 293
   attttgcagg tgggcttaac t 21
<210> 294
   <211> 21
   <212> DNA
   <213> Artificial
<220>
   <223> PCR primer
<400> 294
   aaacatggcc ccattctaaa c 21
<210> 295
   <211> 21
   <212> DNA
   <213> Artificial
<220>
   <223> PCR primer
<400> 295
   gccggtatga ccagtacaag a 21
<210> 296
   <211> 21
   <212> DNA
   <213> Artificial
<220>
   <223> PCR primer
<400> 296
   cttgaagctc ctgttcctcc t 21
<210> 297
   <211> 21
   <212> DNA
   <213> Artificial
<220>
   <223> PCR primer
<400> 297
   aacaacaaaa tggtggagtg c 21
<210> 298
   <211> 21
   <212> DNA
   <213> Artificial
<220>
   <223> PCR primer
<400> 298
   ggtgcaatgc cagtgtaact t 21
<210> 299
   <211> 21
   <212> DNA
   <213> Artificial
<220>
   <223> PCR primer
<400> 299
   aacgaggagg agctgaaaaa g 21
<210> 300
   <211> 21
   <212> DNA
   <213> Artificial
<220>
   <223> PCR primer
<400> 300
   tgtagggaat gttgaggttg c 21
<210> 301
   <211> 21
   <212> DNA
   <213> Artificial
<220>
   <223> PCR primer
<400> 301
   agccctgatc atcctcttga t 21
<210> 302
   <211> 21
   <212> DNA
   <213> Artificial
<220>
   <223> PCR primer
<400> 302
   cctggtcctc agtctttacc c 21
<210> 303
   <211> 21
   <212> DNA
   <213> Artificial
<220>
   <223> PCR primer
<400> 303
   gcatttcttt gcttgtgctt t 21
<210> 304
   <211> 21
   <212> DNA
   <213> Artificial
<220>
   <223> PCR primer
<400> 304
   cct ggggt cg tatttttctt c 21
<210> 305
   <211> 20
   <212> DNA
   <213> Artificial
<220>
   <223> PCR primer
<400> 305
   ct gct ct ggc catagtgaca 20
<210> 306
   <211> 20
   <212> DNA
   <213> Artificial
<220>
   <223> PCR primer
<400> 306
   cagcaat t ga ctgctttcca 20
<210> 307
   <211> 21
   <212> DNA
   <213> Artificial
<220>
   <223> PCR primer
<400> 307
   ctggagcggt acaacagaga g 21
<210> 308
   <211> 21
   <212> DNA
   <213> Artificial
<220>
   <223> PCR primer
<400> 308
   ggcaaagct atgcttatct g 21
<210> 309
   <211> 21
   <212> DNA
   <213> Artificial
<220>
   <223> PCR primer
<400> 309
   aatcctccgg gtaccactat g 21
<210> 310
   <211> 21
   <212> DNA
   <213> Artificial
<220>
   <223> PCR primer
<400> 310
   tcttcttatt gcggtcattg c 21
<210> 311
   <211> 21
   <212> DNA
   <213> Artificial
<220>
   <223> PCR primer
<400> 311
   t ct acaaacc gtgcttcgtc c t 21
<210> 312
   <211> 21
   <212> DNA
   <213> Artificial
<220>
   <223> PCR primer
<400> 312
   tcctccttca cctccttctt c 21
<210> 313
   <211> 21
   <212> DNA
   <213> Artificial
<220>
   <223> PCR primer
<400> 313
   tgaacgaaga catccgtatc c 21
<210> 314
   <211> 21
   <212> DNA
   <213> Artificial
<220>
   <223> PCR primer
<400> 314
   ctccagaagt gctcaaggat g 21
<210> 315
   <211> 21
   <212> DNA
   <213> Artificial
<220>
   <223> PCR primer
<400> 315
   cccaactaaa accaacgttc a 21
<210> 316
   <211> 21
   <212> DNA
   <213> Artificial
<220>
   <223> PCR primer
<400> 316
   tgaactgttt ttcggccatag 21
<210> 317
   <211> 20
   <212> DNA
   <213> Artificial
<220>
   <223> PCR primer
<400> 317
   cagcgaagat gacaagtgga 20
<210> 318
   <211> 20
   <212> DNA
   <213> Artificial
<220>
   <223> PCR primer
<400> 318
   tatccgtcaacttccgaacc 20
<210> 319
   <211> 21
   <212> DNA
   <213> Artificial
<220>
   <223> PCR primer
<400> 319
   acagcaagtacttcggcaag a 21
<210> 320
   <211> 21
   <212> DNA
   <213> Artificial
<220>
   <223> PCR primer
<400> 320
   actgctgcgacttctcatag c 21
<210> 321
   <211> 21
   <212> DNA
   <213> Artificial
<220>
   <223> PCR primer
<400> 321
   catggagagaaacagggttca 21
<210> 322
   <211> 21
   <212> DNA
   <213> Artificial
<220>
   <223> PCR primer
<400> 322
   cacctcccac agcacataac t 21
<210> 323
   <211> 20
   <212> DNA
   <213> Artificial
<220>
   <223> PCR primer
<400> 323
   accgct t gt caccagttac 20

<210> 324
   <211> 20
   <212> DNA
   <213> Artificial
<220>
   <223> PCR primer
<400> 324
   ggcaacgcaggaatttatgt 20
<210> 325
   <211> 21
   <212> DNA
   <213> Artificial
<220>
   <223> PCR primer
<400> 325
   tgtctgcatagactgcacca c 21
<210> 326
   <211> 21
   <212> DNA
   <213> Artificial
<220>
   <223> PCR primer
<400> 326
   tagtgtgcacatgcctacag c 21
<210> 327
   <211> 21
   <212> DNA
   <213> Artificial
<220>
   <223> PCR primer
<400> 327
   ctgataagtgccatccgttg t 21
<210> 328
   <211> 21
   <212> DNA
   <213> Artificial
<220>
   <223> PCR primer
<400> 328
   ggtttttcaacatgctttgga 21
<210> 329
   <211> 21
   <212> DNA
   <213> Artificial
<220>
   <223> PCR primer
<400> 329
   tggagatcctcaaagtcatgg 21
<210> 330
   <211> 21
   <212> DNA
   <213> Artificial
<220>
   <223> PCR primer
<400> 330
   gtcccttacacttcccaaag c 21
<210> 331
   <211> 21
   <212> DNA
   <213> Artificial
<220>
   <223> PCR primer
<400> 331
   gaaggaagca caggaagctc t 21
<210> 332
   <211> 21
   <212> DNA
   <213> Artificial
<220>
   <223> PCR primer
<400> 332
   gcatgtcatccatgttcactg 21
<210> 333
   <211> 21
   <212> DNA
   <213> Artificial
<220>
   <223> PCR primer
<400> 333
   ggaccaataagatggctctgcc 21
<210> 334
   <211> 21
   <212> DNA
   <213> Artificial
<220>
   <223> PCR primer
<400> 334
   atgagcgtgtcctcatacagg 21
<210> 335
   <211> 20
   <212> DNA
   <213> Artificial
<220>
   <223> PCR primer
<400> 335
   acctggagat cttccccaac 20
<210> 336
   <211> 20
   <212> DNA
   <213> Artificial
<220>
   <223> PCR primer
<400> 336
   cccttgtgag cttcttcgac 20
<210> 337
   <211> 21
   <212> DNA
   <213> Artificial
<220>
   <223> PCR primer
<400> 337
   atgaacatgtcctacgccaa c 21
<210> 338
   <211> 21
   <212> DNA
   <213> Artificial
<220>
   <223> PCR primer
<400> 338
   gaggttggattgcccatagat 21
<210> 339
   <211> 21
   <212> DNA
   <213> Artificial
<220>
   <223> PCR primer
<400> 339
   tctggaggagaacacagagg a 21
<210> 340
   <211> 21
   <212> DNA
   <213> Artificial
<220>
   <223> PCR primer
<400> 340
   gaactgtgagccattggtgt t 21
<210> 341
   <211> 21
   <212> DNA
   <213> Artificial
<220>
   <223> PCR primer
<400> 341
   gatgcgttaacacgggatct a 21
<210> 342
   <211> 21
   <212> DNA
   <213> Artificial
<220>
   <223> PCR primer
<400> 342
   ttcaggagcagccaaagttt a 21
<210> 343
   <211> 21
   <212> DNA
   <213> Artificial
<220>
   <223> PCR primer
<400> 343
   gatgatgggaactcagccat a 21
<210> 344
   <211> 21
   <212> DNA
   <213> Artificial
<220>
   <223> PCR primer
<400> 344
   tccaatacgccagtttggta g 21
<210> 345
   <211> 21
   <212> DNA
   <213> Artificial
<220>
   <223> PCR primer
<400> 345
   gacttccttatgctccccat c 21
<210> 346
   <211> 21
   <212> DNA
   <213> Artificial
<220>
   <223> PCR primer
<400> 346
   ggaaaaggtagcttccctcct 21
<210> 347
   <211> 21
   <212> DNA
   <213> Artificial
<220>
   <223> PCR primer
<400> 347
   tagagcagagatggtggcact 21
<210> 348
   <211> 21
   <212> DNA
   <213> Artificial
<220>
   <223> PCR primer
<400> 348
   aagttgtgggtgtgagtgagg 21
<210> 349
   <211> 21
   <212> DNA.
   <213> Artificial
<220>
   <223> PCR primer
<400> 349
   atctttcacccttgcattgtg 21
<210> 350
   <211> 21
   <212> DNA
   <213> Artificial
<220>
   <223> PCR primer
<400> 350
   ggttcccactgtcaagaaac a 21
<210> 351
   <211> 21
   <212> DNA
   <213> Artificial
<220>
   <223> PCR primer
<400> 351
   gagctcctgttgtcaaacgt c 21
<210> 352
   <211> 21
   <212> DNA
   <213> Artificial
<220>
   <223> PCR primer
<400> 352
   agccataaac agcagaaagc a 21
<210> 353
   <211> 21
   <212> DNA
   <213> Artificial
<220>
   <223> PCR primer
<400> 353
   gtccagtttgttggtgaagg a 21
<210> 354
   <211> 21
   <212> DNA
   <213> Artificial
<220>
   <223> PCR primer
<400> 354
   gggaggagagagtcgttatgg 21
<210> 355
   <211> 21
   <212> DNA
   <213> Artificial
<220>
   <223> PCR primer
<400> 355
   aatccctttgtgaccaattc c 21
<210> 356
   <211> 21
   <212> DNA
   <213> Artificial
<220>
   <223> PCR primer
<400> 356
   tctactccaaggaggggaaa a 21
<210> 357
   <211> 21
   <212> DNA
   <213> Artificial
<220>
   <223> PCR primer
<400> 357
   gtcggctgtactctgtccaa g 21
<210> 358
   <211> 21
   <212> DNA
   <213> Artificial
<220>
   <223> PCR primer
<400> 358 21
   cgacttgatgaagatggggt a 21
<210> 359
   <211> 21
   <212> DNA
   <213> Artificial
<220>
   <223> PCR primer
<400> 359 21
   act ggaaagggctctaagctg 21
<210> 360
   <211> 21
   <212> DNA
   <213> Artificial
<220>
   <223> PCR primer
<400> 360 21
   caacattccagtggcttcaa t 21
<210> 361
   <211> 21
   <212> DNA
   <213> Artificial
<220>
   <223> PCR primer
<400> 361 21
   gctgacttggaagcagacaa c 21
<210> 362
   <211> 21
   <212> DNA
   <213> Artificial
<220>
   <223> PCR primer
<400> 362 21
   tccaggaactgctcaagaag a 21
<210> 363
   <211> 21
   <212> DNA
   <213> Artificial
<220>
   <223> PCR primer
<400> 363 21
   ctccttgctggatgagaaca g 21
<210> 364
   <211> 21
   <212> DNA
   <213> Artificial
<220>
   <223> PCR primer
<400> 364
   gtgctccagcaggtacatca t 21
<210> 365
   <211> 21
   <212> DNA
   <213> Artificial
<220>
   <223> PCR primer
<400> 365
   ggaacagctgctaaacgtca c 21
<210> 366
   <211> 21
   <212> DNA
   <213> Artificial
<220>
   <223> PCR primer
<400> 366
   tctagggttctcgcttccttc 21
<210> 367
   <211> 21
   <212> DNA
   <213> Artificial
<220>
   <223> PCR primer
<400> 367
   ctccatgcactctctctacg g 21
<210> 368
   <211> 21
   <212> DNA
   <213> Artificial
<220>
   <223> PCR primer
<400> 368
   tcaccaggaaatatgctaccg 21
<210> 369
   <211> 21
   <212> DNA
   <213> Artificial
<220>
   <223> PCR primer
<400> 369
   ggcatttctccatttctttc c 21
<210> 370
   <211> > 21
   <212> DNA
   <213> Artificial
<220>
   <223> PCR primer
<400> 370
   agctccttgatctgctccttc 21
<210> 371
   <211> 21
   <212> DNA
   <213> Artificial
<220>
   <223> PCR primer
<400> 371
   gactcctgaggctgtgctatg 21
<210> 372
   <211> 21
   <212> DNA
   <213> Artificial
<220>
   <223> PCR primer
<400> 372
   gatccatgtttgggaaatcc t 21
<210> 373
   <211> 21
   <212> DNA
   <213> Artificial
<220>
   <223> PCR primer
<400> 373
   tcctggtatgcaagatcaag g 21
<210> 374
   <211> 21
   <212> DNA
   <213> Artificial
<220>
   <223> PCR primer
<400> 374
   agagcgtcctttgagaactc c 21
<210> 375
   <211> 21
   <212> DNA
   <213> Artificial
<220>
   <223> PCR primer
<400> 375
   agcagctgaagcagaagtac g 21
<210> 376
   <211> 21
   <212> DNA
   <213> Artificial
<220>
   <223> PCR primer
<400> 376
   gctcatcagacaaaagcttg g 21